# EUROPÄISCHE PATENTSCHRIFT

(11) **EP 1 315 968 B1**
(45) Veröffentlichungstag und Bekanntmachung des Hinweises auf die Patenterteilung: **13.02.2008**
(21) Anmeldenummer: 01969680.6
(22) Anmeldetag: 31.08.2001
(51) Int. Cl.: G01N 33/544, G01N 33/551, A61L 27/30, A61L 29/10, A61L 31/08, A61L 33/00

(54) **VERFAHREN ZUR ABSCHEIDUNG VON MONO- UND MEHRFACHSCHICHTEN VON ORGANOPHOSPHOR- UND -PHOSPHONSÄUREN UND DEREN SALZEN SOWIE DEREN VERWENDUNG**
METHOD FOR PRECIPITATING MONO AND MULTIPLE LAYERS OF ORGANOPHOSPHORIC AND ORGANOPHOSPHONIC ACIDS AND THE SALTS THEREOF IN ADDITION TO USE THEREOF
PROCEDE DE PRECIPITATION DE COUCHES SIMPLES OU MULTIPLES D'ACIDES ORGANOPHOSPHORIQUES ET ORGANOPHOSPHONIQUES ET DE LEURS SELS, ET UTILISATION DE CES COMPOSES

(30) Priorität: 05.09.2000 CH 173200
(43) Veröffentlichungstag der Anmeldung: 04.06.2003
(73) Patentinhaber: Bayer Technology Services GmbH, 51368 Leverkusen (DE)
(72) Erfinder: HOFER, Rolf, CH-8447 Dachsen (CH); PAWLAK, Michael, 79725 Laufenburg (DE); TEXTOR, Marcus, CH-8200 Schaffhausen (CH); SCHÜRMANN-MADER, Eveline, CH-5089 Zeihen (CH); EHRAT, Markus, CH-4312 Magden (CH); TOSATTI, Samuele, CH-6983 Magliaso (CH)
(86) Internationale Anmeldenummer: PCT/EP2001/010077
(87) Internationale Veröffentlichungsnummer: WO 2002/020873

(56) Entgegenhaltungen:
- WO-A-98/29580
- WO-A-98/43086
- WO-A-99/52574
- US-A- 5 958 430
- TAGAKI W, ET AL: "The Syntheses and Hydrolyses of p-Substituted Phenyl Phosphosulfates." BULL. CHEM. SOC. JAPAN, Bd. 44, 1971, Seiten 1139-1141, XP002209350
- EIKI T, ET AL: "Phosphonosulfates. Metal Ion Catalysis in the Hydrolysis of 2-Pyridyl- and 2-Pyridylmethylphosphonosulfate." J. AM. CHEM. SOC., Bd. 104, 1982, Seiten 1986-1991, XP002209351
- ROSS KC, ET AL: "Use of Bis[2-(trialkylsilyl)ethyl] N,N-Dialkylphosphoramidites for the Synthesis of Phosphate Monoesters." J. CHEM. SOC. PERKIN TRANS. 1, Bd. 4, 1995, Seiten 421-426, XP002209352
- PATENT ABSTRACTS OF JAPAN vol. 1999, no. 09, 30. Juli 1999 (1999-07-30) & JP 11 112061 A (SHARP CORP.;AGENCY OF IND. SCIENCE & TECHNOL.), 23. April 1999 (1999-04-23) & US 6 225 239 B1 (SHARP CORP.; AGENCY OF IND. SCIENCE & TECHNOL.) 1. Mai 2001 (2001-05-01)

## Beschreibung

Die Erfindung betrifft ein Verfahren zur Abscheidung von Mono- oder Mehrfachschichten aus Organophosphorsäuren der allgemeinen Formel I (A)

**Y-B-OPO₃H₂** **(IA)**

oder aus Organophosphonsäuren der allgemeinen Formel I (B)

**Y-B-PO₃ H₂** **(IB)**

und deren Salzen, in denen B einen Alkyl-, Alkenyl-, Alkinyl-, Aryl-, Aralkyl-, Hetaryl- oder Hetarylalkylrest und Y Wasserstoff oder eine funktionelle Gruppe aus der Reihe Hydroxy, Carboxy, Amino, gegebenfalls durch Niederalkyl substituiertes Mono-oder Dialkylamino, Thiol, oder eine negative Säuregruppe aus der Reihe Ester, Phosphat, Phosphonat, Sulfat, Sulfonat, Maleimid, Succinimydyl, Epoxy, Acrylat bedeutet, wobei an B oder Y ein biologisches, biochemisches oder synthetisches Erkennungselement durch Addition- oder Substitutionsreaktion angedockt sein kann, wobei auch Verbindungen angelagert sein können, die der Substratoberfläche eine Resistenz gegen Proteinadsorption und/oder Zelladhäsion verleihen und in B in der Kette gegebenenfalls anstelle einer oder mehrerer-CH₂- Gruppen eine oder mehrere Ethylenoxidgruppen enthalten sein können, auf Substratoberflächen von reinen oder gemischten Oxiden, Nitriden oder Carbiden von Metallen und Halbleitern, dadurch gekennzeichnet, dass zur Behandlung dieser Oberflächen, insbesondere als Oberflächen von Sensorplattformen, Implantaten und medizinischen Hilfsgeräten, wasserlösliche Salze einer Verbindung der Formel (IA) bzw. (IB) in wässriger Lösung eingesetzt werden. Ferner betrifft die Erfindung deren Verwendung als Teil von beschichteten Sensorplattformen, Implantaten und medizinischen Hilfsgeräten.

Die gegebenenfalls substituierten Verbindungen der Formeln (IA) und (IB), in denen die Gruppierungen B und Y die oben erwähnten Bedeutungen haben, d.h. gemeinsam gegebenfalls substituiertes Alkyl, Alkenyl, Alkinyl, Aryl, Aralkyl, Hetaryl oder Hetaryl bedeuten, sollen auch nachfolgend unter den Bezeichnungen Organophosphorsäuren bzw. - phosphonsäuren zusammengefasst werden.

Für die Herstellung von Sensoroberflächen mit auf einem sogenannten Transducer immobilisierten biologischen oder biochemischen Erkennungselementen zur hocheffizienten und hochselektiven Bindung eines oder mehrerer in einer Probe nachzuweisender Analyten ist die Beschaffenheit der Transduceroberfläche von grosser Bedeutung. Zur Erreichung möglichst tiefer Nachweisgrenzen ist es erwünscht, auf kleinem Raum möglichst viele Erkennungselemente zu immobilisieren, an die in dem späteren Nachweisverfahren dann möglichst viele Analytmoleküle einer Sorte gebunden werden können. Zugleich ist es erwünscht, bei der Immobilisierung die Reaktivität und biologische oder biochemische Funktionalität der Erkennungselemente in möglichst hohem Masse zu erhalten, d.h. jegliche Denaturierungserscheinungen infolge der Immobilisierung zu minimieren. Ein weiteres Ziel ist es, unspezifische Bindung oder Adsorption von Analytmolekülen, welche in vielen Fällen limitierend auf die erreichbaren Nachweisgrenzen einwirkt, weitestgehend zu verhindern. Zur Erfüllung dieser Aufgaben sind eine Reihe verschiedener Methoden zur chemischen Modifikation, mit dem Ziel der Herstellung sogenannter biokompatibler Oberflächen, entwickelt worden. Zum Teil ähnliche Anforderungen werden in der Medizin an die Beschaffenheit der Oberflächen von Implantaten gestellt.

Im Bereich der medizinischen Implantate, wie z.B. Dentalimplantate, künstliche Hüftgelenke oder intravaskuläre Stents, oder bei biomedizinischen Hilfsgeräten, wie z.B. Kathetern oder Endoskopen, spielt die Oberflächenbeschaffenheit eine wichtige Rolle für die Funktionalität des Teils (B. Kasemo, J. Lausmaa, "Surface Science Aspects on Inorganic Biomaterials", CRC Crit. Rev. Biocomp. 2 (1986), 335-380). Die wichtigste Ursache dafür ist die Tatsache, dass die Oberfläche des Implantates nach einer Implantation im Körper die Grenzfläche zwischen der biologischen Umgebung und dem Fremdmaterial bildet, an der sich die wichtigen Prozesse wie Fremdkörperreaktionen, Entzündungen, Zelladhäsion und Neubildung von Gewebe abspielen. Von besonderer Bedeutung ist die Adsorption von Proteinen aus der Körperflüssigkeit (Blut, interzelluläre Flüssigkeit etc.), da diese Prozesse kurze Zeit nach der Implantation stattfinden und die weiteren biologisch wichtigen Vorgänge, insbesondere die Anlagerung von Zellen, steuern. Die Oberflächeneigenschaften des Implantates sind dabei entscheidend, da sie direkt die Art der Proteine, deren Bindungsstärke an der Oberfläche und deren Konformation und Orientierung bestimmen (B. Ratner, "New ideas in biomaterials science - a path to engineered biomaterials", J. Biomed. Mat. Res. 27 (1993), 837-850), Diese Bemerkungen gelten für die verschiedensten Anwendungen. Beispiele im Bereich metallischer Implantate sind:
1. Permanente oder temporär implantierte Komponenten aus Stahl, Kobalt-Chrom-Legierungen, Titan oder Titanlegierungen im Knochenbereich, wie z.B. künstliche Hüftgelenke, Zahnwurzelimplantate, Osteosynthese-Platten oder -schrauben. Hier wird eine Adsorption von zelladhäsiven Proteinen gewünscht, welche die Integration des Teils im Knochen (Osteointegration) günstig beeinflussen.
2. Metallische Teile aus Stahl, Titan oder Nickel-Titan-Legierungen, die Funktionen im Kontakt mit Blut übernehmen, wie z.B. Stents in der Blutbahn, um Arterien- oder Venenverschlüsse permanent zu verhindern. Solche Teile müssen eine den spezifischen Anforderungen entsprechende Blutkompatibilität aufweisen. Oft wird hier eine Oberfläche des Implantates gefordert, welche resistent gegen Proteinadsorption und Blutplättchenadhäsion ist und damit eine Oberfläche darstellt, welche keine oder nur eine geringe Neigung zu unerwünschter Thrombosebildung aufweist.
3. Teile aus Titan oder Stahl, welche im Kontakt mit Weichgewebe stehen, z.B. bei Osteosyntheseanwendungen zur Unterstützung der Heilung von Knochenbrüchen oder bei Dentalimplantaten im Bereich des Kontaktes mit Zahnfleisch. Hier wird oft gewünscht, dass das Weichgewebe zwar dicht an dem Implantat anliegt, aber keine feste Verbindung eingeht.

Gelingt es, die Oberflächen solcher Implantate gezielt auf die Einsatzbedingungen hin einzustellen, können die oben genannten Prozesse günstig beeinflusst und damit die Funktionalität des Implantats entscheidend verbessert werden. Es gibt eine ganze Reihe von Oberflächeneigenschaften, welche erwiesenermassen oder hypothetisch die Kompatibilität mit der spezifischen Anwendung im Körper beeinflussen:
1. Die Benetzbarkeit der Oberfläche oder Abstossung von Wasser von der Oberfläche (Hydrophilizität / Hydrophobizität der Oberfläche, gemessen z.B. als Kontaktwinkel gegen Wasser), die mit der Oberflächenenergie in Verbindung steht. Dabei kann es vorteilhaft sein, für eine bestimmte Anwendung eine ideale Benetzbarkeit gegen Wasser einzustellen.
2. Die Ladung der Oberfläche (positive, negative oder keine Ladung) zeigt einen starken Einfluss auf das Verhalten von Zellen an der Oberfläche eines Implantates, beispielsweise gegenüber Knochenzellen ( J. E. Davies, B. Causton, Y. Bovell, K. Davy, C. S. Sturt, "The Migration of Osteoblasts Over Substrata of Discrete Surface-Charge", Biomaterials, Vol. 7 (1986), 231 - 233; R. M. Shelton, I. M. Whyte, J. E. Davies, "Interaction between Primary Bone Cell and Biomaterials. Part 4: Colonization of Charged Polymer Surfaces"; Biomaterials and clinical applications: proceedings of the Sixth European Conference on Biomaterials, Bologna, Italy, September 14-17, 1986; Elsevier; 1987; 597-602).
3. Die Anwesenheit von funktionellen Gruppen oder biologischen Molekülen, welche auf die Oberfläche des Implantates aufgebracht werden und nach der Implantation das biologische Geschehen an der Oberfläche beeinflussen. Dazu gehören z.B. spezifische Peptide, Proteine oder Wachstumsfaktoren (S.-J. Xiao, M. Textor, N.D. Spencer, H. Sigrist, "Covalent Attachment of Cell-Adhesive, RGD-Containing Peptides on Titanium Surfaces", Langmuir 14 (1998), 5508 - 16, 1998).
4. Die Anwesenheit von proteinabweisenden Molekülen, wie z.B. Polyethylenoxid, Polyethylenglykol oder Heparin an der Oberfläche.

Die chemischen Eigenschaften der Oberfläche kommerzieller Implantate sind oft nicht in einem Masse auf die Anwendung hin optimiert, wie man sich dieses für die medizinische Anwendung wünschen würde. Sie werden zudem in der kommerziellen Fertigung oft nur unvollständig kontrolliert. Verunreinigungen werden festgestellt, welche die Folge der Verarbeitungs- oder Lagerbedingungen sind. Solche Verunreinigungen sind potentiell schädlich für die Anwendung im Körper. Zudem sind sie meist nicht konstant von Fertigungscharge zu Fertigungscharge bzw. über die Lagerzeit und stellen ein Risiko betreffend Qualitätssicherung medizinischer Produkte dar . Besonders davon betroffen sind metallische Implantate, wie z.B. solche aus Stahl oder anderen Eisenlegierungen, Titan und dessen Legierungen (TiAIV, TiAlNb, etc.), Cobalt-Chrom Legierungen (CoCr, CoCrMo), welche alle mit einer dünnen, natürlichen Oxidschicht hoher Oberflächenenergie bedeckt sind und dabei besonders anfällig auf Kontamination durch die Umgebung sind (z.B. durch Adsorption von organischen Komponenten aus der Luft, wie Kohlenwasserstoffe, Alkohole, etc., oder durch Adsorption aus Flüssigkeiten (Adsorption von Silikonölen, etc.).

Aufgabe der Erfindung ist die Bereitstellung eines solchen Verfahrens zur Behandlung der Oberflächen, insbesondere von Oxiden, Nitriden oder Carbiden von Metallen oder Halbleitern oder deren Mischungen bzw. von oxidbedeckten Metallen oder Halbleitern, unter Verwendung von Mono- bzw. Mehrfachschichten von entsprechend oben definierten Organophosphor- oder Organophosphonsäuren oder deren Derivaten, insbesondere deren Salzen, dass dabei Oberflächen entstehen, welche reproduzierbare, chemische Zusammensetzung aufweisen, und die Eigenschaften dieser Oberflächen für den spezifischen Anwendungsfall im Bereich der Biomaterialien / Implantate bzw. der Biosensorik masszuschneidern.

In der US 5,997,621 und der US 5,873,931 werden Beschichtungsmischungen und Verfahren zur Antireflexions- und Antibeschlagbeschichtung von Oberflächen beschrieben. Die Verwendung einer Reihe verschiedener Amphiphiler wird genannt, jedoch dient deren Kombination mit porösen Metalloxiden, welche beispielsweise in einer Dispersion vorliegen, nur zur Ausbildung eines Netzwerks aus Metalloxiden und Amphiphilen, welches dann seinerseits auf die zu beschichtende Oberfläche aufgebracht wird. Hinweise auf eine Beschichtung mit Amphiphilen in einem Selbstorganisationsprozess ("self assembled monolayer"- Ausbildung) finden sich nicht.

In der US 5,922,550 wird die Übertragung von selbstorganisierten Monoschichten mittels Stempelverfahren auf metallbeschichtete Plastikfilme beschrieben, wobei in die zu übertragenden Monoschichten analytspezifische Rezeptoren eingelagert sind. Massive Substrate oder andere Materialien als Träger der Metallschicht (aus Gold, Silber, Aluminium, Chrom, Kupfer, Zirkonium, Platin und Nickel sowie deren Oxiden) werden nicht beschrieben.

Insbesondere wird ein Abscheidungsprozess des SAM auf einem Metalloxidfilm, der als optischer Wellenleiter geeignet wäre, entsprechend einer bevorzugten Ausführungsform unseres erfindungsgemässen Verfahrens, nicht erwähnt. Es werden zwar Alkylphosphonate der -phosphonsäuren erwähnt, jedoch keine Alkylphosphate oder Alkylphosphorsäuren.

Die Benutzung von Dodecylphosphat-Ammoniumsalz (DDPO₄-Ammoniumsalz, DDPO₄(NH₄)₂ ist in einer Reihe von Patenten beschrieben (zum Beispiel US 4,005,173; US 4,491,531; US 4,838,556; US 5,873,931; US 5,997,621), jedoch nicht dessen Verwendung zur Ausbildung einer selbstorganisierten Monoschicht auf einem makroskopischen Substrat durch Abscheidung aus wässriger Lösung.

In J. G. van Alsten, "Self-assembled monolayers on engineering metals: structure, derivatization and utility", Langmuir 15 (1999) 7605 - 7614, wird die Abscheidung von SAMs basierend auf alkylphosphonischen Säuren aus wässrigen und alkoholischen Lösungen auf sogenannten "engineering metals" (Stahl, Aluminium, Kupfer, Messing) beschrieben, jedoch nicht die Bildung von SAMs basierend auf Alkylphosphorsäuren oder deren Derivaten.
In der WO 98/29580 wird ein Verfahren zur Behandlung von metallischen Oberflächen aus Zink, Magnesium, Aluminium oder deren Legierungen, durch Spritzen, Tauchen oder Walzen, beschrieben, um die Haftung und Korrosionsbeständigkeit lackierter und mit Kunststoffen beschichteter Produkte zu verbessern. Dabei werden Alkylphosphorsäuren- oder Alkylphosphonsäuren bzw. deren Derivate mit 2 - 50 Kohlenstoffatomen in der Alkylkette und verschiedenen endständigen funktionellen Gruppen beschrieben. Die Abscheidung erfolgt aus wässriger Lösung, wobei die Löslichkeit einiger dieser Verbindungen durch Zusätze von organischen Lösungsmitteln erhöht wird. Nicht beschrieben werden die schlecht wasserlöslichen Phosphonsäuren mit endständiger Methylgruppe, wie z.B. die 1-Phosphonsäure-dodecan oder 1-Phosphorsäure-octadecan. Hingegen werden die Hydroxy-terminierten Moleküle beschrieben, wie z.B. 1-Phosphorsäure-12-Hydroxydodecan. Die Anwendung dieser Molekülklasse ist auf die oben genannten Metalle beschränkt, und nach der Oberflächenbehandlung wird darauf jeweils eine weitere kompakte organische Deckschicht, z. B. ein Lack oder Kunststoff, aufgebracht. Die beispielsweise mit Alkylphosphor- oder Alkylphosphonsäuren oder deren Derivaten modifizierte Metallschicht dient also nie als äusserste, der Umgebung ausgesetzte Schicht.

Seit einigen Jahren werden Oberflächen mittels sogenannter selbstorganisierter Monoschichten ("self-assembled monolayers", im folgenden als "SAM" abgekürzt) modifiziert. Dabei handelt es sich um sehr dünne, monomolekulare Schichten, die sich spontan durch Kontaktieren einer Oberfläche mit einer Lösung des entsprechenden Moleküls bilden und sich durch eine geordnete Struktur der Molekülketten auszeichnen. Am besten bekannt sind langkettige Alkylthiole, welche SAMs auf Goldoberflächen bilden (R. G. Nuzzo, F. A. Fusco, D. L. Allara, "Spontaneously Organized Molecular Assemblies .3. Preparation and Properties of Solution Adsorbed Monolayers of Organic Disulfides On Gold Surfaces"; Journal of the American Chemical Society, 109 (1987), 2358-2368)). Solche Gold / Alkylthiol-SAM-Oberflächen finden im biomedizinischen Bereich als Modelloberflächen dank der perfekt kontrollierten chemischen Oberflächeneigenschaften zwar Anwendungen, für die Herstellung von Implantaten sind sie jedoch bedeutungslos, da Gold in diesem Anwendungsgebiet von nur untergeordneter Bedeutung ist.

Demgegenüber spielen Goldoberflächen eine grössere Rolle in der Bioanalytik. Beispielsweise ist das Phänomen der Oberflächenplasmonen-Resonanz charakteristisch für eine bestimmte Sorte dünner Metallfilme, insbesondere aus Gold. Daher fanden in den vergangenen Jahren Oberflächenmodifikationen mittels SAM-Herstellung aus Alkylthiolen insbesondere hier Anwendung.

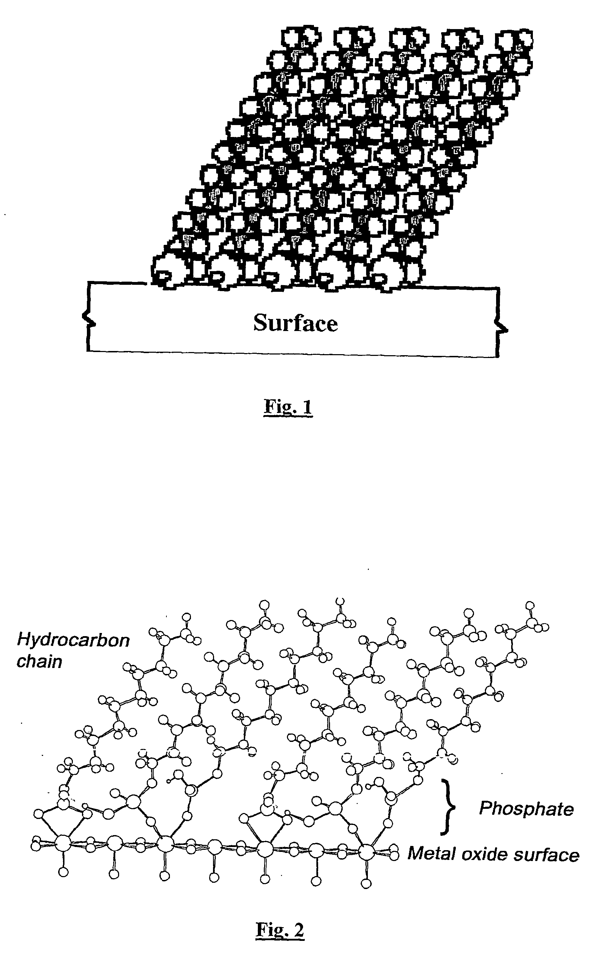

Andererseits wurde gezeigt, dass sich Alkylphosphate an oxidischen Oberflächen adsorbieren. Erstmals gezeigt wurde dieses auf Glimmeroberflächen (J. T. Woodward et al., Larrgmuir 12 (1996) 6429) sowie auf Aluminiumoberflächen (D. L. Allara, R. G. Nuzzo, "Spontaneously Organized Molecular Assemblies .1. Formation, Dynamics, and Physical-Properties of Normal-Alkanoic Acids Adsorbed From Solution On an Oxidized Aluminum Surface", Langmuir 1 (1985), 45-52; D. L. Allara, R. G. Nuzzo, "Spontaneously Organized Molecular Assemblies .2. Quantitative Infrared Spectroscopic Determination of Equilibrium Structures of Solution-Adsorbed Normal-Alkanoic Acids On an Oxidized Aluminum Surface", Langmuir 1 (1985), 52-66; T. Maege, E. Jaehne, A. Henke, H.-P. Adler, C. Bram, C. Jung, M. Stratmen, Macromol. Symp. 126 (1997), 7 - 24). Die auf Glimmer gebildeten Monoschichten waren jedoch wenig stabil und damit für technische Anwendungen von geringer Bedeutung. Hingegen wurde gefunden, dass Octadecylphosphat auf Tantaloxid (Ta₂O₅) SAMs bildet, welche denjenigen von Thiolen auf Gold strukturell sehr ähnlich und zudem bedeutend stabiler als auf Glimmer sind (M. Textor, L. Ruiz, R. Hofer, A. Rossi, K. Feldman, G. Hähner, N.D. Spencer, "Structural Chemistry of Self-Assembled Monolayers of Octadecylphosphoric Acid on Tantalum Oxide Surfaces" Langmuir 16 (2000), 3257-3271; D. Brovelli, G. Hähner, L. Ruiz, R. Hofer, G. Kraus, A. Waldner, J. Schlösser, P. Oroszlan, M. Ehrat, N. D. Spencer, "Highly Oriented, Self-Assembled Alkanephosphate Monolayers on Tantalum(V) Oxide Surfaces", Langmuir 15 (1999), 4324-4327). Die Technik der selbstorganisierten Schichten aus Octadecylphosphat erlaubt es, die Oberfläche von Tantaloxid dank geordneter Struktur der hydrophoben Alkylketten extrem hydrophob zu machen (Kontaktwinkel gegenüber Wasser: 112 - 115°) (Fig. 1 und 2).

Da beispielsweise Alkylphosphate, in denen B und Y die oben angegebenen Bedeutungen haben, grundsätzlich auch mit anderen endständigen Gruppen als Methyl, z.B. mit Hydroxyl (-OH), Amin (-NH₂) oder Carboxyl (-COOH) ausgerüstet werden können, bietet die Technik natürlicherweise die Möglichkeit, oxidbedeckte medizinische Implantatoberflächen bzw. (optische) Sensoren mit Oxidoberfläche gezielt chemisch zu behandeln und die chemischen Oberflächeneigenschaften auf den Einsatzfall hin masszuschneidern und stabil zu halten.
Diese geordneten Alkylphosphatschichten auf Tantaloxid wurden durch Kontaktieren der oxidischen Oberflächen mit einer Lösung des Alkylphosphats auf Basis organischer Lösungsmittel (Heptan / Isopropanol) hergestellt. Wässrige Lösungen konnten deshalb nicht verwendet werden, da diese langkettigen Alkylphosphate nicht genügend wasserlöslich sind.
Kurzkettige Alkylphosphate wären zwar genügend wasserlöslich, sie bilden aber fast keine geordneten SAM-Schichten, da die Wechselwirkungen zwischen den langen Alkylketten innerhalb der organisierten Adsorbatschicht für die Ausbildung von gerichteten Ketten und Ordnung erforderlich sind.

Muss der Oberflächenbehandlungsprozess in organischen Lösungsmitteln durchgeführt werden, stellen sich Probleme, welche die kommerziellen Anwendungen zumindestens sehr einschränken, wenn nicht gar überhaupt verunmöglichen:
1. Diese Art der Modifikation ist beschränkt auf Oberflächen von Substraten, deren Materialien eine ausreichend hohe Resistenz gegen organische Lösungsmittel aufweisen. Beispielsweise ist ein solcher Prozess der Oberflächenmodifikation von Metall- oder Metalloxidfilmen auf Plastiksubstraten nahezu auszuschliessen.
2. Die Verwendung flüchtiger organischer Lösungsmittel ist mit Emissionen verbunden, welche Arbeitsplatz, Umgebung und Atmosphäre belasten. Die Gesetzgebung ist in vielen Ländern diesbezüglich sehr streng geworden und hat in verschiedenen Industrien dazu geführt, dass solche Lösungsmittel gänzlich ersetzt wurden durch Prozesse, welche keine oder weniger Umweltprobleme verursachen.
3. Falls diese Emissionen eliminiert werden sollen, muss mit einem hohen Mehraufwand gerechnet werden (geschlossene Anlagen, Abluftreinigung, Rückgewinnung der Lösungsmittel). Damit ergeben sich erhöhte Kosten, welche oft die ökonomische Bilanz solcher Prozesse soweit verschlechtern, dass die für eine kommerzielle Fertigung unattraktiv werden.
4. Spezifisch im Falle der Biomaterialien und Implantate haben organische Lösungsmittel den gravierenden Nachteil, dass sie oft toxisch auf Zellen wirken oder die Entwicklung von Zellen und Geweben negativ beeinflussen. Da solche aus organischen Lösungsmitteln hergestellte SAM-Schichten nie hundertprozentig frei sind von organischen Lösungsmitteln, besteht immer das Risiko, dass die in der SAM-Schicht verbleibenden organischen, flüchtigen Moleküle sich später im Körper negativ auswirken.
5. Oftmals muss damit gerechnet werden, dass die erwünschten spezifischen Eigenschaften nicht durch eine einzelne Art von Molekülen erreicht werden können, sondern den Einsatz einer Mischung von SAM-Molekülen erfordern. Da aber verschiedene SAMs sehr unterschiedliche Löslichkeit in organischen Lösungsmitteln haben (insbesondere bei unterschiedlich polaren ω-endständigen Gruppen), ist es oft schwierig oder unmöglich, ein einzelnes Lösungsmittel zu finden, das die Herstellung einer Mischung der beiden (oder mehrerer) Moleküle für die Abscheidung einer gemischten SAM-Schicht erlaubt.

Gemäss vorliegender Erfindung hat man sich zum Ziel gesetzt, die oben genannten Nachteile bei der Herstellung von SAM-Schichten, basierend auf den vorangehend definierten Organophosphaten oder -phosphonaten oder deren Derivaten, insbesondere Salzen, funktionalisierten Organophosphaten bzw. den entsprechenden Phosphonaten, auszuräumen und ein Verfahren zu entwickeln, das die Bildung von wohldefinierten SAM-Schichten auf einer Reihe von Metall-, Halbleiter-, Oxid-, Carbid- oder Nitridoberflächen ohne Verwendung von Lösungsmitteln erlaubt sowie die Herstellung von Schichten ermöglicht, welche aus zwei oder mehr verschiedenen, funktionalisierten und / oder nicht funktionalisierten Organophosphaten oder -phosphonaten bestehen.

Die Erfindung betrifft ein Verfahren zur Abscheidung von Mono- oder Mehrfachschichten aus Organophosphorsäuren der allgemeinen Formel I (A)

**Y-B-OPO₃ H₂** **(IA)**

oder aus Organophosphonsäuren der allgemeinen Formel I (B)

**Y-B-PO₃ H₂** **(IB)**

und deren Salzen, in denen B einen Alkyl-, Alkenyl-, Alkinyl-, Aryl-, Aralkyl-, Hetaryl- oder Hetarylalkylrest und Y Wasserstoff oder eine funktionelle Gruppe aus der Reihe Hydroxy, Carboxy, Amino, gegebenfalls durch Niederalkyl substituiertes Mono-oder Dialkylamino, Thiol, oder eine negative Säuregruppe aus der Reihe Ester, Phosphat, Phosphonat, Sulfat, Sulfonat, Maleimid, Succinimydyl, Epoxy, Acrylat bedeutet, wobei an B oder Y ein biologisches, biochemisches oder synthetisches Erkennungselement durch Addition- oder Substituionsreaktion angedockt sein kann, wobei auch Verbindungen angelagert sein können, die der Substratoberfläche eine Resistenz gegen Proteinadsorption und/oder Zelladhäsion verleihen und in B in der Kette gegebenenfalls anstelle einer oder mehrerer-CH₂- Gruppen eine oder mehrere Ethylenoxidgruppen enthalten sein können, auf Substratoberflächen von reinen oder gemischten Oxiden, Nitriden oder Carbiden von Metallen und Halbleitern,
dadurch gekennzeichnet, dass zur Behandlung dieser Oberflächen, insbesondere als Oberflächen von Sensorplatten, Implantaten und medizinischen Hilfsgeräten, wasserlösliche Salze einer Verbindung der Formel (IA) bzw. (IB) in wässriger Lösung eingesetzt werden. Ferner betrifft die Erfindung deren Verwendung als Teil von beschichteten Sensorplattformen, Implantaten und medizinischen Hilfsgeräten.

In erster Linie betrifft die Erfindung Verfahren zur Abscheidung von Mono- oder Mehrfachschichten aus Organophosphorsäuren der Formel I(A) oder aus Oganophosphonsäuren der Formel I(B) und deren Salzen, in denen die Gruppen B und Y eine Alkylgruppe oder eine gegebenenfalls substituierte Alkylgruppe von 2 - 24 C-Atomen bedeuten.

Insbesondere betrifft die Erfindung Verfahren zur Abscheidung von Mono- oder Mehrfachschichten aus Organophosphorsäuren der Formel I(A) oder aus Organophosphonsäuren der Formel I(B) und deren Salzen, in denen die Gruppen B und Y gemeinsam eine Alkylgruppe oder eine gegebenenfalls substituierte Alkylgruppe von 2 - 12 C-Atomen bedeuten.

Von besonderer Bedeutung können jedoch je nach Verwendungszweck auch Verfahren zur Abscheidung von Mono- oder Mehrfachschichten aus Organophosphorsäuren der Formel I(A) oder Organophosphorsäuren der Formel I(B) und deren Salzen, in denen die Gruppen B und Y gemeinsam eine Alkylgruppe oder eine gegebenenfalls substituierte Alkylgruppe mit 2-5 C-Atomen bedeuten.

Als Substituenten kommen in erster Linie die eingangs unter Y aufgezählten Substituenten in Frage.

Die bevorzugten Schichten, d.h. Mono- und Mehrfachschichten aus Organophosphorsäuren der Formel I(A) und Organophosphonsäuren der Formel I(B) dienen auch zur Verwendung als Teil von beschichteten Sensorplattformen, Implantaten und medizinischen Hilfsgeräten.

Je nach Verwendungszweck können bevorzugt Verbindungen der Formel I(A) bzw. I(B) mit verschiedenen Kettenlängen, wie oben angegeben, verwendet werden.

Alkyl ist beispielsweise C2-C24-Alkyl, wie Ethyl, Propyl, , Butyl, Pentyl, Hexy, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosanyl, Heneicosanyl, Docosanyl, Tricosanyl oder Tetracosanyl.
Alkenyl ist beispielsweise Ethylen, Propylen, Butylen, Pentylen, 2-Methyl-penten-(1) und weitere höhergliedrige wie zm Beispiel Hexadecenyl, Heptadecenyl oder auch Octadecenyl.

Alkinyl ist beispielsweise Ethinyl, 2- Propinyl, 2-oder 3-Butinyl oder auch höhergliedrige wie zum Beispiel 4-Pentinyl.

Aryl als solches ist z.B. Phenyl oder Naphthyl, wie z.B. 1- oder 2-Naphthyl oder substitueirtes Phenyl oder Naphthyl, wie z.B. Phenyl oder Naphthyl, welche neben Y auch zusätzlich noch durch Niederalkyl, Halogen-niederalhyl, Hydroxy, Niederalkoxy, Niederalkanoyloxy, Halogen, und/oder Cyano substituiert sind. Aryl ist bevorzugt unsubstituiertes oder wie oben angegeben substituiertes Phenyl, insbesondere Phenyl.

Arylalkyl ist vorzugsweise Arylniederalkyl, insbesondere Phenylniederalkyl, ganz besonders Phenylethyl oder Benzyl.

Niederalkoxy ist z.B. n-Propoxy, Isopropoxy, n-Butoxy oder tert.-Butoxy, vorzugsweise Ethoxy und vor allem Methoxy.

Niederalkanoyloxy ist beispielsweise Propionyloxy oder Pivaloyloxy, vorzugsweise Acetyloxy.

Halogen ist beispielsweise Chlor oder Fluor, ferner Brom und Jod.

Halogenniederalkyl ist beispielsweise 2- oder 3-Halogenniederalkyl, wie beispielsweise 2-Halopropyl, 3-Halopropyl oder 3-Halo-2-methyl-propyl.

Unter Hetaryl ist ein insbesondere monocyclischer, aber auch bi- oder polycyclischer Rest aromatischen Charakters zu verstehen. Bi- und polycyclisches Hetaryl kann aus mehreren heterocyclischen Ringen zusammengesetzt sein oder bevorzugt aus einem Heterocyclus und einem oder mehrere, z.B. einem oder zwei und insbesondere einem, annellierten carbocyclischen Ring, insbesondere Benzoring bestehen. Jeder einzelne Ring enthält z.B. 3, 5, 6, 7 und insbesondere 5 oder 6 Ringglieder.
Hetaryl steht insbsondere für einen aza-, thia- oxa-, thiaza-, oxaza-, diaza- und tetrazacyclischen Rest.

Hetaryl steht in erstzer Linie für monocyclische monoaza-, monothia- oder monooxacyclische Reste, wie etwa Pyrryl, z.B. 2-Pyrryl oder 3-Pyrryl, Pyridyl, Thienyl, z.B. 2- oder 3-Thienyl, oder Furyl, z.B. 2-Furyl, bicyclische monoaza-, monooxa- oder monothiacyclische Reste, z.B. Indolyl, z.B. 2- oder 3-Indolyl, Chinolinyl, z.B 2- oder 4 Chinolinyl, Isochinolyl, 1-Isochninolin, Benzofuran, z.B. 2- oder 3-Benzofuranyl, oder Benzothienyl, z.B. 2- oder 3-Benzothienyl, monocyclische diaza-, triaza-, tetraza-, oxaza-, thiaza- oder thiadiazacyclische Reste, wie Imidazolyl, z.B. 2-Imidazolyl, Pyrimidinyl, z.B. 2- oder 4-Pyrimidinyl, Triazolyl, z.B. 1,2,4-Triazol-3-yl, Tetrazolyl, z.B. 1- oder 5-Tetrazolyl, Oxazolyl, z.B 2-Oxazolyl, Isoxazolyl, z.B. 3- oder 4-Isoxazolyl, Thiazolyl, z.B. 2-Thiazolyl, Isothiazolyl, z.B. 3- oder 4-Isothiazolyl oder 1,2,4- oder 1,3,4-Thiadiazolyl, z.B. 1,2,4-Thiadiazol-3-yl oder 1,3,4-Thiadiazol-2-yl, oder bicyclische diaza-, oxaza-, thiazacyclische Reste, wie Benzimidazolyl, z.B. 2-Benzimidazolyl, Benzoxazolyl, z.B. 2-Benzoxazolyl oder Benzthiazoyl, z.B. 2-Benzthiazolyl.

Hetarylreste sind unsubstituiert oder tragen Substituenten wie unter Aryl angegeben.

Hetaryl ist vor allen Dingen Pyridyl, Thienyl, Pyridyl oder Furyl.

Hetarylalkylreste setzen sich aus den obengenannten Hetarylresten und den vorher genannten Alkylresten zusammen.
Die im erfindungsmässen Verfahren verwendeten Organo-phosporsäuren der Formel I(A) und der -phosphonsäuren der Formel (IB) können mit Basen Salze bilden, z.B. entsprechende Alkalimetall- oder Erdalkalimetallsalze, z.B. Natrium-, Kalium- oder Magnesiumsalze, ferner auch Übergangsmetallsalze, wie Zink- und Kupfersalze, oder insbesondere Salze mit Ammoniak oder organischen Aminen, wie cyclischen Aminen, wie Mono-, Di- oder Triniederalkylaminen, wie Hydroxyniederalkylaminen oder Polyhydroxyniederalkylaminen. Cyclische Amine sind z.B. Morpholin, Thiomorpholin, Piperidin oder Pyrrolidin. Als Mononiederalkylamine kommen beispielsweise Ethyl- und teit.-Butylamin, als Diniederalkylamine beispielsweise Diethyl- und Diisopropylamin und als Triniederalkylamine beispielsweise Trimethyl- und Triethylamin in Betracht, welche quaternäre Ammoniumsalze bilden.

Entsprechende Hydroxyniederalkylamine sind z.B. Mono-, Di- und Triethanolamin, Hydroxyniederalkylamine sind z.B. N,N-Dimethyl-mino- und N,N-Diethylamino-ethanol. Umfasst sind ferner die bereits genannten Uebergangssalze, welche für die Verwendung eventuell ungeeignet sind, jedoch für die Isolierung bzw. Reinigung von Organophosphorsäuren der Formel I(A) bzw. Organophosphonsäuren der Formel I(B) vorteilhaft verwendet werden können.

Auch der Substituent Y kann als Carboxylgruppe analoge basische Salze bilden:

Organophosphorsäuren der allgemeinen Formel I(A) bzw. Organophosphonsäuren der allgemeinen Formel I(B) können als Substituenten auch eine basische Gruppierung Y aufweisen.

Beispielsweise, wenn Y Amino oder gegebenfalls durch Niederalkyl oder Diniederalkyl substituiertes Amino bedeutet. Verbindungen mit einer basischen Gruppe Y können z.B. Säureadditionssalze mit geeigneten Mineralsäuren, wie Halogenwasserstoffsäuren, Schwefelsäure oder Phosphorsäure, z.B. Hydrochloride, Hydrobromide, Sulfate, Hydrogensulfate oder Phosphate, Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate), oder Salze mit starken organischen Carbonsäuren, wie Niederalkancarbonsäuren.

Gegenstand der Erfindung sind auch neue Organophoshorsäuren der Formel

**Y-B-OPO₃ H₂** **(IA)**

und Organophosphonsäuren der Formel

**Y-B-PO₃ H₂** **(IB)**

in denen B und Y die oben angegebenen Bedeutungen mit Ausnahme von der gemeinsamen Bedeutung von Alkyl haben und deren Salze.

Insbesondere betrifft die Erfindung neue Organophosphorsäuren der Formel I(A) und Organophosphonsäuren der Formel I(B) in denen B und Y gemeinsam einen Aryl bzw. Arylalkylrest bedeuten und deren Salze.

Offenbart wird die Herstellung eines Salzes der freien Organophosphorsäure oder Organophosphonsäure in Wasser, in einem anderen Lösungsmittel oder einer Mischung und die gleichzeitige oder anschliessende Fällung des entsprechenden Salzes durch Einbringen einer Base, welche das Kation im entstandenen Salz bildet. Die Fällung erfolgt entweder spontan oder nach einer entsprechenden Kühlung oder Aufkonzentration der Lösung. Als Kationen kommen alle gängigen positiv geladenen Teilchen in Frage, wie die Alkalimetalle (z.B. Na⁺, K⁺), die Erdalkalimetalle, Ammonium (NH₄⁺) oder andere quarternäre Ammoniumionen (wie z.B. Tetrabutylammonium). Die Salzbildung erfolgt dabei durch Einbringen der entsprechenden wässrigen Base (z.B. KOH oder NH₄OH) in die Lösung der Organophosphor- oder Organophosphonsäure, oder durch Einbringen einer Base in einem organischen Lösungsmittel (z.B. Tetrabutylammoniumhydroxid in alkoholischer Lösung) in die Lösung der Organophosphor- oder Organophosphonsäure, oder aber durch Einbringen einer bei Zimmertemperatur oder erhöhter Temperatur flüchtigen (gasförmigen) Base (wie z.B. Ammoniak oder ein flüchtiges Amin) in die Lösung der Organophosphor- oder Organophosphonsäure. Die entstandene, salzartige Verbindung hat dann in der gewählten Lösung entweder eine ungenügende Löslichkeit und fällt spontan als Salz aus, oder die Fällung wird durch Abkühlen oder Einengen des Lösungsvolumens erreicht. Es wird bevorzugt, dass als Salze der Verbindungen der Formel (IA) und / oder (IB) insbesondere Natrium-, Kalium- oder Ammoniumsalze verwendet werden. Insbesondere bevorzugt wird dabei die Bildung des Ammoniumsalzes, da Ammonium im nachfolgenden SAM-Prozess dank seiner geringen Affinität für Oberflächen die Bildung der SAM-Schicht keineswegs stört.
Ein weiterer Vorteil dieser Salze für ihre Verwendung in dem erfindungsgemässen Verfahren ist die Tatsache, dass sie anschliessend leicht durch Umkristallisation gereinigt und von unerwünschten Verunreinigungen der freie Säure befreit werden können.
Ein wichtiges Kennzeichen des erfindungsgemässen Verfahrens ist also, dass vor der Abscheidung besagter Mono- oder Mehrfachschichten das wasserlösliche Salz einer Verbindung der Formel (IA) und / oder (IB) in wässriger Lösung isoliert wird.
Die Herstellung des Ammoniumsalzes der Dodecanphsophorsäure ist zwar bereits beschrieben worden (siehe oben), jedoch nicht für die Anwendung als Salz in einem nachfolgenden SAM-Prozess basierend auf einer wässrigen Lösung des entsprechenden Salzes.
Der erfindungsgemässe Einsatz der gebildeten Salze betrifft die Verwendung von wässrigen Lösungen des Salzes im SAM-Bildungsprozess für die Behandlung von Oxid-, Carbid- oder Nitrid-Oberflächen wie oben spezifiziert. Dadurch lassen sich einerseits SAM-Schichten herstellen, welche frei sind von unerwünschten Lösungsmittel-Verunreinigungen, andererseits ist es sehr einfach, wässrige Mischungen mehrerer Salze verschiedener funktionalisierter und / oder nicht funktionalisierter Organophosphate und / oder Organolphosphonate herzustellen.
Ein weiteres wichtiges und vorteilhaftes Kennzeichen des erfindungsgemässen Verfahrens ist also, dass die besagten Mono- bzw. Mehrfachschichten von Verbindungen der Formel (IA) und / oder (IB) frei von organischen Lösungsmitteln sind.

Ebenfalls beschrieben ist in der WO 98/29580 die Herstellung von SAM-Schichten aus wässriger Lösung der (löslichen) Organophosphat- oder Organophosphonat-Derivate sowie die Bildung von Natrium- und Kaliumsalzen der Organophosphate oder Organophosphonate direkt in wässriger Lösung (siehe oben). Die Unterschiede zu der vorliegenden Erfindung sind die folgenden:
1. Die Salze werden gemäss Beschreibung in der WO 98/29580 nicht isoliert, sondern *lediglich in situ* hergestellt. Dadurch entfällt die bevorzugte Reinigungsmöglichkeit durch Umkristallisation des Salzes vor der Anwendung als SAM Bildner. Zudem erlaubt diese Technik die sehr kontrollierte Einstellung der Stöchiometrie der zu verwendenden SAM Lösung.
2. Die Anwendungen betreffen völlig andere Anwendungsgebiete mit völlig anderen Anforderungen (Lackhaftung, Korrosionsschutz für lackierte Produkte) auf anderen Substratmaterialien (aus Zink, Aluminium, Magnesium oder deren Legierungen).

Als Substrate für die Anwendung der erfindungsgemässen Oberflächenbehandlungstechniken kommen folgende Materialien / Oberflächen in Frage: Oxide, Nitride oder Carbide von Tantal, Niob, Titan, Vanadium, Zirkon, Hafnium, Molybdän, Wolfram, Silizium oder deren Mischungen in Form massiver Körper oder als Schichten auf beliebigen Unterlagen verwendet werden. Insbesondere eignen sich Metalle oder Metallegierungen, welche an der Oberfläche eine natürliche, spontan sich bildende oder eine künstlich (z.B. durch Anodisation) hergestellte Oxidschicht aufweisen, wobei es sich vorzugsweise um die Metalle Titan, Tantal, Niob, Vanadium, Zirkon, Hafnium, Molybdän, Wolfram, Silizium bzw. Legierungen dieser Metalle bzw. Halbleiter handelt.

Von grosser Bedeutung sind dabei optisch transparente Oxide für Anwendungen im Bereich der optischen Biosensorik, z.B. für Sensoren basierend auf dem Prinzip der optische Wellenleitertechnik. Dazu gehören insbesondere, aber nicht ausschliesslich, Metalloxide mit hohem Brechungsindex wie Tantaloxid (Ta₂O₅), Nioboxid (Nb₂O₅), Titanoxid (TiO₂), Zirkonoxid (ZrO₂) sowie Mischungen dieser Oxide.

Im Bereich der Biomaterialien sind vor allem oxidkeramische Werkstoffe zu erwähnen, wie z.B. Aluminiumoxid (Al₂O₃) und Zirkonoxid (ZrO₂). Da metallische Werkstoffe mit biokompatiblen Eigenschaften an der Oberfläche praktisch immer eine natürliche Oxidschicht ausbilden (Passivschicht, verantwortlich für die Korrosionsbeständigkeit und Biokompatibilität), lässt sich die erfindungsgemässe Technik vorteilhaft auch auf eine Reihe von Implantatwerkstoffen anwenden. Zu erwähnen sind in diesem Bereich die Materialien Titan (mit TiO₂-Oberfläche), Niob (mit Nb₂O₅-Oberfläche), Zirkon (mit ZrO₂-Oberfläche) und Tantal (mit Ta₂O₅-Oberfläche). Im weiteren kann die Technik auf Legierungen angewendet werden, insbesondere auf Ti-Al-V, Ti-Al-Nb, Ti-Nb-Zr, Ti-Nb-Zr-Ta, Chromnickelstähle (Fe-Cr-Ni, Fe-Ni-Mo), Co-Cr und Co-Cr-Mo.

Während im Bereich der Sensorik oft (aber nicht ausschliesslich, ein Beispiel sind Kapillaren-basierende Sensoren) im wesentlichen planare, glatte Substrate verwendet werden, sind im Biomaterial- oder Implantatbereich oft rauhe oder topographisch gezielt strukturierte Oberflächen von Interesse, da diese in gewissen Einsatzfällen eine bevorzugte biologische Reaktion im Körper auslösen. Insbesondere bei metallischen Implantaten für den Knochenbereich (künstliche Hüftgelenke, Dentalimplantate, Osteosynthese-Platten und - schrauben) werden oft rauhe oder spezifisch aufgerauhte Oberflächen eingesetzt (Sittig 98). Solche Oberflächen mit komplexer Oberflächentopographie oder -Porosität bieten oft spezielle Schwierigkeiten, wenn es um die gezielte Einstellung chemischer Eigenschaften geht. Die erfindungsgemässe Technik der Bildung von SAM-Schichten basierend auf Alkylphosphaten, Alkylphosphonaten und deren funktionalisierten Derivaten (siehe auch weiter unten) eröffnet besonders interessante Möglichkeiten, solche komplexen, rauhen Metalloberflächen zu funktionalisieren, da die Technik genauso gut auf rauhe, strukturierte oder poröse Oberflächen von oxidbedeckten Metallen und Metalloxiden anwendbar ist. Dieses ist im Detail in Beispiel 2 ausgeführt.

Das erfindungsgemässe Verfahren zur Oberflächenmodifikation eignet sich also für Substrate, welche eine fast glatte Oberfläche mit niedriger Rauheit aufweisen oder auch rauhe Oberflächen besitzen, wobei diese Oberflächen gegebenenfalls eine zusätzliche Strukturierung aufweisen.

Insbesomdere für Anwendungen in der Biosensorik wird bevorzugt, dass als biochemische, biologische oder synthetische Erkennungselemente (an B oder Y angedockt) solche ausgewählt werden aus der Gruppe von Nukleinsäuren, wie z.B. DNA, RNA, Oligonukleotide, Nukleinsäureanaloge, wie z.B. PNA, mono- oder polyklonalen Antikörpern, Peptiden, Enzymen, Aptameren, synthetischen Peptidstrukturen, löslichen menbrangebundenen und aus einer Membran isolierten Proteinen, wie z.B. Rezeptoren, deren Liganden, Antigenen für Antikörper, Biotin, "Histidin-Tag-Komponenten" und deren Komplexbildungspartner.

Insbesondere für Anwendungen des erfindungsgemässen Verfahrens zur biologisch verträglichen Oberflächenmodifikation von Implantaten wird demgegegenüber bevorzugt, dass ein biologisch wirksames Erkennungselement Peptide, Proteine, Glycoproteine, Wachstumsfaktor, wie z.B. TGF-β oder BMP (Bone Morphogenic Protein) umfasst.

Mit dem erfindungsgemässen Verfahren ist es auch möglich, stark hydrophobe Oberflächen herzustellen, welche sich beispielsweise durch Kontakt mit Albuminen (beispielsweise Humanserumalbumin (HSA) oder Rinderserumalbumin (BSA)) zur Erreichung einer proteinresistenten Oberfläche passivieren lassen. Dieses kann für Anwendungen beispielsweise im Bereich der blutkontakrtierenden Devices wichtig sein (Blutkompatibilität, Verhinderung von Blutplättchenadsorption und Thrombusbildung).

Hierzu kann vorteilhaft an B oder Y eine Verbindung angelagert sein, welche der Substratoberfläche eine Resistenz gegen Proteinadsorption und / oder Zelladhäsion verleiht, wobei diese Verbindung vorzugsweise ausgewählt ist aus den Gruppen von Verbindungen, die von Oligo(ethylenoxid), Phosphorylcholin, Heparin, Sacchariden, Albuminen, insbesondere Rinderserumalbumin oder Humanserumalbumin, Casein, unspezifischen, polyklonalen oder monoklonalen, artfremden oder empirisch für den oder die nachzweisenden Analyten unspezifischen Antikörpern (insbesondere für Immunoassays), Detergentien - wie beispielsweise Tween 20 -, nicht mit zu analysierenden Polynukleotiden hybridisierender, fragmentierter natürlicher oder synthetischer DNA, wie beispielsweise ein Extrakt von Herings- oder Lachssperma (insbesondere für Polynukleotid-Hybridisierungsassays), oder auch ungeladenen, aber hydrophilen Polymeren, wie beispielsweise Polyethylenglycole oder Dextrane, gebildet werden. Gruppen wie Oligo(ethylenoxid) zeigen ein besonders gutes Verhalten in bezug auf die Proteinresistenz und wurden bei anderen Systemen, z.B. bei Thiol-basierten SAMs auf Goldoberflächen bereits beschrieben (P. Harder, M. Grunze, R. Dahint, G. M. Whitesides, P. E. Laibinis, J.Phys. Chem.B, 102 (1998), 426 - 436). Typische Anwendungen dienen beispielsweise der Verbesserung der Blutkompatibilität von Stents oder der Verhinderung von Blutplättchenadsorption und Thrombusbildung im Kontakt mit fliessendem Blut.

Es ist ausserdem auch möglich, dass Verbindungen der Formel (IA) und / oder (IB) verschiedene funktionelle Gruppen und / oder biologische oder biochemische oder synthetische Erkennungselemente und / oder Verbindungen zur Verleihung einer Resistenz der Oberfläche gegen Proteinadsorption und / oder Zelladhäsion in einem Moleküle im gleichen SAM-Molekül umfassen, z.B. eine Protein-resistente Oligo(ethylenoxid)-Gruppe in Kombination mit einem biologischen Erkennunsgelement, wie beispielsweise Biotin.

Im Sinne der Erfindung ist ausserdem möglich, dass auf einer ersten Monoschicht aus Organophosphaten und / oder Organophosphonaten eine zweite oder sequentiell noch weitere Monoschichten abgeschieden werden, so dass auf besagten Substratoberflächen eine Doppelschicht oder Mehrfachschicht erzeugt wird.

Eine mögliche Variante des Verfahrens ist dadurch gekennzeichnet, dass durch die erste Monoschicht aus Organophosphaten und / oder Organophosphonaten eine hydrophobe Oberfläche erzeugt wird, auf der eine weitere Schicht aus synthetischen oder natürlichen Lipiden abgeschieden wird.

Eine spezielle Ausführungsform ist dabei dadurch gekennzeichnet, dass die besagte weitere Schicht aus synthetischen oder natürlichen Lipiden aus einer Lipidvesikelsuspension durch spontane Anlagerung der Vesikel an die hydrophobe Oberfläche einer ersten Monoschicht aus Organophosphaten und / oder Organophosphonaten, gefolgt vom Ausspreiten der Vesikelmembran auf dieser ersten Monoschicht, erzeugt wird.

Die synthetischen oder natürlichen Lipide können ausgewählt sein aus der Gruppe, die von Phosphorglycerolipiden etc..... gebildet wird, oder eine Mischung dieser Moleküle umfassen. Dabei können mit der besagten weiteren Schicht molekulare Gruppen Y und / oder B und /oder biologische oder biochemische oder synthetische Erkennungselemente und / oder Verbindungen, die der Oberfläche eine Resistenz gegen Proteinadsorption und / oder Zelladhäsion verleihen, gemäss deren vorangehend beschriebenen Ausführungsformen, assoziiert sein.

Eine bevorzugte Ausführungsform des erfindungsgemässen Verfahrens ist dadurch gekennzeichnet, dass auf einer Mehrfachschicht mit einer ersten Monoschicht aus Organophosphaten und / oder Organophosphonaten auf einer Substratoberfläche synthetische oder natürliche Vesikel oder Mikrosomen immobilisiert werden, gegebenfalls mit damit assoziierten biologischen oder biochemischen oder synthetischen Erkennungselementen, welche ausgewählt sind aus der Gruppe, die von Nukleinsäuren (beispielsweise DNA, RNA, Oligonukleotiden) und Nukleinsäureanalogen (z. B. PNA), mono- oder polyklonalen Antikörpern, Peptiden, Enzymen, Aptameren, synthetischen Peptidstrukturen, löslichen, membrangebundenen und aus einer Membran isolierten Proteinen, wie beispielsweise Rezeptoren, deren Liganden, Antigenen für Antikörper, Biotin, "Histidin-Tag-Komponenten" und deren Komplexbildungspartnern gebildet wird.

Eine weitere Ausführungsform ist die Verwendung von Mischungen verschiedener SAM-Moleküle. Solche gemischten SAM-Schichten können dabei auf zwei Arten hergestellt werden: entweder durch Herstellung einer wässrigen Lösung, die beide SAM-Typen enthält, gefolgt von einer Behandlung der Oberfläche in dieser Mischung, oder aber durch sequentielle Adsorption unter Verwendung jeweils reiner SAM-Lösungen. Ein Beispiel für eine solche Verwendung von Mischungen verschiedener SAM-Moleküle in wässriger Lösung zur Bildung gemischter SAM-Systeme an der Oberfläche ist die Abscheidung aus einer Mischung von SAM-Molekülen, welche ein biologisches Erkennungselement wie beispielsweise Biotin enthalten und solchen SAM-Molekülen, welche eine Gruppe wie Oligo(ethylenoxid), umfassen, zur Herstellung einer Oberfläche mit Protein-resistentem Hintergrund bei gleichzeitiger Anwesenheit einer biologisch-spezifischen Funktion (Biotin).

Als gemischte SAMs werden solche bezeichnet, die mindestens zwei Moleküle in einem beliebigen Mischungsverhältnis enthalten, wobei die oben genannten Klassen von Molekülen in beliebiger Kombination vertreten sein können.

Ein weiterer Gegenstand der Erfindung ist daher ein Verfahren zur Abscheidung gemischter Mono- oder Mehrfachschichten aus Organophosphorsäuren der allgemeinen Formel I (A)

**Y-B-OPO₃ H₂** **(IA)**

und / oder aus Organophosphonsäuren der allgemeinen Formel I (B)

**Y-B-PO₃ H₂** **(IB)**

und deren Salzen, in denen B einen Alkyl-, Alkenyl-, Alkinyl-, Aryl-, Aralkyl-, Hetaryl- oder Hetarylalkylrest und Y Wasserstoff oder eine funktionelle Gruppe aus der Reihe Hydroxy, Carboxy, Amino, gegebenfalls durch Niederalkyl substituiertes Mono-oder Dialkylamino, Thiol, oder eine negative Säuregruppe aus der Reihe Ester, Phosphat, Phosphonat, Sulfat, Sulfonat, Maleimid, Succinimydyl, Epoxy, Acrylat bedeutet, wobei an B oder Y ein biologisches, biochemisches oder synthetisches Erkennungselement durch Additions- oder Substitutionsreaktion angedockt sein kann, wobei auch Verbindungen angelagert sein können, die der Substratoberfläche eine Resistenz gegen Proteinadsorption und/oder Zelladhäsion verleihen und in B in der Kette gegebenenfalls anstelle einer oder mehrerer-CH₂- Gruppen eine oder mehrere Ethylenoxidgruppen enthalten sein können, auf Substratoberflächen von reinen oder gemischten Oxiden, Nitriden oder Carbiden von Metallen und Halbleitern,
dadurch gekennzeichnet, dass zur Behandlung dieser Oberflächen, insbesondere als Oberflächen von Sensorplatten, Implantaten und medizinischen Hilfsgeräten, wasserlösliche Salze einer Verbindung der Formel (IA) bzw. (IB) in wässriger Lösung eingesetzt werden.

Wiederum wird bevorzugt, dass vor der Abscheidung besagter gemischter Mono- oder Mehrfachschichten das wasserlösliche Salz einer Verbindung der Formel (IA) und / oder (IB) in wässriger Lösung isoliert wird.

Auch im Falle gemischter SAM's ist das erfindungsgemässe Verfahren dadurch gekennzeichnet, dass die besagten gemischten Mono- bzw. Mehrfachschichten von Verbindungen der Formel (IA) und / oder (IB) frei von organischen Lösungsmittel sind.

Bevorzugt werden auch hier als Salze der Verbindungen der Formel (IA) und / oder (IB) insbesondere Natrium-, Kalium- und Ammoniumsalze verwendet.

Als Substrate eignen sich auch für diese Ausführungsform des erfindungsgemässen Verfahrens Oxide, Nitride oder Carbide von Tantal, Niob, Titan, Vanadium Zirkon, Hafnium, Molybdän, Wolfram, Silizium oder deren Mischungen in Form massiver Körper oder als Schichten auf beliebigen Unterlagen.

Eine bevorzugte Ausführungsform des erfindungsgemässen Verfahrens zur Abscheidung gemischter Mono- oder Mehrfachschichten ist dadurch gekennzeichnet, dass man Verbindungen der Formel (IA) und / oder (IB), in denen die Gruppen B und Y gemeinsam eine Alkylgruppe oder eine gegebenenfalls substituierte Alkylgruppe von 2 - 24 C-Atomen bedeuten, zur Beschichtung verwendet.

Insbesomdere für Anwendungen in der Biosensorik wird wiederum bevorzugt, dass als biochemische, biologische oder synthetische Erkennungselemente (an B oder Y angedockt) solche ausgewählt werden aus der Gruppe von Nukleinsäuren, wie z.B. DNA, RNA, Oligonukleotide, Nukleinsäureanaloge, wie z.B. PNA, mono- oder polyklonalen Antikörpern, Peptiden, Enzymen, Aptameren, synthetischen Peptidstrukturen, löslichen menbrangebundenen und aus einer Membran isolierten Proteinen, wie z.B. Rezeptoren, deren Liganden, Antigenen für Antikörper, Biotin, "Histidin-Tag-Komponenten" und deren Komplexbildungspartner.

Insbesondere für Anwendungen des erfindungsgemässen Verfahrens zur biologisch verträglichen Oberflächenmodifikation von Implantaten wird demgegegenüber auch hier bevorzugt, dass ein biologisch wirksames Erkennungselement Peptide, Proteine, Glycoproteine, Wachstumsfaktor, wie z.B. TGF-β oder BMP (Bone Morphogenic Protein) umfasst.

Mit dem erfindungsgemässen Verfahren zur Abscheidung gemischter Mono- oder Mehrfachschichten ist es auch möglich, stark hydrophobe Oberflächen herzustellen, welche sich beispielsweise durch Kontakt mit Albuminen (beispielsweise Humanserumalbumin (HSA) oder Rinderserumalbumin (BSA)) zur Erreichung einer proteinresistenten Oberfläche passivieren lassen.

Hierzu kann wiederum vorteilhaft an B oder Y eine Verbindung angelagert sein, welche der Substratoberfläche eine Resistenz gegen Proteinadsorption und / oder Zelladhäsion verleiht, wobei diese Verbindung vorzugsweise ausgewählt ist aus den Gruppen von Verbindungen, die von Oligo(ethylenoxid), Phosphorylcholin, Heparin, Sacchariden, Albuminen, insbesondere Rinderserumalbumin oder Humanserumalbumin, Casein, unspezifischen, polyklonalen oder monoklonalen, artfremden oder empirisch für den oder die nachzweisenden Analyten unspezifischen Antikörpern (insbesondere für Immunoassays), Detergentien - wie beispielsweise Tween 20 -, nicht mit zu analysierenden Polynukleotiden hybridisierender, fragmentierter natürlicher oder synthetischer DNA, wie beispielsweise ein Extrakt von Herings- oder Lachssperma (insbesondere für Polynukleotid-Hybridisierungsassays), oder auch ungeladenen, aber hydrophilen Polymeren, wie beispielsweise Polyethylenglycole oder Dextrane, gebildet werden.

Es ist ausserdem auch möglich, dass Verbindungen der Formel (IA) und / oder (IB) verschiedene funktionelle Gruppen und / oder biologische oder biochemische oder synthetische Erkennungselemente und / oder Verbindungen zur Verleihung einer Resistenz der Oberfläche gegen Proteinadsorption und / oder Zelladhäsion in einem Moleküle im gleichen SAM-Molekül umfassen.

Wiederum können die Substrate eine glatte Oberfläche mit niedriger Rauheit aufweisen oder rauhe Oberflächen besitzen, wobei diese Oberflächen gegebenenfalls eine zusätzliche Strukturierung aufweisen.

Ein besonderes Kennzeichen des erfindungsgemässen Verfahrens zur Herstellung gemischter SAM's ist, dass es die Möglichkeit schafft, durch Wahl des Mischungsverhältnisses die Hydrophilizität oder Hydrophobizität der Oberfläche zu kontrollieren. Dieses eröffnet die Möglichkeit für eine kontrollierte Einstellung der Benetzbarkeit der Oberfläche (Kontaktwinkel gegen Wasser). Die Benetzbarkeit ist ein wichtiges Merkmal im Bereich der Biokompatibilität. Für bestimmte Anwendungen kann es vorteilhaft sein, mittlere Kontaktwinkel einzustellen. Als Ausführungsbeispiel wird hierzu später die Herstellung und Charakterisierung eines gemischten SAM aus Dodecylphosphat/ω-Hydroxy-dodecylphosphat beschrieben (siehe Beispiel 1).

Ein weiteres Kennzeichen ist, dass durch Wahl des Mischungsverhältnisses eine kontrollierte Dichte von positiven und / oder negativen Ladungen auf der Oberfläche eingestellt werden kann. Die Oberflächenladung spielt eine grosse Rolle in Bezug auf die Wechselwirkung mit biologischen Zellen (J. E. Davies, B. Causton, Y. Bovell, K. Davy, C. S. Sturt, "The Migration of Osteoblasts Over Substrata of Discrete Surface-Charge", Biomaterials 7 (1986), 231 - 233; R. M. Shelton, I. M. Whyte, J. E. Davies, "Interaction between Primary Bone Cell and Biomaterials. Part 4: Colonization of Charged Polymer Surfaces", Biomaterials and clinical applications: proceedings of the Sixth European Conference on Biomaterials, Bologna, Italy, September 14-17, 1986; Elsevier (1987), 597 - 602). Als System kommen z.B. in Frage: Organophosphate oder -phosphonate mit endständiger Amingruppe (positiv geladen beim Körper-pH von 7.4) oder Organophosphate oder -phosphonate mit ω-endständiger negativ geladener chemisch-funktioneller Gruppe, wie Phosphat oder Phosphonat, Sulfat oder Sulfonat, Carboxylat, etc.

Ausserdem ermöglicht das erfindungsgemässe Verfahren, dass durch Wahl des Mischungsverhältnisses eine kontrollierte Dichte von reaktiven Gruppen und / oder biochemischen Erkennungselementen oder biologischen "Funktionen" eingestellt werden kann.

Da bei Verwendung der wässrigen Lösungen der Organophosphate oder -phosphonate selektive Adsorptionseigenschaften beobachtet werden (siehe Beispiel 1), erlaubt diese Technik in einem einzigen Behandlungsschritt die Herstellung von Oberflächen, welche nur lokal mit dem SAM belegt sind, während andere Zonen der Oberfläche unbeschichtet bleiben.

Ein weiterer Gegenstand der Erfindung ist daher ein Verfahren zur Erzeugung von chemisch strukturierten Oberflächen durch lokale Abscheidung von Mono- oder Mehrfachschichten aus Organophosphaten und / oder Organophosphonaten auf Substratoberflächen von reinen oder gemischten Oxiden, Nitriden oder Carbiden von Metallen oder Halbleitern, dadurch gekennzeichnet, dass zur Behandlung der Oberflächen wasserlösliche salzartige Verbindungen der zugehörigen Organophosphorsäure oder Organophosphonsäure eingesetzt werden und dass die Abscheidung der reinen oder gemischten Mono- oder Mehrfachschichten mittels einer der vorgenannten Ausführungsformen des Verfahrens erfolgt.

Insbesondere zeigt Siliziumoxid praktisch keine Tendenz der Adsorption von Organophosphaten oder -phosphonaten aus wässriger Lösung. Damit können chemisch strukturierte Oberflächen, wie sie z.B. durch lithographische oder andere Maskentechniken herstellbar sind, zur gezielten Produktion von Oberflächen genutzt werden, welche gezielt unterschiedliche chemische Eigenschaften aufweisen (siehe Beispiel 1). Diese Technik ist von Nutzen sowohl für die chemische Strukturierung von Biomaterial- oder Implantatoberflächen, um z.B. die Adsorption von Proteinen, Wachstumsfaktoren, bzw. die Adhäsion von Zellen lokal zu steuern und damit Einfluss auf die spezifische Antwort der biologischen Umgebung (in vitro oder im Körper) auf die Oberfläche des Fremdmaterials zu nehmen, als auch für die Strukturierung der Oberflächen von Biosensoren, mit entsprechenden lokal veränderlichen Eigenschaften.

Eine bevorzugte Variante des Verfahrens besteht daher darin, dass die Substratoberfläche ein definiertes Muster mit Siliziumoxid bzw. Übergangsmetalloxiden aufweist. Eine Weiterentwicklung dieser Variante ist dadurch gekennzeichnet, dass auf den Siliziumoxidbereichen eine weitere Abscheidung von Mono- oder Mehrfachschichten aus Organophosphaten und / oder Organophosphonaten in wässriger Lösung durchgeführt wird.

Schließlich eignet sich die Technik für die partielle Beschichtung von Teilen. Dieses ist z.B. von Interesse im medizinischen Implantatbereich, wo oft an verschiedene Bereiche des Implantates unterschiedliche Anforderungen gestellt werden. Beispielsweise weisen Dental-Wurzelimplantate Zonen auf, die nach der Implantation in Kontakt mit Knochengewebe stehen, während andere Zonen des gleichen Implantates in Kontakt mit dem Zahnfleisch stehen. Diese beiden Zonen können mit der erfindungsgemässen Technik so modifiziert werden, dass diese Zonen eine auf das lokale Anforderungsprofil hin optimierte Oberflächenzusammensetzung aufweisen Die unterschiedlichen SAM-Schichten können lokal selektiv aufgebracht werden, z.B. durch partielles Tauchen, Aufpinseln, Aufstreichen, Aufdrucken oder mittels Inkjet-Techniken.

Für die Aufbringung der Mono- oder Mehrfachschichten insbesondere auf planaren Substraten gibt es eine Vielzahl einsatzbarer Methoden. Es wird bevorzugt, dass die Abscheidung der Mono- oder Mehrfachschichten, gegebenenfalls lokal selektiv, unter Verwendung einer Abscheidungsmethode erfolgt, die ausgewählt ist aus der Gruppe, die von Tauchen, Aufstreichen, Aufpinseln, "Ink jet spotting", mechanischem Spotting mittels Stift, Feder oder Kapillare, "micro contact printing", fluidischer Kontaktierung der Substratoberfläche mit parallelen oder gekreuzten Mikrokanälen, unter Einwirkung von Druckunterschieden oder elektrischen oder elektromagnetischen Potentialen" etc. gebildet wird.

Eine spezielle Ausführungsform des erfindungsgemässen Verfahrens zur Erzeugung von chemisch strukturierten Oberflächen ist dadurch gekennzeichnet, dass durch lokale Abscheidung von Mono- oder Mehrfachschichten aus Organophosphaten und / oder Organophosphonaten auf Substratoberflächen von reinen oder gemischten Oxiden, Nitriden oder Carbiden von Metallen oder Halbleitern lokale hydrophile oder hydrophobe Bereiche erzeugt werden und deren umgebende Substratoberfläche anschliessend mit einer endständig hydrophoben beziehungsweise hydrophilen Monoschicht abgedeckt wird.

Eine Weiterentwicklung besteht darin Verfahren, dass auf einer nach dem erfindungsgemässen Verfahren erzeugten chemisch strukturierten Oberfläche sequentiell eine oder mehrere Monoschichten wie vorangehend beschrieben lokal abgeschieden werden und damit lokal Doppel- oder Multischichten erzeugt werden.

Wie vorangehend erläutert, eignet sich das erfindungsgemässe Abscheidungsverfahren insbesondere zur Herstellung von Substratoberflächen in zwei unterschiedlichen Anwendungsgebieten. Ein bevorzugter Gegenstand der Erfindung betrifft die Herstellung von Implantatoberflächen mit Implantaten aus oxidbedeckten Metallen, wie z.B. Titan, Tantal, Niob, Legierungen wie Titan-Aluminium-Vanadium, Titan-Aluminium-Niob, Titan-Niob-Zirkon, Titan-Niob-Zirkon-Tantal, Cobalt-Chrom, Cobalt-Chrom-Molybdän, Eisen-Nickel-Chrom, wobei auf der Oberfläche die Abscheidung von reinen oder gemischten Mono- oder Mehrfachschichten aus Organophosphaten und / oder Organophosphonaten nach einer der vorgenannten Ausführungsformen erfolgt.

Ein weiterer Gegenstand der Erfindung ist ein Verfahren zur Herstellung von Sensorplattformoberflächen, dadurch gekennzeichnet, dass auf der Oberfläche die Abscheidung von reinen oder gemischten Mono- oder Mehrfachschichten aus Organophosphaten und / oder Organophosphonaten nach einer der vorgenannten Ausführungsformen erfolgt.

Die Erfindung umfasst auch ein Implantat mit einer Mono- oder Mehrfachschicht aus Organophosphaten und / oder Organophosphonaten als Oberfläche, dadurch gekennzeichnet, dass die Erzeugung der Oberfläche mit einem Abscheidungsverfahren nach einer der vorgenannten Ausführungsformen erfolgt.

Das erfindungsgemässe Implantat kann ausgewählt sein aus der Gruppe von Wurzelimplantaten für den Dentalbereich, künstlichen Prothesen, wie z.B. Hüftgelenkschäften, -kugeln bzw. -pfannen, künstlichen Kniegelenken, Osteosynthesekomponenten, wie z.B. Knochenplatten, Schrauben, "fixateur externe", Komponenten zur Reparatur von Schäden im Schädelbereich ("maxillofacial devices"), Komponenten im Bereich der Rückgratchirurgie ("spinal surgery implants"), Stents, Herzschrittmacherkomponenten.

Weiterer Gegenstand der Erfindung sind medizinische Hilfsgeräte aus Metallen oder Keramik mit einer Mono- oder Mehrfachschichtschicht aus Organophosphaten und / oder Organophosphonaten als Oberfläche, dadurch gekennzeichnet, dass die Erzeugung der Oberfläche mit einem Abscheidungsverfahren nach einer der vorgenannten Ausführungsformen erfolgt.

Die erfindungsgemässen medizinischen Hilfsgeräte aus Metallen oder Keramik können ausgewählt sein aus der Gruppe umfassend Katheter, Ballonkatheter, Endoskope, Komponenten für körperexterne, blutführende Systeme wie z.B. Herz-Kreislaufmaschinen.

Weiterer Gegenstand der Erfindung ist eine Sensorplattform mit einer Mono- oder Mehrfachschicht aus Organophosphaten und / oder Organophosphonaten als Oberfläche, dadurch gekennzeichnet, dass die Erzeugung der Oberfläche mit einem Abscheidungsverfahren nach einer der vorgenannten Ausführungsformen erfolgt.

Es wird bevorzugt, dass die Sensorplattform mindestens ein Array von in diskreten Messbereichen immobilisierten biologischen oder biochemischen oder synthetischen Erkennungselementen zur spezifischen Erkennung und / oder Bindung eines oder mehrerer Analyten und / oder spezifischen Wechselwirkung mit besagten Analyten umfasst.

Viele mögliche Ausführungsformen der erfindungsgemässen Sensorplattform zeichnen sich dadurch aus, dass der Nachweis des einen oder der mehreren Analyten mithilfe von Labeln erfolgt, welche ausgewählt sind aus der Gruppe, die von beispielsweise Lumineszenzlabeln, insbesondere lumineszenten Interkalatoren oder "molecular beacons", Absorptionslabeln, Massenlabeln, insbesondere Metallkolloiden oder Plastikbeads, Spin-Labeln, wie ESR- oder NMR-Labeln, radioaktiven Labeln gebildet wird.

Eine mögliche Variante ist dadurch gekennzeichnet, dass der Analytnachweis auf der Bestimmung einer Änderung des effektiven Brechungsindex aufgrund molekularer Adsorption oder Desorption auf den Messbereichen beruht.

Eine Untervariante besteht darin, dass der Analytnachweis auf der Bestimmung einer Änderung der Bedingungen zur Erzeugung eines Oberflächenplasmons in der Metallschicht eines Mehrschichtsystems beruht, wobei die Metallschicht vorzugsweise aus Gold oder Silber besteht.

Eine bevorzugte Ausführungsform der erfindungsgemässen Sensorplattform ist dadurch gekennzeichnet, dass der Analytnachweis auf der Bestimmung einer Änderung einer oder mehrerer Lumineszenzen beruht.

Eine mögliche Variante zeichnet sich dadurch aus, dass das Anregungslicht in einer Auflichtanregung eingestrahlt wird.

In Abhängigkeit von der spezifischen Ausführungsform werden solche Varianten bevorzugt, welche dadurch gekennzeichnet sind, dass das mit den Messbereichen in Kontakt stehende Material der Sensorplattform innerhalb einer Tiefe von mindestens 200 nm von den Messbereichen bei mindestens einer Anregungswellenlänge transparent oder absorbierend ist.

Eine andere mögliche Ausführungsform ist so gestaltet, dass das Anregungslicht in einer Transmissionsanordnung eingestrahlt wird. Für diese Ausführungsform ist es notwendig, dass das Material der Sensorplattform bei mindestens einer Anregungswellenlänge transparent ist.

Eine bevorzugte Ausführungsform der erfinungsgemässen Sensorplattform ist dadurch gekennzeichnet, dass die Sensorplattform als ein optischer Wellenleiter ausgebildet ist, welcher vorzugsweise im wesentlichen planar ist.

Es wird bevorzugt, dass die Sensorplattform ein optisch transparentes Material aus der Gruppe umfasst, die von Silikaten, z. B. Glas oder Quarz, transparenten thermoplastischen oder spritzbaren Kunststoff, beispielsweise Polycarbonat, Polyimid, Acrylaten, insbesondere Polymethylmethacrylat, oder Polystyrolen gebildet wird.

Eine besonders bevorzugte Ausführungsform der erfindungsgemässen Sensorplattform ist dadurch gekennzeichnet, dass diese einen optischen Dünnschichtwellenleiter mit einer bei mindestens einer Anregungswellenlänge transparenten Schicht (a) auf einer bei mindestens dieser Anregungswellenlänge ebenfalls transparenten Schicht (b) mit niedrigerem Brechungsindex als Schicht (a) umfasst.

Verschiedene Ausführungsformen derartiger Sensorplattformen und Verfahren zum Nachweis einer oder mehrerer Analyten unter Verwendung solcher Sensorplattformen sind ausführlich beispielsweise in den Patenten US 5,822,472, US 5,959,292 und US 6,078,705 sowie in den Patentanmeldungen WO 96/35940, WO 97/37211, WO 98/08077, WO 99/58963, PCT/EP 00/04869 und PCT/EP 00/07529 beschrieben. Die darin beschriebenen Ausführungsformen von Sensorplattformen, deren Oberfläche nach dem erfindungsgemässen Verfahren modifiziert wurde, und Verfahren zum Nachweis einer oder mehrerer Analyten unter Verwendung solcher modifizierter Sensorplattformen sind ebenfalls Gegenstand der vorliegenden Erfindung.

Weiterer Gegenstand der Erfindung ist ein Verfahren zum gleichzeitigen qualitativen und / oder quantitativen Nachweis einer Vielzahl von Analyten, dadurch gekennzeichnet, dass eine oder mehrere auf besagte Analyten zu untersuchende flüssige Proben mit den Messbereichen auf einer erfindungsgemässen Sensorplattform in Kontakt gebracht und resultierende Änderungen von Signalen aus den Messbereichen gemessen werden.

Es wird bevorzugt, dass der Analytnachweis auf der Bestimmung der Änderung einer oder mehrerer Lumineszenzen beruht.

Eine mögliche Ausführungsform des erfindungsgemässen Verfahrens besteht darin, dass das Anregungslicht von einer oder mehreren Anregungslichtquellen in einer Auflichtanregung eingestrahlt wird.

Eine andere mögliche Ausführungsform ist dadurch gekennzeichnet, dass das Anregungslicht von einer oder mehreren Anregungslichtquellen in einer in einer Transmissionsanordnung eingestrahlt wird.

Es wird bevorzugt, dass die Sensorplattform als ein optischer Wellenleiter ausgebildet ist, welcher vorzugsweise im wesentlichen planar ist, und dass das Anregungslicht von einer oder mehrerer Lichtquellen eingekoppelt wird in den optischen Wellenleiter mittels eines Verfahrens, welches ausgewählt ist aus der Gruppe, die von Stirnflächenkopplung, Einkopplung über angebrachte optische Fasern als Lichtleiter, Prismenkopplung, Gitterkopplung oder evaneszenter Kopplung durch Überlappung des evaneszenten Feldes besagten optischen Wellenleiters mit dem evaneszenten Feld eines weiteren, damit in Nahfeldkontakt gebrachten Wellenleiters gebildet wird.

Die Zugabe der einen oder mehreren Proben und der im Nachweisverfahren einzusetzenden Nachweisreagentien kann sequentiell in mehreren Schritten erfolgen. Es wird bevorzugt, dass die eine oder mehreren Proben mit einer Mischung aus den verschiedenen Nachweisreagentien zur Bestimmung der in besagten Proben nachzuweisenden Analyten vorinkubiert werden und diese Mischungen dann in einem einzigen Zugabeschritt den dafür vorgesehenen Arrays auf der Sensorplattform zugeführt werden.

Weiterer Gegenstand einer erfindungsgemässen Ausführungsform des Verfahrens ist, dass die Kalibration von infolge der Bindung eines oder mehrerer Analyten oder infolge der spezifischen Wechselwirkung mit einem oder mehreren Analyten im Nahfeld der Schicht (a) erzeugten Lumineszenzen die Zugabe von einer oder mehreren Kalibrationslösungen mit bekannten Konzentrationen besagter zu bestimmender Analyten auf die gleichen oder andere Messbereiche oder Segmente von Messbereichen oder Arrays von Messbereichen auf einer Sensorplattform umfasst, denen im gleichen oder einem separaten Zugabeschritt die eine oder die mehreren zu untersuchenden Proben zugeführt werden.

Bestandteil der Erfindung ist ein Verfahren nach einer der vorgenannten Ausführungsformen zur gleichzeitigen oder sequentiellen, quantitativen oder qualitativen Bestimmung eines oder mehrerer Analyten aus der Gruppe von Antikörpern oder Antigenen, Rezeptoren oder Liganden, Chelatoren oder "Histidin-tag-Komponenten", Oligonukleotiden, DNA- oder RNA-Strängen, DNA- oder RNA-Analoga, Enzymen, Enzymcofaktoren oder Inhibitoren, Lektinen und Kohlehydraten.

Mögliche Ausführungsformen des Verfahrens sind auch dadurch gekennzeichnet, dass die zu untersuchenden Proben natürlich vorkommende Körperflüssigkeiten wie Blut, Serum, Plasma, Lymphe oder Urin oder Eigelb oder optisch trübe Flüssigkeiten oder Gewebeflüssigkeiten oder Oberflächenwasser oder Boden- oder Pflanzenextrakte oder Bio- oder Syntheseprozessbrühen oder aus biologischen Gewebeteilen oder aus Zellkulturen oder -extrakten entnommen sind.

Weiterer Gegenstand der Erfindung ist die Verwendung einer erfindungsgemässen Sensorplattform und / oder eines erfindungsgemässen analytischen Systems und / oder eines erfindungsgemässen Verfahrens zu quantitativen oder qualitativen Analysen zur Bestimmung chemischer, biochemischer oder biologischer Analyten in Screeningverfahren in der Pharmaforschung, der Kombinatorischen Chemie, der Klinischen und Präklinischen Entwicklung, zu Echtzeitbindungsstudien und zur Bestimmung kinetischer Parameter im Affinitätsscreening und in der Forschung, zu qualitativen und quantitativen Analytbestimmungen, insbesondere für die DNA- und RNA-Analytik, für die Erstellung von Toxizitätsstudien sowie für die Bestimmung von Gen- oder Protein-Expressionsprofilen sowie zum Nachweis von Antikörpern, Antigenen, Pathogenen oder Bakterien in der pharmazeutischen Protduktentwicklung und -forschung, der Human- und Veterinärdiagnostik, der Agrochemischen Produktentwicklung und -forschung, der symptomatischen und präsymptomatischen Pflanzendiagnostik, zur Patientenstratifikation in der pharmazeutischen Produktentwicklung und für die therapeutische Medikamentenauswahl, zum Nachweis von Pathogenen, Schadstoffen und Erregern, insbesondere von Salmonellen, Prionen, Viren und Bakterien, in der Lebensmittel- und Umweltanalytik.

In den nachfolgenden Ausführungsbeispielen wird die Erfindung beispielhaft erläutert.

### Beispiel 1: Herstellung von Dodecylphosphat SAM und Hydroxy-dodecylphosphat SAM sowie von gemischten SAMs auf Metalloxidsubstraten aus wässriger Lösung der entsprechenden Ammoniumsalze

### 1.1 Ziel und Aufgabenstellung

Das Ziel dieses Ausführungsbeispiels ist zu zeigen, dass die erfindungsgemässe Anwendung von Ammoniumsalzen von Alkylphosphaten in wässriger Lösung die gezielte Modifikation von verschiedenen oxidischen Oberflächen erlaubt. Ferner wird am Beispiel des Ammoniumsalzes der Hydroxy-terminierten Dodecylphosphorsäure bewiesen, dass die Erfindung auch bei endständig funktionalisierten Alkylphosphaten die Herstellung wohldefinierter SAM-Schichten erlaubt. Ferner wird gezeigt, dass die Anwendung von Mischungen verschiedener Alkylphosphate die gezielte, "stufenlose" Feineinstellung von Oberflächenzuständen erlaubt, was sowohl für die Funktionalisierung der Oberflächen von Sensorplattformen ("Biosensor-Chips") als auch von Implantatoberflächen und deren Funktionsfähigkeit von entscheidender Bedeutung ist.

### 1.2 Materialien and Methoden

### 1.2.1 Substrate

Ta₂O₅, Nb₂O₅: Glassplättchen (15 x 15 x 1 mm) wurden mit einer 150-nm dicken Schicht von Ta₂O₅ oder Nb₂O₅ beschichtet (Balzers AG, Balzers, Liechtenstein).

Ti_{0.4}Si_{0.6}O₂, Fe₂O₃, ZrO₂, SiO₂: AF45 Glasträger (8 x 12 x 1 mm) wurden mit einer 200-nm dicken Ti_{0.4}Si_{0.6}O₂ Schicht belegt. Für die Untersuchungen der Fe₂O₃, ZrO₂ and SiO₂ Oberflächen wurde eine zusätzliche Schicht (Dicke: 14 nm) des entsprechenden Metalloxids als äusserste Schicht abgeschieden (Microvacuum, Ltd., Budapest, Ungarn).

TiO₂: Glasträger wurden mittels Sputtering mit einer 100 nm dicken Schicht von TiO₂ belegt (Paul Scherrer Institut, Villigen, Schweiz).

Al₂O₃: Al-Proben (99.9 % Reinheit) von 1 mm Dicke wurden bei einer Spannung von 25 V in einem Sperrschichtelektrolyten anodisiert, was zu einer Oxidschichtdicke von ca. 30 nm führt (Alusuisse Technology Center, Neuhausen am Rheinfall, Switzerland).

### 1.2.2 Alkylphosphate

### a) DDPO₄(NH₄)₂:

### Fällung des Ammoniumsalzes

2.00 g Dodecylphosphat (DDPO₄) (technische Qualität, Aldrich) wurden in 200 ml 2-Propanol (UVASOL, Merck) gelöst, auf 82°C aufgeheizt und unter Rückfluss gekocht. Dann wurden 6 ml Ammoniak (25% aq., p.a., Merck) zugefügt. Nach Abkühlung in Eiswasser wurde das gefällte Ammoniumsalz von DDP filtriert, mit Eiswasser gewaschen und bei 60°C and 10 mbar Vakuum während 20 h getrocknet. 61 g eines weissen Pulvers (Smp: 225°) wurden isoliert, was einer Ausbeute von 71% entspricht.

### Kontrolle mittels ¹H-NMR

(DMSO or CD₃OD): 0.88 ppm (t, 3H, -(CH₂)ₙC**H**₃), 1.28 ppm (m, 18H, -CH₂C**H**₂(CH₂)₉CH₃), 1.54 ppm (m, 2H, -CH₂C**H**₂(CH₂)₉CH₃), 3.72 ppm (q, 2H, -OC**H**₂CH₂(CH₂)₉CH₃), 4.9 (s, 8H, N**H**₄). Das ³¹P-NMR besteht aus einem einzelnen Peak, was auf eine reine Verbindung hinweist.

### Elementaranalyse

### Berechnet als Monoammoniumsalz: [C] 50.87%, [H] 10.67%, [N] 4.94% [O] 22.59%, [P] 10.93%

### Experimentelle Analyse: [C] 50.61%, [H] 10.94%, [N] 4.95% [O] 22.75%, [P] 10.69%

### b) OH-DDPO₄(NH₄)₂:

### Füllung des Ammoniumsalzes

500 mg Hydroxydodecylphosphat (OH-DDPO₄) wurden in 20 ml 2-Propanol (LTVASOL, Merck) gelöst, und NH₃-Gas wurde durch die Lösung während 5 Minuten geleitet. Das gefällte Ammoniumsalz von OH-DDPO₄ wurde duch Zentrifugieren isoliert, gewaschen und in einem Gasstrom von trockenem Stickstoff getrocknet. 515 mg weisses Pulver wurden isoliert (Ausbeute: 91%).

### Kontrolle mittels ¹H-NMR

(DMSO or CD₃OD): 1.24 ppm (m, 16H, -CH₂CH₂(C**H**₂)₈CH₂CH₂OH), 1.38 ppm (m, 2H, -CH₂CH₂(C**H**₂)₈CH₂CH₂OH), 1.44 ppm (m, 2H, -CH₂C**H**₂(CH₂)₁₀OH), 3.35 ppm (t, 2H, -(CH₂)ₙC**H**₂OH), 3.57 ppm (q, 2H, -OC**H**₂CH₂(CH₂)₁₀OH), 5.2 (s, 8H, N**H**₄).

### Elementaranalyse

### Berechnet als Diammoniumsalz: [C] 45.6%, [H] 10.5%, [N] 8.9%

### Experimentelle Analyse: [C] 44.1%, [H] 9.6%, [N] 5.7%

Die Elementaranalyse zeigt, dass im Vergleich mit dem Diammoniumsalz der Stickstoffgehalt etwas niedriger als erwartet ist. Dieses dürfte auf einen gewissen Verlust von Ammoniak und Konversion in das Monoammoniumsalz zurückzuführen sein. Die NMR-Spektren, die keine nicht-identifizierbaren Peaks enthalten, lassen darauf schliessen, dass keine Verunreinigungen in höheren Konzentrationen vorhanden sind.

### 1.2.3 Herstellung der Behandlungslösung

A) 150 mg von DDPO₄(NH₄)₂ wurden in 5 ml Reinstwasser gelöst, wobei die Lösung leicht auf 50°C erwärmt wurde. Das Volumen wurde auf 100 ml ergänzt.
B) 158 mg von OH-DDPO₄(NH₄)₂ werden in 5 ml Reinstwasser durch Erwärmen auf ca. 80 °C gelöst. Die Lösung wurde durch 0.22 µm Filter (MILLEX-GV, MILLIPORE, Bedford, MA) filtriert und das Volumen auf 100 ml gebracht.

Die beiden Lösungen a) and b) wurden in den verschiedenen Verhältnissen gemischt, von 0 bis 100 vol.-% in Bezug auf OH-DDPO₄(NH₄)₂. 11 verschiedene Lösungen, deren Gehalt an OH-DDPO₄(NH₄)₂ jeweils um 10% erhöht wurde, wurden hergestellt. Der Gesamtgehalt an Phosphat wurde dabei bei 0.5 mM konstant gehalten.

### 1.2.4 Probenbehandlung

Die Substrate für die Oberflächenmodifikationsversuche wurden für 15 Min in Reinstwasser und anschliessend 15 Min in 2-Propanol unter Einwirkung von Ultraschall gereinigt. Nach dem Trocknen wurden sie während 3 Min einer Sauerstoff-Plasmabehandlung (Harrick Plasma Cleaner/Sterilizer PDC-32G, Ossining, NY, USA) unterzogen. Dann wurden sie in ein Glasgefäss gebracht, in das anschliessend die wässrige Lösung des Alkylphosphats zugefügt wurde. Nach 48 h Behandlungszeit wurden die Proben entnommen, mit Wasser gespült und im Stickstoffstrom getrocknet.

### 1.3 Resultate

### a) DDPO₄ auf verschiedenen Substraten

Die Alkylphosphate wurden auf die folgenden Oberflächen angewendet: Al₂O₃, Ta₂O₅, Nb₂O₅, ZrO₂, Fe₂O₃, TiO₂, Ti_{0.4}Si_{0.6}O₂ und SiO₂. Die Behandlung erfolgte in 0.5 mM DDPO₄(NH₄)₂, wie im vorangegangenen Abschnitt ausgeführt.

### 1. Kontaktwinkel

Die (fortschreitenden, "advancing") Kontaktwinkel sind in Tab. 1 zusammengestellt. Die Resultate zeigen, dass hoch hydrophobe, selbstorganisierte Monoschichten auf allen Metalloxiden gebildet werden. Ein Kontaktwinkel von ≥ 110° ist typisch für einwandfreie SAMs. Ausnahmen sind Siliziumoxid (SiO₂) and TiO_{0.6}Si_{0.4}O₂. Der isolelektrische Punkt spielt dabei keine sichtbare Rolle; er variiert von 2.7 - 3.0 für Ta₂O₅ bis 7.0 - 8.6 für Fe₂O₃ (Tab. 1). Auf der SiO₂-Oberfläche bleibt der Kontaktwinkel im Bereich der Werte vor der Behandlung, d.h. die Oberfläche bleibt vollständig hydrophil. Damit ist klar, dass diese Oberfläche nicht mit den Alkylphosphaten reagiert und keine SAM-Schicht bildet.
Die Ti_{0.4}Si_{0.6}O₂-Schicht auf den Glas Chips besteht aus einer heterogenen Struktur von TiO₂ und SiO₂. Die Ti_{0.4}Si_{0.6}O₂-Oberflächen bilden in der Behandlungslösung eine unvollständige DSAM Schicht aus, indem der Kontaktwinkel zwar ansteigt, aber lediglich Werte von ca. 64° erreicht. Dieses stimmt mit der Beobachtung, dass SAMs zwar auf TiO₂, aber nicht auf SiO₂ gebildet werden, überein.

| Substrate | IEP | Ref (IEP) | KW (± Standardabw.) | MTD (± Standardabw.) |
|---|---|---|---|---|
| | | | | |
| Ta₂O₅ | 2.7 - 3.0 | 14 | 114.6 ± 0.48 | 129 ± 19 |
| Al₂O₃ | 7.5 - 8.0 | 15 | 111.4 ± 1.07 | 152 ± 22 |
| Nb₂O₅ | 3.4 - 3.6 | 16 | 109.7 ± 0.63 | 120 ± 18 |
| ZrO₂ | 4.0 | 17 | 110.1 ± 0.61 | 258 ± 39 |
| Fe₂O₃ | 7.0 - 8.6 | 18/19 | 110.8 ± 0.64 | 197 ± 30 |
| Ti_{0.4}Si_{0.6}O₂ | 3.6 | 20 | 63.8 ± 1.93 | 334 ± 50 |
| TiO₂ | 4.7 - 6.2 | 21 | 111.4 ± 1.18 | 221 ± 33 |
| SiO₂ | 1.8 - 2.2 | 22 | 10.0 ± 3.42 | > 3000 |

**Tab. 1**. Literaturwerte der isoelektrischen Punkte (IEP) der verwendeten Metalloxide, experimentell gemessene Kontaktwinkel (KW) nach der Behandlung mit wässriger DDP0₄(NH₄)₂-Lösung sowie Mikrotropfendichte (MTD), gemessen nach Bildung der Kondensationsfiguren an denselben, mit DDPO₄(NH₄)₂ behandelten Oberflächen.

### 2. Mikrotropfendichte

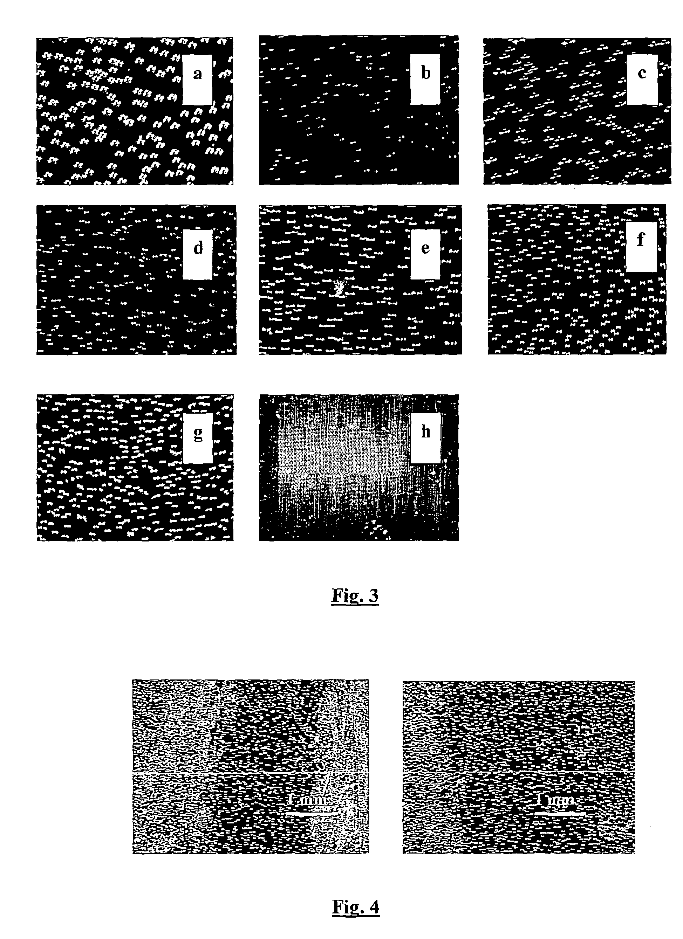

DDPO₄-SAMs auf den reinen Metalloxiden zeigen nach der Ausbildung des Kondensationsfilmes eine sehr niedrige Mikrotropfendichte von 120 - 260 Tropfen/mm² (Tab. 1) und eine homogene Verteilung der Tropfen über die Oberfläche. SiO₂ bleibt hydrophil, und es bildet sich ein durchgehender Wasserfilm, da die hohe Dichte an Mikrotropfen rasch zur Koaleszenz führt. Auf der DDPO₄-bedeckten Ti_{0.4}Si_{0.6}O₂ Oberfläche bildete sich eine MDT von ca. 350 Tropfen/mm² aus. Dieser Wert ist erwartungsgemäss höher als auf den SAM-bildenden reinen Metalloxidoberflächen und deutet auf eine unvollständige SAM-Bildung hin.
Fe₂O₃-Streifen wurden mittels Abscheidung aus der Gasphase auf Ti_{0.4}Si_{0.6}O₂-beschichtete Glasplättchen in Form von 2 mm breiten Streifen aufgebracht. Während des Spülvorganges nach der Beschichtung in der DDPO₄-Lösung wurde offensichtlich, dass nur die Fe₂O₃-Zonen hydrophob wurden, während die benachbarten Ti_{0.4}Si_{0.6}O₂ Zonen hydrophiler blieben. Der beobachtete 2 mm breite wasserabweisende Streifen in Fig. 4 entspricht der Fe₂O₃-Zone.

### 3. Röiztgen-Photoelektronenspektroskopie

Nach der Tauchbehandlung der Metalloxid- und Siliziumoxid-Probenoberflächen in einer 0.5 mM wässrigen DDPO₄(NH₄)₂-Lösung wurden die Oberflächen mittels Röntgen-Photoelektronenspektroskopie (X-ray photoelectron spectroscopy, XPS) bei zwei verschiedenen Austrittswinkeln analysiert. Bei einem Elektronenaustrittswinkel von 15° (in Bezug auf die Oberfläche) ist die XPS-Analyse sehr oberflächenempfindlich, während bei einem Winkel von 75° auch tiefere Zonen (Substrat) mitanalysiert werden.
Die Konzentrationen von C, O, P und der entsprechenden Metalloxid-kationen wurden mittels Standard-Empfindlichkeitsfaktoren quantifiziert (Tab. 2a and b).

Die Menge der analysierten SAM-Schicht liegt im selben Bereich für alle untersuchten Metalloxide und weist auf eine Monolagen-Oberflächenbelegung hin. In Übereinstimmung mit den Kontaktwinkel- und Mikrotropfendichte-Messungen konnte keine SAM-Schicht auf den SiO₂ Proben festgestellt werden (kein Phosphorsignal und vergleichsweise wenig Kohlenstoff). Auf der Mischschicht (Ti_{0.4}Si_{0.6}O₂) wird etwa halb so viel Titan festgestellt wie auf der reinen Titanoxid (TiO₂) -Schicht. Entsprechend findet man auch nur etwa die Hälfte der Konzentrationen an P und C, verglichen mit der entsprechenden TiO₂ Oberfläche. Dies belegt wiederum, dass sich die SAM Schicht nur auf TiO₂, aber nicht auf SiO₂, bildet.

| Substrat | Atomkonzentrationen (15° Austrittswinkel) | | | |
|---|---|---|---|---|
| MOₓ | %C | %0(tot) | % M | %P |
| | | | | |
| Ta₂O₅ | 67.8 | 23.2 | 6.71 | 2.36 |
| Al₂O₃ | 67.6 | 21.1 | 8.85 | 2.53 |
| Nb₂O₅ | 59.7 | 29.6 | 8.5 | 2.26 |
| ZrO₂ | 72.2 | 22.0 | 3.06 | 2.77 |
| Fe₂O₃ | 66.1 | 27.5 | Fe: 3.09 / Ti: 0.41 | 2.86 |
| TiO₂ | 68.6 | 23.0 | 6.13 | 2.23 |
| SiO₂ | 6.2 | 70.8 | 22.9 | 0 |
| Ti_{0.4}Si_{0.6}O₂ | 26.2 | 54.5 | Ti: 3.04/Si: 15.1 | 1.13 |

| Substrat | Atomkonzentration (75° Austrittswinkel) | | | |
|---|---|---|---|---|
| MOₓ | % C | % O (tot) | % M | % P |
| | | | | |
| Ta₂O₅ | 27.3 | 53.7 | 17.4 | 1.61 |
| Al₂O₃ | 25.3 | 47.1 | 25.5 | 2.10 |
| Nb₂O₅ | 31.9 | 49.4 | 17.3 | 1.30 |
| ZrO₂ | 35.9 | 49.0 | 12.9 | 2.28 |
| Fe₂O₃ | 28.9 | 56.5 | Fe: 10.2 / Ti: 2.46 | 1.93 |
| TiO₂ | 31.2 | 49.4 | 17.6 | 1.83 |
| SiO₂ | n.b.^{a)} | n.b.^{a)} | n.b.^{a)} | n.b.^{a)} |
| Ti_{0.4}Si_{0.6}O₂ | 14.9 | 60.2 | Ti: 9.97 / Si: 14.1 | 0.85 |

| | | | | |
|---|---|---|---|---|
| a) nicht beobachtet | | | | |

**Tab. 2a and b).** Atomkonzentrationen gemessen mit XPS von DDPO₄-beschichteten Metalloxidsubstraten. Elektronen-Austrittswinkel: 15° and 75° in Bezug auf die Oberfläche. Die Werte in Klammern entsprechen den Atomkonzentrationen von Titan aus dem Substrat unter der sputter-beschichteten Fe₂O₃ Probe, bzw. der Atomkonzentration von Silizium im Falle der Ti_{0.4}Si_{0.6}O₂ Probe.

### b) OH-DDPO₄ auf Ta₂O₅ und Nb₂O₅

Selbstorganisierte Schichten von 12-Hydroxydodecylphosphat (OH-DDPO₄) auf Ta₂O₅-und Nb₂O₅-beschichteten Proben wurden durch Tauchen in einer wässrigen 0.5 mM OH-DDPO₄(NH₄)₂-Lösung hergestellt, wie im experimentellen Teil (siehe oben) beschrieben.

### 1. Kontaktwinkel und XPS

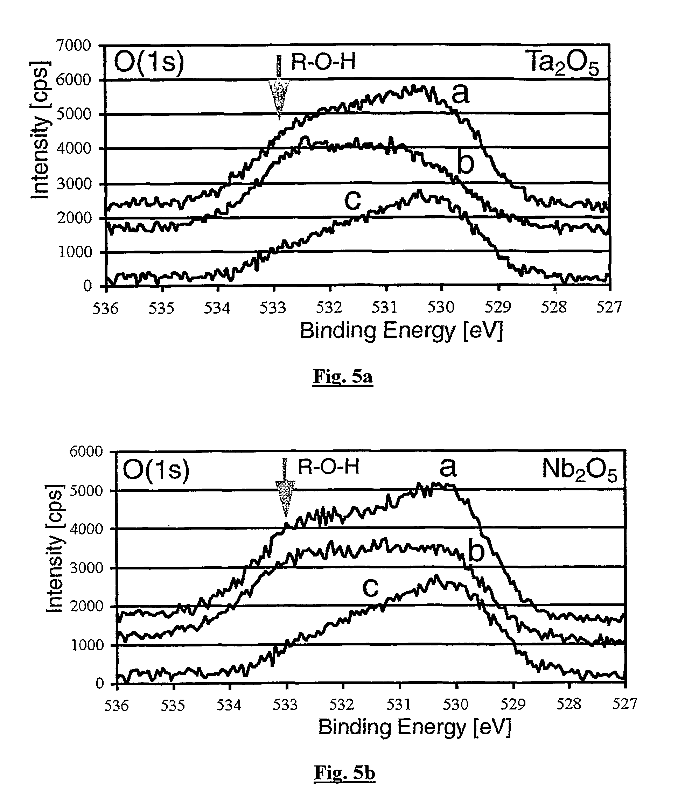

Der fortschreitende Kontaktwinkel wurde unmittelbar nach der Oberflächenbehandlung der Proben gemessen. Er beträgt ca. 50°, d.h. die Oberfläche ist im Vergleich mit der Methylterminierten Schicht aus DDPO₄ deutlich hydrophiler, aber weniger hydrophil als die gereinigte Metalloxidoberfläche (Kontaktwinkel < 10°).
Dies ist ein deutlicher Hinweis darauf, dass die Hydroxygruppen an der Oberfläche tatsächlich exponiert sind. Um dieses zu beweisen, wurden XPS-Spektren bei verschiedenen Austrittswinkeln gemessen. Die Variation der Signalintensität als Funktion des Austrittswinkels erlaubt es, Hinweise zur Lage des entsprechenden Elementes zu erhalten. Das O(1s) Signal ist für die beiden verschiedenen Austrittswinkel 11.5° and 20.5° in Fig. 5 gezeigt. Im Vergleich zu der reinen DDPO₄-Schicht zeigt das Sauerstoffsignal (O1s) eine zusätzliche Schulter bei einer Bindungsenergie von 533.4 eV (Fig. 5), wobei die Intensität bei 11.5° significant höher ist als bei 20.5. Die Lage des O(1s) Signals ist dabei typisch für Hydroxyfunktionen.
Kein Stickstoff wurde detektiert, was darauf hinweist, dass das Ammonium-Kation an der SAM-Bildung auf den hier untersuchten Oberflächen nicht teilnimmt.

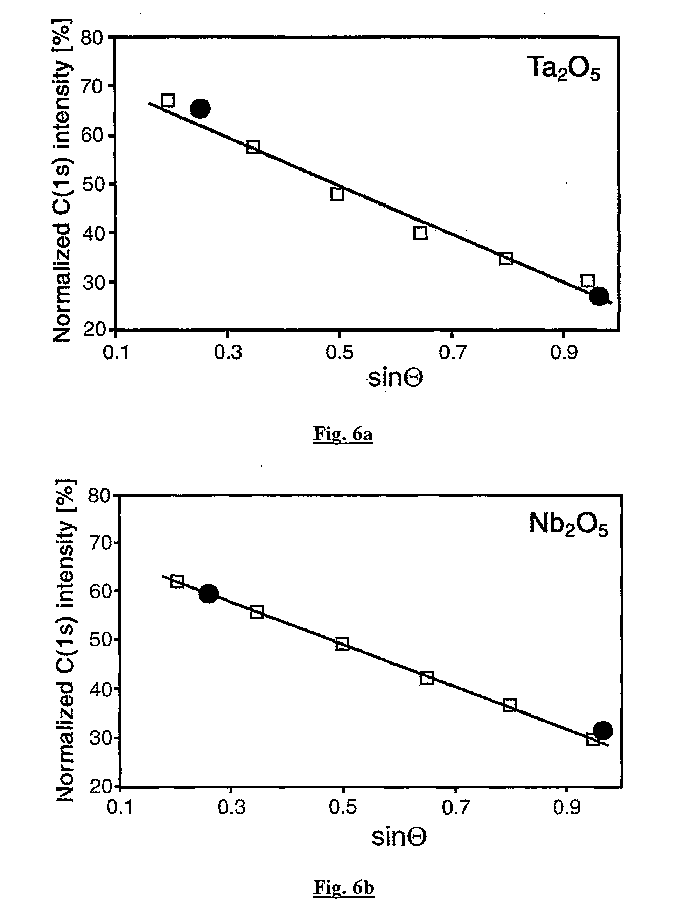

Es wurde bereits gezeigt, dass der Wasser-Kontaktwinkel mit dem SAM-Bedeckungsgrad korreliert. Kontaktwinkel von Hydroxy-terminierten SAM-Schichten wurden mit 50 - 80° bestimmt. Um zu testen, ob auch die Hydroxy-terminierten Alkyphosphate eine ähnlich dichtgepackte Schicht bilden wie das DDPO₄, wurden die XPS C1s Intensitäten der DDPO₄-SAMs mit denjenigen der OH-DDPO₄-SAMs bei verschiedenen Austrittswinkeln verglichen (Fig. 6). Die Resultate zeigen, dass die entsprechenden C1s Signale sehr gut vergleichbar sind. Dieses beweist, dass Hydroxy-terminiertes Alkylphosphat (OH-DDPO₄) ebenfalls dicht gepackte Schichten bildet, wie sie für geordnete SAMs typisch sind.

Die Atomkonzentrationen von C, O, P und des Substrat-Kations Ta bzw. Nb wurden aus den entsprechenden XPS-Intensitäten berechnet (Tab. 3). Die Daten sind konsistent mit dem Modell einer Oberfläche, bei dem die Phosphate an das Metalloxid angebunden sind, während die endständigen Gruppen (Hydroxy bzw. Methyl) von der Oberfläche weg zeigen ("tail-up" Konfiguration).

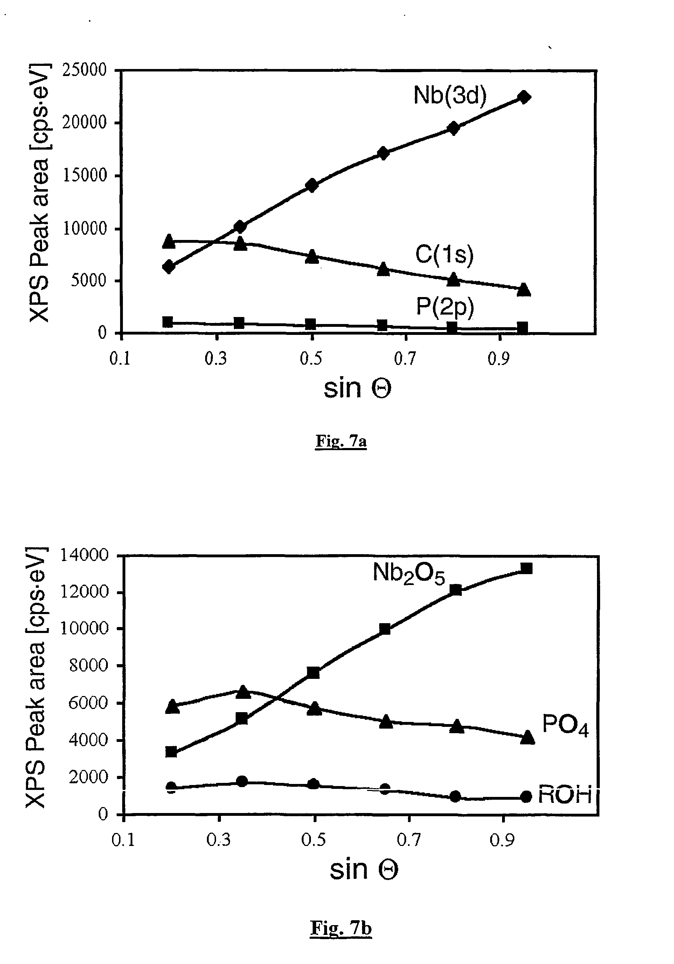

Die O1s Sauerstoffsignale wurden in drei Komponenten dekonvoluiert: a) den Metalloxid-Sauerstoff (530.2 eV), b) den Phosphat-Sauerstoff (531.4 eV für P-O-Metall and P=O und 532.6 eV für R-O-P and P-OH) sowie c) den Hydroxid-Sauerstoff (OH) bei 533.4 eV. Diese Zuordnung basiert auf einer entsprechenden detaillierten Analyse der XPS Spektren. Die Daten belegen, dass nicht nur das reine Alkylphosphat, sondern auch das OH-DDPO₄ in einer orientierten Art an die Oberfläche anbinden (Fig. 7).

Der Anstieg des Sauerstoffsignals des Metalloxid-Substrates mit zunehmendem Detektionswinkel ist bedingt durch die zunehmende Informationstiefe der Methode (höchste analysierte Tiefe bei senkrechter (90°) Detektion. Das Sauerstoffsignal der Phosphatgruppe nimmt mit zunehmendem Detektionswinkel leicht ab. Dieses ist typisch für Elemente, die sich an der Grenzfläche befinden. Das Sauerstoffsignal der Hydroxygruppe nimmt mit abnehmendem Detektionswinkel leicht zu, typisch für eine Lage der Hydroxygruppe an der Grenzfläche SAM/Luft bzw. Vakuum.

| Detektionswinkel Θ | Sin Θ | Atom-% Ta | Atom-% % O | Atom-% % P | Atom-% % C |
|---|---|---|---|---|---|
| 11.5 | 0.20 | 3.05 | 26.6 | 3.69 | 66.72 |
| 20.5 | 0.35 | 6.27 | 33.0 | 3.91 | 56.79 |
| 30.0 | 0.50 | 9.21 | 39.8 | 3.12 | 47.78 |
| 40.5 | 0.65 | 11.9 | 45.8 | 2.83 | 39.46 |
| 53.1 | 0.80 | 13.42 | 50.3 | 2.00 | 34.29 |
| 71.8 | 0.95 | 14.88 | 53.3 | 2.12 | 29.65 |

| Detektionswinkel Θ | Sin Θ | Atom-% Nb | Atom-% O | Atom-% P | Atom-% C |
|---|---|---|---|---|---|
| 11.5 | 0.20 | 4.64 | 29.3 | 3.86 | 62.21 |
| 20.5 | 0.35 | 6.74 | 33.9 | 3.32 | 55.97 |
| 30.0 | 0.50 | 9.53 | 38.5 | 3.07 | 48.62 |
| 40.5 | 0.65 | 11.97 | 43.6 | 2.78 | 41.97 |
| 53.1 | 0.80 | 14.05 | 47.7 | 2.01 | 36.24 |
| 71.8 | 0.95 | 16.38 | 51.6 | 2.07 | 29.98 |

**Tab. 3a and b.** XPS-Atomkonzentrationen von selbstorganisierenden OH-DDPO₄ Schichten auf Ta₂O₅ und Nb₂O₅ als Funktion des verwendeten Detektionswinkel Θ. Das Sauerstoffsignal wurde in drei Komponenten dekonvoluiert.

### c) Gemischte SAMs von OH-DDPO₄/DDPO₄ auf Ta₂O₅ und TiO₂

Mischungen wässriger Lösungen von 0.5 mM OH-DDPO₄(NH₄)₂ und 0.5 mM DDPO₄(NH₄)₂ wurden gemäss Angaben im experimentellen Teil hergestellt. Ta₂O₅-beschichtete Glasplättchen wurden gereinigt und in den Lösungen behandelt gemäss Angaben im experimentellen Teil.

### 1. Kontaktwinkel und Mikrotropfendichte

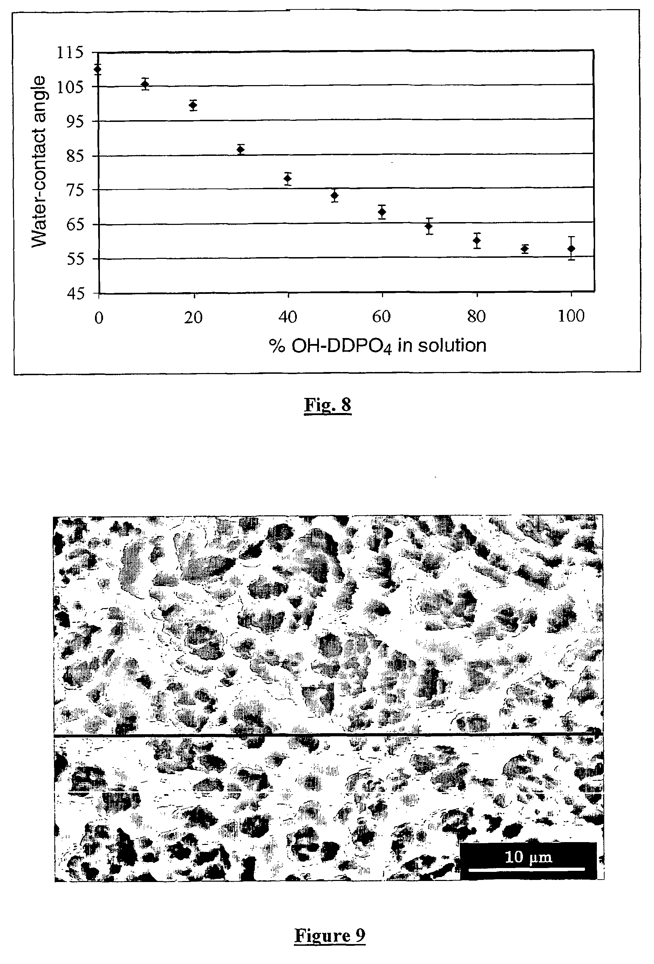

Der Kontaktwinkel wurde sofort nach der Herstellung der Oberflächen vermessen. Die Resultate (Fig. 8) zeigen eine deutlich ausgebildete Korrelation mit dem molaren Mischungsverhältnis der beiden SAM-Komponenten in der Lösung.

Die Mikrotropfendichte ist im Makro- und Mikrometermassstab homogen über die Oberfläche verteilt, so dass gefolgert werden kann, dass die beiden SAM-Komponenten in dieser Grössenordnung homogen verteilt sind. Die Differenz der Benetzbarkeit (Kontaktwinkel) beeinflusst die Mikrotropfendichte nur unwesentlich (Tab. 4). Eine Zunahme dieser Dichte ist zu erwarten, falls die Rauhigkeit, Oberflächenladung und / oder Unordnung der Schichten zunimmt. Die niedrige Dichte der Tröpfchen von 100 - 200 per mm² legt nahe, dass die Schichten relativ gut geordnet und glatt sind (Tab. 4). Der Einbau von OH-DDPO₄ in die Alkylphosphatschicht ändert also den Ordnungsgrad der SAM-Schichten nicht wesentlich.

| Vol-% OH-DDPO₄ in der Lösung | Kontaktwinkel (± Std.abweichung) | Mikrotropfendichte (± Std.abweichung) |
|---|---|---|
| | | |
| 0 | 110.1 ±0.8 | 144±29 |
| 10 | 105.6 ± 1.7 | 126 ± 26 |
| 20 | 103.6 ± 1.0 | 104 ± 21 |
| 30 | 86.6 ± 1.4 | 167 ± 33 |
| 40 | 81.6 ± 1.4 | 125 ± 25 |
| 50 | 73.0 ± 1.8 | 102 ± 21 |
| 60 | 70.9 ± 1.9 | 72 ± 30 |
| 70 | 64.1 ± 2.3 | 1611 ± 32 |
| 80 | 58.2 ± 1.5 | 123 ± 25 |
| 90 | 57.3 ± 1.2 | 229 ± 46 |
| 100 | 54.3 ± 3.5 | 159 ± 32 |

**Tab. 4.** Kontaktwinkel und Mikrotropfendichte der gemischten OH-DDPO₄/DDPO₄-SAM auf Ta₂O₅.

### 1.4 Schlussfolgerung

Es konnte gezeigt werden, dass Alkylphosphate aus wässrigen Lösungen der entsprechenden Salze (z.B. Ammoniumsalze) wohldefinierte selbstorganisierende Schichten ("SAM") auf einer Reihe von Metalloxidoberflächen bilden. Mittels XPS konnte kein Stickstoff nachgewiesen werden, so dass gefolgert werden kann, dass diese SAM-Schichten besonders rein sind, d.h. weder das Kation des Salzes noch organische Lösungsmittel enthalten. Lediglich ein gewisser Wasseranteil an der Grenzfläche kann erwartet werden. Dieser ist aber weder für die Biomaterial- (Implantats-) noch die Biosensoranwendung kritisch.

Im Falle des reinen Alkylphosphates konnten mit dem erfindungsgemässen Verfahren hoch hydrophobe Oberflächen hergestellt werden. Dabei zeigte sich, dass das Alkylphosphat selektiv auf Übergangsmetalloxiden sowie auf Aluminiumoxid geordnete SAM-Schichten bildet, während Siliziumoxid nicht belegt wird. Dieses eröffnet Möglichkeiten der Herstellung chemisch strukturierter Oberflächen vom Millimeter- bis in den Submikrometer- oder Nanometerbereich, indem die erfindungsgemässe Behandlung auf chemisch strukturierte Oberflächen (z.B. unter Verwendung von Mikrofabrikationstechniken wie Lithographie) angewendet wird. Zum Beispiel können beliebige, Photo- oder Elektronenstrahl-lithographische Muster mit Flächen, die lokal aus Silizium(oxid) bzw. aus einem Übergangsmetalloxid oder Aluminiumoxid bestehen, selektiv mit Alkylphosphaten oder endständig funktionalisierten Alkylphosphaten beschichtet werden. Die Siliziumoxid-Flächen können zudem in einem zweiten Schritt mit einer anderen Methode funktionalisiert werden. Dazu gehört auch die Möglichkeit, auf diesen Flächen Alkylphosphate oder funktionalisierte Alkylphosphate aus organischen Lösungsmitteln abzuscheiden, da bekannt ist, dass aus solchen nicht-wässrigen Lösungen auch Silziumoxid beschichtet werden kann. So können z.B. Muster mit lokal hydrophilen / hydrophoben Eigenschaften hergestellt werden. Ebenso ist es damit möglich, lokal unterschiedliche funktionelle Gruppen aufzubringen. Dazu gehören unterschiedliche Ladungen (Verwendung von z. B. positiv geladenen, endständigen Amin- oder Ammoniumgruppen, negativ geladenen Carboxy-, Phosphat- oder Phosphonat-Gruppen. Eine weitere Möglichkeit ist die lokale Aufbringung von Protein- und Zelladhäsionsresistenten Gruppen (z.B. unter Verwendung von endständig mit Oligo-Ethylenoxid modifizierten Alkylphosphaten), während andere Zonen der Oberfläche adhäsiven Charakter haben, indem sie Protein-adsorbierende Eigenschaften aufweisen. Letztere können auch noch verstärkt werden, indem in diesen Zonen Alkylphosphate aufgebracht werden, welche zelladhäsive Proteine, wie z.B. RGD-haltige Peptide, endständig angebunden enthalten. So ist es möglich, Proteine und biologische Zellen selektiv auf gewisse Zonen der Oberfläche zu dirigieren und die spezifischen Verhaltensmuster solcher isoliert aufgebrachten Zellen zu nutzen. Ebenso ist es für Anwendungen in der Biosensorik möglich, lokal und gezielt Erkennungseinheiten wie z.B. Antikörper von Proteinen, einsträngige DNA oder RNA Ketten, etc, aufzubringen und damit die Möglichkeit zu schaffen, Bioaffinitätstests gezielt auf bestimmten Zonen der Oberfläche durchzuführen. Dies ist im Hinblick auf die "multiarray" Technik in der Sensorik interessant.
Die obigen Ausführungen gelten sinngemäss auch für Salze der Alkylphosphonsäuren, deren Eigenschaften, insbesondere bezüglich Modifikation von metalloxidischen Oberflächen, sehr ähnlich sind.

### Beispiel 2: Herstellung von Dodecylphosphat-SAM und Hydroxy-Dodecylphosphat-SAM sowie von gemischten SAMs auf glatten und rauhen metallischen Titan-Implantatoberflächen

### 2.1 Ziel und Aufgabenstellung

Dieses Anwendungsbeispiel soll belegen, dass die erfindungsgemässe Anwendung von Salzen verschiedener Alkylphosphor- oder Alkylphosphonsäuren in wässriger Lösung auch für die Modifikation von metallischen, oxidbedeckten Implantatoberflächen anwendbar ist. Es soll zudem belegt werden, dass nicht nur glatte (z.B. polierte), sondern auch rauhe, topographisch strukturierte Oberflächen erfolgreich behandelt werden können.

### 2.2 Materialien and Methoden

Substrate: Metallproben aus cp (commercially pure) Titan, welche immer eine natürlich gebildete Titanoxidschicht aufweisen, wurden wie folgt in zwei unterschiedlichen Varianten behandelt:
A) Mechanisches Schleifen und Polieren, anschliessendes Reinigen in den organischen Lösungsmitteln Hexan, Aceton und Alkohol, gefolgt von einer Passivierung in 30% HNO₃-Lösung und einer Endreinigung in Sauerstoff-Plasma. Dies führt zu einer sauberen und glatten Oberfläche.
B) Strahlen mit Aluminiumoxidpartikeln, gefolgt von einer chemischen Ätzung in Chlorid-haltiger Schwefelsäure. Dies führt zu einer stark aufgerauhten Oberfläche, wie im rasterelektronmikroskopischen Bild von Fig. 9 gezeigt. Solche Oberflächen werden bevorzugt für Implantate im Knochenbereich eingesetzt, da sie eine hervorragende Fähigkeit zur vollständigen Einheilung im Knochen ("Osseointegration") aufweisen.

### 2.2.1 Alkylphosphate

Es wurden dieselben Materialien wie im Beispiel 1 eingesetzt.

### 2.2.2 Herstellung der Behandlungslösungen und Behandlungen der metallischen Proben.

Es wurden dieselben Verfahren wie in Beispiel 1 eingesetzt.

### 2.3 Resultate

### a) DDPO₄(NH₄)2 und OH-DDPO₄(NH₄)₂ auf glatter Titanoberfläche

Es wurden wässrige Lösungen sowohl von reinem DDPO₄(NH₄)₂ bzw. reinem OH-DDPO₄(NH₄)₂ wie auch verschiedene Mischungsverhältnisse dieser beiden Substanzen für die Behandlung glatter und rauher Titanoberflächen eingesetzt.

Tab. 5 zeigt die XPS-Resultate der behandelten Oberflächen. Analog wie im Beispiel 1 belegen diese Daten, dass auch metallische Titanoberflächen (mit oxidischer Passivschicht) analog zu reinen Titanoxidschichten nach dem erfindungsgemässen Verfahren mit den entsprechenden Molekülen belegt werden.

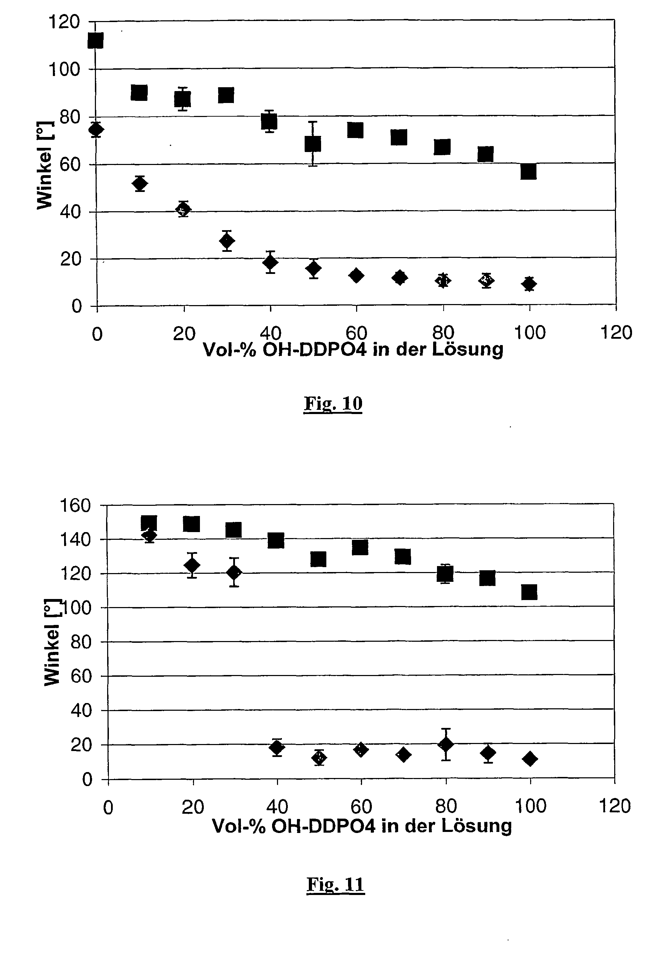

Fig. 10 zeigt die entsprechenden Kontaktwinkel, gemessen gegen Wasser. Diese sind den entsprechend behandelten Metalloxid-beschichteten Oberflächen (Beispiel 1) sehr ähnlich. Wiederum wird eine charakteristische Abhängigkeit des Kontaktwinkels von der Zusammensetzung der SAM-Lösung festgestellt. Die Technik eignet sich also auch bei diesen Materialien für die gezielte Einstellung oberflächenrelevanter Eigenschaften (hier Benetzbarkeit).

| Vol-% OH-DDPO₄ in der Lösung | Atomkonzentrationen (90° Austrittswinkel) | | | |
|---|---|---|---|---|
| | % C | % O (tot) | % Ti | % P |
| 0 | 46.66 | 36.59 | 13.76 | 3 |
| 10 | 55.81 | 31.06 | 10.16 | 2.98 |
| 20 | 57.03 | 30.49 | 9.62 | 2.86 |
| 30 | 48.11 | 36.73 | 12.24 | 2.92 |
| 40 | 49.01 | 36.55 | 12 | 2.44 |
| 50 | 54.31 | 32.49 | 10.27 | 2.93 |
| 60 | 47.06 | 37.86 | 12.61 | 2.47 |
| 70 | 49.28 | 36.07 | 12.1 | 2.55 |
| 80 | 49.81 | 35.94 | 11.82 | 2.43 |
| 90 | 49.66 | 36.58 | 11.84 | 1.92 |
| 100 | 45.63 | 39.32 | 12.36 | 2.68 |

**Tab. 5** Oberflächenkonzentration von glatten, metallischen Titanoberflächen, behandelt gemäss dem erfindungsgemässen Verfahren mit wässrigen Lösungen der Salze DDPO₄(NH₄)₂, OH-DDPO₄(NH₄)₂ bzw. deren Mischungen in unterschiedlichen Konzentrationsverhältnissen.

### b) DDPO₄(NH₄)₂ und OH-DDPO₄(NH₄)₂ auf rauher Titanoberfläche

Tab. 6 zeigt die XPS-Daten der mit wässrigen Lösungen der Salze DDPO₄(NH₄)₂ und OH-DDPO₄(NH₄)₂ bzw. deren Mischungen behandelten metallischen, rauhen Oberflächen. Diese Proben weisen eine Oberflächenstruktur auf, wie sie in Fig. 9 gezeigt ist. Wiederum weisen die Oberflächenkonzentrationen auf eine vollständige Ausbildung der entsprechenden SAM-Schichten weist. Die Konzentrationen sind dabei leicht unterschiedlich von denjenigen der glatten Oberflächen, was auf den Einfluss der Oberflächentopographie auf die XPS-Messung zurückzuführen ist.

Fig. 11 zeigt die entsprechenden Kontaktwinkel analog zu Fig 10. Die Kontaktwinkel zeigen dabei einen deutlich anderen Verlauf als die entsprechenden Kurven der glatten Oberflächen. Dieses ist auf den Einfluss der Oberflächentopographie zurückzuführen. Es ist bekannt, dass rauhe (topographisch strukturierte), hydrophobe Oberflächen deutlich höhere Kontaktwinkel (reduzierte Benetzung) als glatte hydrophobe Oberflächen aufweisen (Ref). Dieser Effekt ist auch in der Natur beobachtet worden, und den entsprechenden Beobachtungen wurde der Name "Lotus-Effekt" gegeben, da z.B. Lotus-Blütenblätter genau diese Kombination von Rauheit und Hydrophobizität zur Ausbildung einer selbstreinigenden Oberfläche ausnutzen.

Die hohen Kontaktwinkel der DDP0₄(NH₄)₂-behandelten Oberfläche geben einen eindeutigen Hinweis darauf, dass diese rauhen Oberflächen mit der DDPO₄-SAM-Schicht einwandfrei beschichtet sind.

| Vol-% OH-DDPO₄ in der Lösung | Atomkonzentrationen (90° Austrittswinkel) | | | |
|---|---|---|---|---|
| | % C | % O (tot) | % Ti | % P |
| 0 | 42.64692 | 38.87785 | 15.28833 | 3.186893 |
| 10 | 42.01745 | 39.3542 | 14.94158 | 3.686767 |
| 20 | 42.21079 | 39.2392 | 14.57747 | 3.972532 |
| 30 | 41.14441 | 40.02102 | 14.01979 | 4.814779 |
| 40 | 41.1987 | 41.81364 | 13.49958 | 3.488083 |
| 50 | 42.50703 | 40.95192 | 12.98231 | 3.55874 |
| 60 | 41.44062 | 42.45984 | 12.19712 | 3.902415 |
| 70 | 42.90176 | 40.70315 | 12.57557 | 3.819517 |
| 80 | 42.77291 | 40.86229 | 12.66196 | 3.702839 |
| 90 | 41.26897 | 40.97354 | 13.70276 | 4.054732 |
| 100 | 42.8869 | 40.12049 | 13.88565 | 3.106954 |

**Tab. 6:** Oberflächenkonzentration von rauhen, metallischen Titanoberflächen, behandelt gemäss der Erfindung mit wässrigen Lösungen der Salze DDPO₄(NH₄)₂, OH-DDPO₄(NH₄)₂ bzw. deren Mischungen in unterschiedlichen Konzentrationsverhältnissen.

Es zeigt sich also, dass nicht nur glatte Titanoberflächen, sondern auch solche mit hoher spezifischer Oberfläche und komplexer Rauheit, wie sie für Implantate, z.B. für Knochenimplantate, oft Verwendung finden, mit Erfolg nach dem erfindungsgemässen Verfahren beschichtet werden können.

### Beispiel 3: Sandwich-Immunoassays auf DDP-beschichteten Sensorchips: Quantitativer Nachweis der Analyten Immunoglobulin (Kaninchen-IgG) und des menschlichen Zytokin Interleukin 6 (hIL-6) mittels Fluoreszenzdetektion auf planaren Wellenleitern als Sensorplattform

Ziel der Experimente war es, zu zeigen, dass durch eine erfindungsgemässe Oberflächenmodifikation eines planaren Dünnschichtwellenleiters als Sensorplattform, mittels Aufbringung von Monoschichten aus Alkylphosphaten oder -phosphonaten eine deutliche Steigerung der Empfindlichkeit, im Vergleich zu Sensorplattformen ohne eine solche erfindungsgemässe Vorbehandlung, erreicht werden kann. Die dabei erzielten Nachweisempfindlichkeiten liegen im niederen pg/ml Bereich. Eine solche Empfindlichkeit kann nur erreicht werden, wenn entsprechend viele Erkennungsantikörper auf der Sensoroberfläche in aktiver Form, d.h. ohne zu denaturieren, aufgebracht werden können. Dieses kann durch die Aufbringung eines Dodecylphosphat-SAM (DDP-SAM) erreicht werden. Des weiteren sollte demonstriert werden, dass Assays auf Sensorchips ohne vorherige Abscheidung einer DDP-Monolage, aber bei ansonsten gleicher Vorbehandlung, entsprechend niedrigere Assaysignale aufweisen. Weiterhin sollte gezeigt werden, dass eine Monolage von DDP und damit die Anzahl der oberflächengebundenen Erkennungsmoleküle über die Dauer eines Experiments von mehreren Stunden stabil bleibt und auch durch zusätzlichen mechanischen Stress, wie z.B. durch starkes Bespülen der Oberfläche mit Messpuffer, keinen Schaden nimmt. Die Stabilität manifestiert sich durch eine gleichbleibende Fluoreszenzsignalantwort nach einem durchgeführten Bindungsexperiment.

### 3.1. Sensorplattformen

Als Sensorplattformen wurden rechteckige, planare Wellenleiterchips, bestehend aus einer dünnen hochbrechenden Wellenleiterschicht auf einem 0.7 mm dicken transparentem Substrat, mit den äusseren Abmessungen 16 mm Höhe x 48 mm Breite x 0.7 mm Dicke, benutzt. Das optisch transparente Substratmaterial des Sensorchips bestand aus AF45 Glas (Brechungsindex n = 1.52 bei 633 nm). Im Substrat waren fünf Oberflächenreliefgitter mit einer Breite von 0.5 mm (in Ausbreitungsrichtung des über die Gitterstruktur in die Schicht der Sensorplattform einzukoppelnden Anregungslichts) über die Höhe des Substrats strukturiert. Die Gitter waren in einem Abstand von 9 mm entlang der Breite des Chips angebracht. Die Fläche zwischen zwei Gittern wurde als Messfläche benutzt. Die Reliefgitter wiesen eine Periode von 360 nm und eine Tiefe von 12 nm auf. Die wellenleitende, optisch transparente Schicht bestehend aus Ta₂O₅ hatte einen Brechungsindex von 2.11 bei 633 nm (Schichtdicke 150 nm).

Die Reliefgitter dienen zur Einkopplung von Laserlicht einer definierten Wellenlänge (hier 633 nm) in die Sensorschicht. In die Sensorschicht eingekoppeltes und dort geführtes Licht wird zur Anregung von oberflächennahen, d.h. im evaneszenten Feld des geführten Lichts befindlichen, Fluorophoren benutzt. Das so an der Oberfläche erzeugte Fluoreszenzlicht dient als Messsignal für die Bindung von Analytmolekülen an auf der Sensoroberfläche immobilisierte spezifische Erkennungselemente. Aus der Intensität der emittierten Fluoreszenz kann die Anzahl gebundener Analytmoleküle bestimmt werden.

Zum Kontaktieren der Analyseprobe mit dem Sensorchip wurde eine Flusszellenstruktur mit der vorbehandelten Sensorplattform verbunden. Die Flusszellenstruktur umfasste Probenbehältnisse, in denen jeweils eine Messfläche zwischen zwei Gittern auf dem Chip vorhanden war. Die Probenbehältnisse hatten ein Volumen von jeweils 15 µl, mit einer Grundfläche von 7 mm x 11 mm auf der Sensoroberfläche und einer Kammerhöhe von 0.2 mm. Die Analyseprobe konnte durch einen Injektions- bzw. Ablaufkanal (Durchmesser 0.5 mm) der Probenkammer zu- bzw. abgeführt werden. Die Proben-/Pufferzugabe erfolgte mittels Dispensierpumpe über Schlauchverbindungen zwischen Pumpe und Einlass.

### 3.2. Chipvorbehandlung

Die Sensoroberfläche wurde vor dem Zusammenfügen mit der Flusszellen-Struktur nasschemisch gereinigt, zuerst mehrfach mit Isopropanol, anschliessend mit konzentrierter Schwefelsäure, welche 2.5 % Ammoniumperoxodisulfat enthielt. Anschliessend wurde eine monomolekulare Schicht (Monolayer) von Dodecylphosphat (DDP) in einem Selbstorganisierungsprozess (Self-Assembly) aus wässriger Phase auf der hydrophilen Wellenleiteroberfläche abgeschieden. Zum Abscheiden wurde eine 0.5 mM DDP/Ammoniumsalz-Lösung in Wasser benutzt. Die Sensoroberfläche wurde über 2 h in der DDP-Lösung unter ständigem Rühren bei Raumtemperatur inkubiert, anschliessend unter fliessendem Wasser ausgiebig gewaschen und danach unter einem Strom von Stickstoff getrocknet. Das Abscheiden der Monolage DDP führte zu einer hydrophoben Oberfläche (Kontaktwinkel ca. 110° gegenüber Wasser).

### 3.3. Aufbringen der biologischen Erkennungselemente

### (A) für Nachweis von Kaninchen-IgG

Die Sensoroberfläche wurde mit einer wässrigen Lösung von biotinyliertem Rinderserumalbumin (BSA-Biotin, Sigma, Katalog-Nr. A-6043) inkubiert. Es wurde eine 100 µg/ml Lösung in phosphatgepufferter Salzlösung (PBS), pH 7.4, verwendet. Der Chip wurde 1 h bei Raumtemperatur inkubiert, unter fliessendem, deionisiertem Wasser (Millipore, spez. Widerstand = 18 mΩ cm) ausgiebig gewaschen und danach unter einem Strom von Stickstoff getrocknet. Anschliessend wurde die Oberfläche mit einer Lösung von 1 mg/ml reinem Rinderserumalbumin (BSA, Sigma, Katalog-Nr. A7906) in PBS, pH 7.4, geblockt. Dazu wurde 15 min lang inkubiert, wiederum unter fliessendem, deionisiertem Millipore-Wasser ausgiebig gewaschen und danach unter einem Strom von Stickstoff getrocknet. Danach wurde in gleicher Weise die geblockte Oberfläche mit 50 µg/ml Streptavidin (Sigma Katalognr. S4762) in PBS, pH 7.4, für 30 min inkubiert. Dabei binden die Streptavidinmoleküle an die zugänglichen Biotingruppen des Albumin. Überschüssiges Streptavidin wurde durch Spülen mit Wasser wieder entfernt. Schliesslich wurde die Oberfläche mit dem biotinylierten Erkennungsantikörper Anti-Kaninchen IgG (Sigma, Katalognr. B-7389) in einer Konzentration von 12 µg/ml in PBS, pH 7.4, für 30 min inkubiert, wiederum ausgiebig gewaschen und mit Stickstoff getrocknet. Der trockene Chip wurde dann mit der Flusszellenstruktur verbunden.

### (B) für Nachweis von hIL-6

Die Sensoroberfläche wurde im Bereich der Messfläche mit 50 µl einer wässrigen Lösung von monoklonalem Maus-Anti-hIL-6 Antikörper (R&D Systems, Katalog-Nr. mAb206) inkubiert. Der gefriergetrocknete Maus-Anti-hIL-6 Antikörper wurde dazu in einer Konzentration von 0.5 mg/ml in zehnprozentiger, phosphatgepufferter Salzlösung (10% PBS), pH 7.4, rekonstituiert und anschliessend mit 10% PBS, pH 7.4, auf eine Konzentration von 10 µg/ml verdünnt. Mit dieser Lösung wurde der Chip 2 h lang bei Raumtemperatur inkubiert, unter fliesendem, deionisiertem Millipore-Wasser (spez. Widerstand = 18 mΩ cm) ausgiebig gewaschen und danach unter einem Strom von Stickstoff getrocknet. Anschliessend wurde die Oberfläche mit einer Lösung von 1 mg/ml Rinderserumalbumin (BSA, Sigma, Katalog-Nr. A7906) in PBS, pH 7.4, geblockt. Dazu wurde 15 min lang inkubiert, wiederum unter fliessendem, deionisiertem Millipore-Wasser ausgiebig gewaschen und danach unter einem Strom von Stickstoff getrocknet. Der getrocknete Chip wurde mit der Flusszellenstruktur verbunden und in die Messapparatur eingesetzt.

### 3.4. Analytische Messapparatur

Der Sensorchip ist auf einer computergesteuerten Justiereinheit montiert, welche die Translation parallel und senkrecht zu den Gitterlinien sowie eine Rotation um eine Drehachse parallel zu den Gitterlinien der Sensorplattform erlaubt. Unmittelbar nach dem als Anregungslichtquelle benutzten Laser befindet sich im Lichtweg ein Shutter, um den Lichtweg zu blockieren, wenn keine Messdaten aufgenommen werden sollen. Zusätzlich können Neutralfilter oder Polarisatoren an dieser Stelle oder auch anderen Positionen im weiteren Weg des Anregungslichts zur Sensorplattform in den Lichtweg gestellt werden, um die Anregungsintensität stufenweise oder kontinuierlich zu variieren.

Der Anregungslichtstrahl eines Helium-Neon-Lasers bei 632.8 nm (Melles-Griot 05-LHP-901, 1.1 mW) wird mit einem Linsensystem unter Verwendung einer Zylinderlinse in einer Dimension aufgeweitet und durch eine spaltförmige Blende (0.5 mm x 7 mm Öffnung) geleitet, um so ein paralleles Lichtbündel von annähernd rechteckigem Querschnitt und annähernd homogener Querschnittsintensität zu erzeugen. Dabei ist die Polarisation des Laserlichts parallel zu den Gitterlinien der Sensorplattform ausgerichtet, zur Anregung des TE₀-Modes unter Einkoppelbedingungen. Das Anregungslicht wird durch die Rückseite der Sensorplattform, d.h. durch die optisch transparente Schicht (b) hindurch auf ein Einkoppelgitter innerhalb eines der 5 Probenbehältnisse gerichtet, wobei das Einkoppelgitter innerhalb eines Probenbehältnisses sich unter den Bedingungen des Ausführungsbeispiels jeweils am linken Rand der rechteckigen Probenkammer befand. Der Winkel zwischen der Sensorplattform und dem eingestrahlten Anregungslichtbündel wird durch Rotation um die vorgenannte Drehachse auf maximale Einkopplung in die optisch transparente Schicht (a) justiert. Mit den vorgenannten Parametern der Sensorplattform betrug der Resonanzwinkel für die Einkopplung in Luft etwa 2.6°.

Als Detektor dient ein rotempfindlicher Photomultiplier (PMT) (H6240, Hamamatsu, Japan), der im Einzelphotonen-Zählmodus mit einem 225 MHz-Zähler (Model 53131A, Hewlett-Packard, USA) ausgelesen wird. Die Signalerfassung und Fokussierung des von der Substratseite des Sensorchip emittierten Fluoreszenzlichtes auf das Detektionsfenster des PMT erfolgt mit Hilfe eines selbstgebauten Objektivs (Abbildung 1:1, numerische Apertur 0.19). Im parallelen Teil des Objektiv-Strahlengangs befinden sich, auf einem Filterwechsler montiert, zwei Interferenzfilter (Omega, Brattleborough, Vermont) mit Zentralwellenlänge von 680 nm und 40 nm Bandbreite, sowie vor der Filtereinheit eine dünne Glasplatte als Strahlteiler, die aus der Sensorfläche herausgestreutes Anregungslicht als Referenzsignal über Messung mit einer Photodiode erfassen kann. Durch diese Anordnung ist gewährleistet, dass das Lumineszenzsignal und das Referenzsignal vom gleichen Messbereich stammen. Die Signale der Referenz (bei der Anregungswellenlänge) und das eigentliche Messignal (bei der Lumineszenzwellenlänge) können gleichzeitig gemessen werden. Gezeigte Messsignale sind bereits mithilfe des Referenzsignals korrigiert. referenziert um Messbereichsschwankungen zu eliminieren. Die Messignale wurden als Quotient von Lumineszenzsignal und Referenzsignal, multipliziert mit dem Faktor 10⁶, ermittelt.

### 3.5. Durchführung des analytischen Nachweisverfahrens

Zur spezifischen Erkennung der nachzuweisenden Analyten (Kaninchen IgG bzw. h-IL-6) wurde das Format des Sandwich-Immunoassays gewählt.

### 3.5.1. Probenvorbereitung

Von den zu quantifizierenden Analyten (Kaninchen IgG bzw. h-IL-6) wurden Standardlösungen von je 500 µl in PBS, pH 7.4, mit 0.1% BSA und 0.05% Tween20 hergestellt. Die Konzentrationen betrugen jeweils 0, 0.067, 0.67, 6.7, 67, bzw. 670 pM für den Analyten Kaninchen-IgG (Sigma, Katalog-Nr. I-5006) bzw. 0, 0.5, 1, 2.5, 5, 25, 50, 250, 500, 2500, 10000 pg/ml für den Analyten hIL-6 (R&D Systems, Katalog-Nr. 206-IL-06) mit einem Zusatz von jeweils 10% Kälberserum (Anawa, Katalog-Nr. 8270-0209). Diese Standardlösungen waren vorgesehen für die Erstellung von Kalibrationskurven der Analyten, um die Konzentrationen von Proben unbekannter Analytmenge ermitteln zu können.

Die Kalibrationslösungen, wie auch die Proben mit unbekannten, zu bestimmenden Konzentrationen der Analyten, wurden anschliessend mit jeweils 500 µl einer Lösung gemischt, welche polyklonalen Nachweisantikörper enthielt. Für den Nachweis von Kaninchen-IgG wurde anti-Kaninchen-IgG aus Ziege, markiert mit Cy5-Farbstoff (Amersham-Pharmacia, Katalog-Nr. PA45004), mit einem Farbstoff-zu-Protein-Verhältnis von 4:1, verwendet. Für den Nachweis von hIL-6 wurde mit Cy5-gelabelter anti-hIL-6-Antikörper (AF-206-NA, R&D Systems, UK), mit einem Farbstoff-zu-Protein-Verhältnis von 5:1, verwendet. Analytlösung und Tracer-Lösung wurden jeweils im Verhältnis 1:1 gemischt und 1 h lang im Dunkeln bei 37°C inkubiert.

### 3.5.2. Durchführung der Messung

Der vorbehandelte, trockene Sensorchip mit aufgebrachter Flusszellenstruktur wurde ins Messgerät eingesetzt. Zur Durchführung eines Experimentes wurde das trockene Probenbehältnis mit Messpuffer (PBS, pH 7.4, 0.05 % Tween 20, 0.1% BSA) 5 min lang mit einem kontinuierlichen Fluss von 0.25 ml/min gespült. Zur Optimierung der Anregungsbedingungen für die Lumineszenzanregung wurde der Sensorchip mit den darauf befindlichen Probenkammern und der darin befindlichen Lösung auf maximale Einkopplung des Anregungslichts über die dem jeweiligen Messbereich zugeordnete Gitterstruktur justiert und die Intensität des Emissionslichtes aus dem Messbereich über die Dauer von 1s gemessen. Die vorinkubierten Standardlösungen wurden sequentiell in aufsteigender Konzentration aus einem Autosampler (231XL, Gilson, USA) mittels Dispensierpumpe in die Probenkammer zugegeben. Beim Bespülen wurde übrige Restlösung über den Abfluss der Probenkammer ausgestossen.

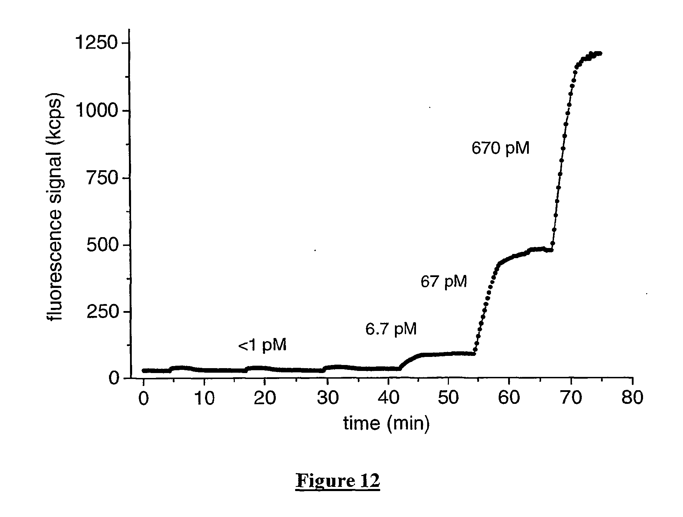

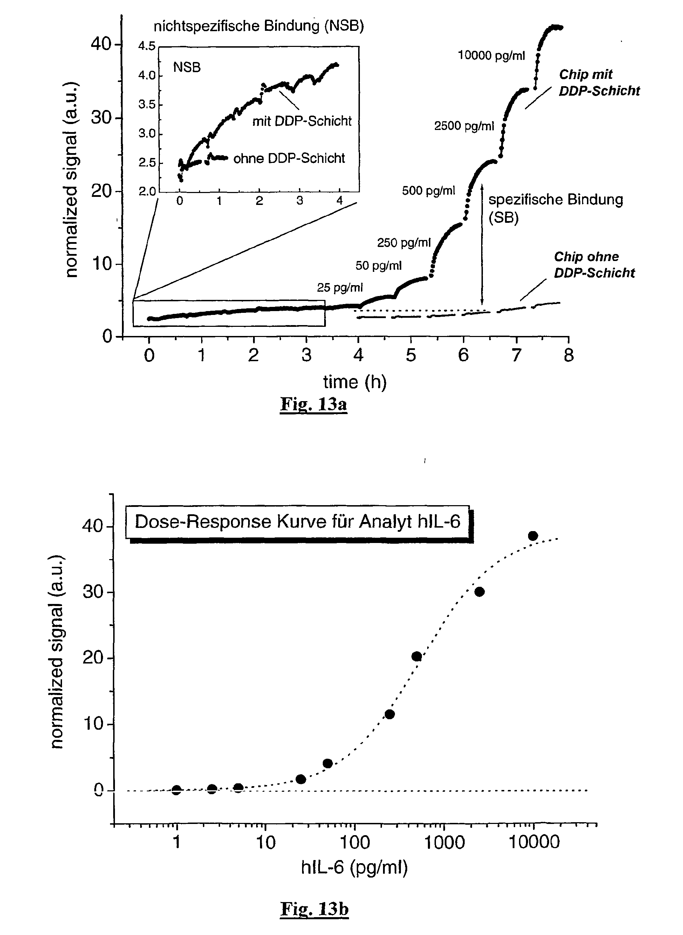

Im Falle des Nachweises von Kaninchen-IgG wurde die gesamte Probe (1 ml) unter kontinuierlichem Fluss (0.25 ml/min) 4 min lang appliziert und während dieser Zeit in Echtzeit das Anbinden des Analyten an die präparierte Sensoroberfläche mittels Fluoreszenzanstieg gemessen (Messpunkte alle 30 s, Fig. 12). Nachfolgend wurde während jeweils 8 min mit Pufferlösung gespült, bevor die Probe mit der nächsthöheren Konzentration appliziert wurde. Im Falle des Nachweises von hIL-6 wurde die Probe nur kurz und schnell injiziiert (Fluss 1 ml/min) und anschliessend ohne Fluss die Bindung der Analytmoleküle aus der stehenden Probe an ihre immobilisierten Erkennungselemente während einer Dauer von 30 min, bis zum Erreichen des Gleichgewichtszustandes, gemessen (Messpunkte alle 60 s, Fig. 13a). In diesem Falle erfolgte kein Spülschritt zwischen sequentiellen Probenzugaben. Der korrigierte Signalverlauf, aus dem Quotienten der gemessenen Fluoreszenz- und Referenzsignale, ist in den Figuren 12 (Kaninchen-IgG als Analyt) und 13a (hIL-6 als Analyt) dargestellt. Aus der ersten Messung mit einer Probe ohne Analyten wurde jeweils das Ausmass der unspezifischen Bindung bestimmt. Die Netto-Bindungssignale bei den verschiedenen Analytkonzentrationen wurden dann als Differenz zwischen dem korrigierten, bei der aktuellen Analytkonzentration gemessenen Brutlosignal und dem unspezifischen Bindungssignal bestimmt. Hieraus wurden die Signal-Konzentrationskurven für Kaninchen-IgG (Figur 13b für hIL-6) generiert.

Figur 13a zeigt den Verlauf der Bindungssignale in einer Messreihe zum Nachweis von hIL-6. Während der Gesamtdauer des Experiments, von mehr als 7 Stunden, wurde keine systematische Abnahme der Signalhöhen, auch nach erreichen der Sättigungssignale im stationären Zustand für die jeweilige Analytkonzentration, beobachtet. Dieses bedeutet, dass weder Signalverlust durch Ausbleichen des Farbstoffes noch Signalverlust durch eventuelles Ablösen von signalgebenden Bindungskomplexen von der Oberfläche stattfand, was auf eine sehr stabile Immobilisierung der eingesetzten biologischen Erkennungselemente auf der Oberfläche der Sensorplattform schliessen lässt. Auch führte starkes Bespülen (Fluss 2 ml/min für die Dauer von 5 min) der Sensoroberfläche mit grösseren Probenmengen, gleichbedeutend mit Anwendung von mechanischem Stress, in einen stationären Signalverlauf und nicht zu einer Signalabnahme, was ein weiterer Hinweis auf die hohe Stabilität der Immobilisierung ist.

### Performance hIL-6 Assay ohne Haftvermittlungsschicht

Um den positiven Einfluss einer Monolage DDP auf die Intensität der Assaysignale, d.h. die Menge an funktionell immobilisierten Erkennungselementen, und damit auf die ereichbaren Nachweisgrenzen, zu untersuchen, wurde zum Vergleich das hIL-6 Immunoassay auf Sensorchips ohne aufgebrachte Monoschicht DDP, aber mit ansonsten gleich vorbehandelter Oberfläche, durchgeführt. Wie in Figur 13a gezeigt, erreichten die Bindungssignale höchstens 10% der Signale von Experimenten mit abgeschiedener Haftvermittlungsschicht aus DDP. Die geringeren Signale sind auf eine geringere Oberflächenbelegung und/oder auf Denaturierung der Erkennungsantikörper auf der unbehandelten Oberfläche zurückzuführen.

### Beispiel 4: Sandwich-Immunoassays auf Monoschichten aus gemischt hydrophob/hydrophil terminierten Alkylphosphaten.

Ziel des Experiments war es zu zeigen, dass Assaysignale und damit Nachweisempfindlichkeiten von Immunoassays deutlich gesteigert werden können auf Sensoroberflächen, die durch Abscheiden von Monoschichten aus definierten Mischungen von hydrophob (alkyl-) und hydrophil (hydroxyl-)terminierten Alkylphosphaten erzeugt wurden. Wie bereits gezeigt, kann der Kontaktwinkel von Metaloxidoberflächen gegen Wasser gezielt durch entsprechende Mischung von hydrophil- und hydrophobterminierten Alkylphosphaten eingestellt werden. Dies erlaubt eine spezifische Optimierung der Anzahl funktionell immobiliserter Erkennungelemente in Abhängigkeit von der Art des verwendeten Erkennungselemtes. Es wurden als Erkennungselemente die in Beispiel 3 beschriebenen Antikörper verwendet.

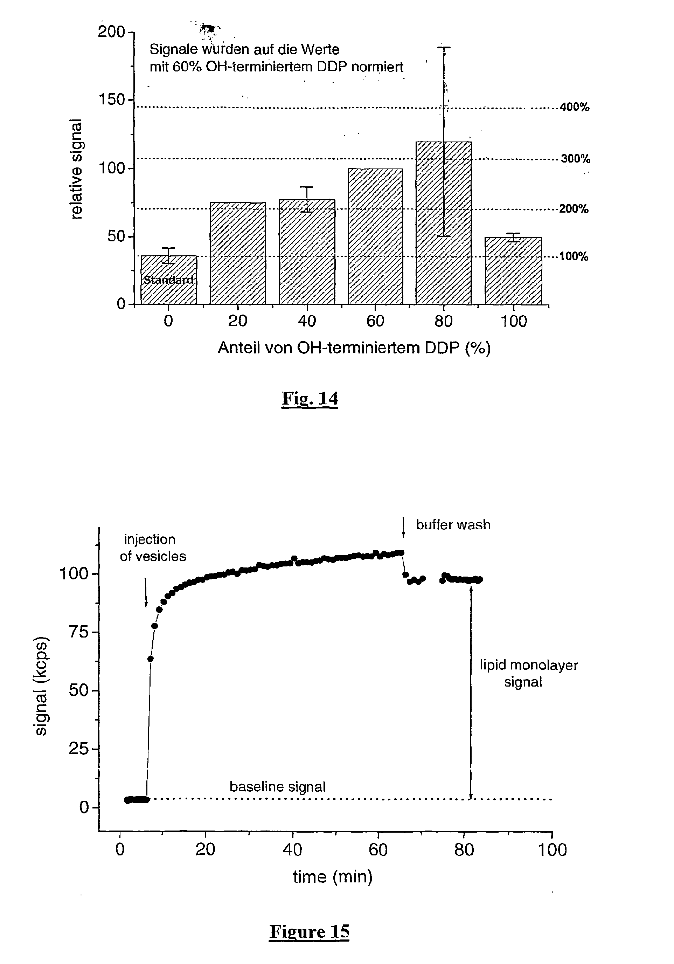

Sensorchipoberflächen wurden hergestellt mit Monoschichten aus wässrigen Mischungen von DDP-OH:DDP im Verhältnis von 0:100, 20:80, 40:60, 60:40, 80:20 und 100:0 Prozent. Die weitere Behandlung der Chips sowie die Durchführung der Messungen erfolgten wie beschrieben in Beispiel 3. In Figur 14 sind die normierten Signalantworten für die verwendeten Mischungen dargestellt. Im Falle des Nachweises von Kaninchen-IgG kann die Signalantwort bei der optimalen Mischung um bis zu 300% gegenüber dem Wert mit einer reinen DDP-Schicht gesteigert werden. Das optimale Mischverhälnis liegt zwischen 80:20 und 60:40 Prozent DDP-OH:DDP, also bei einer eher hydrophilen Oberfläche. Ähnliche Werte wurden erreicht bei den Assays zum Nachweis von hIL-6.

### Beispiel 5: Erzeugung einer biokompatiblen Doppel-Haftvermittlungsschicht (bestehend aus jeweils einer DDP- und einer Lipidschicht) frei von organischen Lösungsmittelresten.

Wie bereits gezeigt, können durch Abscheiden von DDP aus wässriger Phase sehr hydrophobe Monoschichten auf Metalloxidoberflächen erzeugt werden. Solche hydrophobe, selbstorganisierten Schichten eignen sich sehr gut zum Abscheiden von weiteren Monolagen, z.B. bestehend aus natürlichen oder synthetischen Lipiden. So generierte Doppelschichten simulieren an ihrer Oberfläche die Eigenschaften von natürlichen Zellmembranen und sind besonders geeignet für die funktionelle Immobilisierung von membranverankerten oder membrangebundenen Erkennungselementen wie Zellrezeptoren, oder sogar ganzen Membranpartikeln, die bekannterweise sehr empfindlich mit Veränderungen auf Kontakt mit Medien mit anderen physikalisch-chemischen Eigenschaften als ihre natürliche Membranumgebung reagieren. So können kleinste Veränderungen der Proteinstruktur infolge des Kontakts mit einer hydrophoben, harten Sensoroberfläche zum totalen Ausfall der Rezeptorfunktionalität führen.

### Teil A) Erzeugung von biokompatiblen Doppelschichten bestehend aus einer ersten Monolage DDP mit hydrophober Oberfläche und einer zweiten Monoschicht von Lipiden, die durch spontanes Spreiten von Lipidvesikeln auf der ersten hydrophoben Oberfläche erzeugt wurde.

Entsprechend der Beschreibung in Beispiel 3 wurden hydrophobe Sensoroberflächen mit einer Monoschicht aus DDP erzeugt. Lipidvesikel wurden aus synthetischem Lipid 1-Palmitoyl(C₁₆)-2-Oleoyl (C₁₈)-*SN*-Glycero-3-Phosphocholin (POPC) hergestellt (Avanti Polar Lipids, Katalognr. 850457). Dazu wurden 6 mg trockenes POPC in 3 ml Vesikelpuffer (150 mM NaCl, 10 mM Phosphatpuffer, 0.002% NaN₃, pH 7.5) aufgenommen, ca. 12 h lang im Kühlschrank bei 4°C inkubiert und anschliessend gemischt, was zu einer trüben Suspension führte. Monoschichtige Lipidvesikel mit einer Grösse von etwa 100 nm wurden dann durch Extrusion (ca. 20-mal) durch 100 nm Porenfilter (Extruder ?) erzeugt. Die Vesikelformung wurde anhand einer zunehmenden Aufklarung der Suspension verfolgt. Die hydrophoben Oberflächen der Sensorchips wurden mit verdünnter Vesikelsuspension (0.5 mg/ml) mit aufgesetzten Probenbehältnissen eine Stunde lang bei Raumtemperatur inkubiert. Überschüssige Vesikelsuspension wird durch ausgiebiges Spülen mit Vesikelpuffer verdrängt. Die Kinetik der Ausbildung einer zweiten Monolage von Lipiden durch Vesikelspreitung wurde mithilfe von fluoreszenzmarkierten Vesikeln, welche 0.1% eines mit Farbstoff gelabelten Lipids (1,2-Dioleoyl(C₁₈)-*SN*-Glycero-3-Phosphoethanolamin-Fluorescein (DOPE-Fluorescein) enthielten, mittels Fluoreszenzmessung in der in Beispiel 3.4 beschriebenen Messapparatur verfolgt. Die Kinetik der Ausbildung der zweiten Lipidschicht (mit 0.1 % Farbstofflipid) auf der DDP-Schicht ist in Figur 15 gezeigt. Die Signaländerung zwischen anfänglicher Grundlinie und schliesslichem Signalniveau nach Spülen der Oberfläche entspricht dem Signal einer Monolage. Dieses Signal beträgt etwa 50% des Signals bei Ausbildung einer Doppelschicht von in gleicher Weise fluoreszenzmarkierten Lipiden, wenn eine gleichartige Vesikelsuspension mit einer blanken, hydrophilen Metalloxidschicht als Sensoroberfläche (ohne hydrophobe Erstschicht) inkubiert wird.

### Teil B) Demonstration der Biokompatibilität einer DDP-Lipid-Doppelschicht für die Immobilisierung von Membranrezeptoren, nachgewiesen mit einem Bindungsassay an auf der Doppelschicht immobilisierten nativen Membranfragmenten mit eingebundenem Membranenzym Na,K-ATPase.

Das Enzym Na,K-ATPase, integraler Bestandteil natürlicher Zellmembranen, reguliert in seiner Funktion als Ionenpumpe (von K⁺ und Na⁺ Ionen) das Membranpotential lebender Zellen. Es wurde ein fluoreszenzbasierendes Assay für das spezifische Binden von K⁺-Ionen an das Enzym auf Sensoroberflächen entwickelt. Das K⁺-Bindungssignal gilt als ein empfindliches Mass für die Funktionaliät des Enzyms, dem Pumpen der K⁺-Ionen über die Zellmembran. Die für den Pumpvorgang benötigten Ionen werden durch das spezifische Anbinden dieser Ionen ans Protein bereitgestellt.

Es wurden natürliche Membranfragmente, die das Membranenzym Na,K-ATPase in ihrer Membran eingebunden haben, auf ein in Teil A beschriebenes Doppelschichtsystem, DDP (aus wässriger Phase)-Lipid auf einem planaren Wellenleiterchip als Sensorplattform immobilisert. Die funktionale und stabile Immobiliserung der Membranfragmente und damit der Enzyme wird durch die Fähigkeit, oben beschriebene K⁺ Bindungssignale zu generieren, nachgewiesen. Bei ansonsten vergleichbarem Immobilisieren der Membranfragmente auf blanke Sensorchips ohne Doppelschichtsystem geht die Fähigkeit der Generierung des K⁺-Bindungssignals verloren. Damit wird die biokompatible Wirkung der vorgestellten Haftvermittlungsschicht, hier ein Doppelschichtsystem, das frei von organischen Lösungsmittelresten ist, bestehend aus jeweils einer Schicht aus Alkylphosphaten und Lipiden, auf die funktionale Immobilisierung von Membranproteine und ganze Membranfragmente demonstriert. Das Immobilisieren des Membranenzyms mit der Möglichkeit des Kontaktes zur blanken Sensoroberfläche führt zu Denaturieren und damit Funktionsverlust des zu untersuchenden Proteins.

### Besehreibung der Abbildungen:

**Fig. 1****:** Schematische Darstellung der geordeneten Struktur von Alkylphosphat SAMs auf einer Oxidoberfläche
**Fig. 2**: Bindungsverhältnisse in der geordneten SAM Schicht zwischen Alkylphosphaten und einer Tantaloxidoberfläche (Ta₂O₅)
**Fig. 3****:** Mikrotropfendichtemessungen an DDPO₄-SAMs auf verschiedenen glatten Metalloxidoberflächen (Bildgrösse: 1 mm²): a) Al₂O₃, b) Ta₂O₅, c) Nb₂O₅, d) ZrO₂, e) Fe₂O₃, f) TiO₂, g) Ti_{0.4}Si_{0.6}O₂ and h) SiO₂. Siehe Text.
**Fig. 4****:.** DDPO₄-SAM auf der Ti_{0.4}Si_{0.6}O₂-Oberfläche mit einem 2 mm breiten Streifen aus Fe₂O₃. Die Fe₂O₃-Oberfläche ist mit DDPO₄ belegt und hydrophob (niedrige Mikrotropfendichte), während das Substrat (Ti_{0.4}Si_{0.6}O₂) stärker hydrophil bleibt, da nur unvollständig mit DDPO₄ belegt.
**Fig. 5a****:** O1s XPS-Signal von OH-DDPO₄ SAM-Schichten auf Ta₂O₅-Substraten: Die O1s Spektren bei den verschiedenen Austrittswinkeln (20.5° = oberste Kurve a; 11.5° = mittlere Kurve b) werden mit dem entsprechenden Signal der DDPO₄-SAM-Schicht (unterste Kurve c) verglichen.
**Fig. 5b****:** O1s XPS-Signal von OH-DDPO₄ SAM-Schichten auf Nb₂O₅-Substraten: Die O1s Spektren bei den verschiedenen Austrittswinkeln (20.5° = oberste Kurve a; 11.5° = mittlere Kurve b) werden mit dem entsprechenden Signal der DDPO₄-SAM-Schicht (unterste Kurve c) verglichen
**Fig. 6a**: Vergleich der Kohlenstoffkonzentration, bestimmt mittels XPS, von OH-DDPO₄-und DDPO₄-SAMs auf Ta₂O₅, in Abhängigkeit des Sinus des Detektionswinkels Θ. (OH-DDPO₄-Kohlenstoff: gefüllte Kreise; DDPO₄-Kohlenstoff: offene Quadrate.)
**Fig. 6b****:** Vergleich der Kohlenstoffkonzentration, bestimmt mittels XPS, von OH-DDPO₄-und DDPO₄-SAMs auf Nb₂O₅, in Abhängigkeit des Sinus des Detektionswinkels Θ. (OH-DDPO₄-Kohlenstoff: gefüllte Kreise; DDPO₄-Kohlenstoff: offene Quadrate).
**Fig. 7a****:** XPS-Peakflächen als Funktion des Sinus des Detektionswinkels Θ von Nb(3d), C(1s) und P(2p) auf Nb₂O₅. Das O(1s) Signal wurde in drei Komponenten dekonvoluiert: O(Nb₂O₅): Quadrate, O(PO₄): Dreiecke, O(ROH): Kreise. Ähnliche Resultate wurden auch im Falle des Substrates Ta₂O₅ gefunden.
**Fig. 7b****:** XPS-Peakflächen als Funktion des Sinus des Detektionswinkels Θ der verschiedenen Sauerstoffe für OH-DDPO₄-SAM auf Nb₂O₅. Das O(1s) Signal wurde in drei Komponenten dekonvoluiert: O(Nb₂O₅): Quadrate, O(PO₄): Dreiecke, O(ROH): Kreise. Ähnliche Resultate wurden auch im Falle des Substrates Ta₂O₅ gefunden.
**Fig. 8**. Kontaktwinkel von gemischten OH-DDPO₄/DDPO₄-SAMs auf Ta₂O₅. Die Resultate sind als Funktion des Mischungsverhältnisses in der SAM-Lösung (Vol-%) dargestellt. Die totale Alkylphosphat-Konzentration wurde dabei konstant gehalten (0.5 mM).
**Fig. 9** Oberfläche einer durch Strahlen und chemisch Ätzen aufgerauhten Oberfläche einer Rein-Titan (cp Ti) Probe. Herkunft der Probe: Institut Straumann AG, Waldenburg, Schweiz. Rasterlektronenmikroskopische Aufnahme.
**Fig. 10**. Kontaktwinkel als Mass für die Benetzbarkeit von glatten, metallischen Titanoberflächen, behandelt mit wässrigen Lösungen der Salze DDPO₄(NH₄)₂, OH-DDPO₄(NH₄)₂ bzw. deren Mischungen (obere Datenreihe: fortschreitender Kontaktwinkel, untere Datenreihe: rückschreitender Kontaktwinkel).
**Fig. 11**. Kontaktwinkel als Mass für die Benetzbarkeit von rauhen, metallischen Titanoberflächen, behandelt mit wässrigen Lösungen der Salze DDPO₄(NH₄)₂, OH-DDPO₄(NH₄)₂ bzw. deren Mischungen (obere Datenreihe: fortschreitender Kontaktwinkel, untere Datenreihe: rückschreitender Kontaktwinkel).
**Fig. 12****:** Korrigierter Signalverlauf unter Zugabe ansteigender Konzentrationen des Analyten Kaninchen-IgG, detektiert mittels fluoreszenzbasierendem Sandwichassay auf Dünnschichtwellenleitern als Sensorplattformen, auf denen die spezifischen Erkennungsantikörper auf einer Monolage von Dodecylphosphat (DDP) immobilisiert wurden. In den sequentiellen Assayschritten folgt der Zugabe des Analyten während 4 min jeweils ein Spülschritt mit Pufferlösung (8 min).
**Fig. 13a****:** Korrigierter Signalverlauf unter Zugabe ansteigender Konzentrationen des Analyten hIL-6, detektiert in einem fluoreszenzbasierenden Sandwichassay auf Dünnschichtwellenleitern als Sensorplattformen, auf denen die spezifischen Erkennungsantikörper in Anwesenheit und Abwesenheit einer Monolage von Dodecylphosphat (DDP) immobilisiert wurden. Deutlich zu erkennen sind die kräftigen Signalanstiege in Anwesenheit einer Monoschicht DDP, was auf eine funktionale Immobilisierung einer grossen Anzahl von Erkennungsantikörpern schliessen lässt.
**Fig. 13b**: Signal-Konzentrations-Abhängig-keit (Dose-Response Kurve) generiert aus den stationären Signalantworten gemäss Fig. 13a (Chip mit DDP Monoschicht). Der Verlauf der Datenpunkte entspricht einer Langmuir Bindungsisotherme (1:1 Bindung), die an die Daten angefittet wurde.
**Fig. 14**: Relative Assaysignale von Experimenten zum Nachweis von Kaninchen-IgG (250 pM), durchgeführt auf Chipoberflächen mit Monoschichten, die aus unterschiedlichen, wässrigen Mischungen von OH-terminiertem DDP und hydrophob terminiertem DDP abgeschieden wurden. Die Fehlerbalken zeigen die Abweichungen von Messungen auf zwei Chips. Normiert wurde auf die Signale bei 60% OH-terminiertem DDP-Anteil.
**Fig. 15**: Signalverlauf bei der Ausbildung einer zweiten Monochicht aus fluoreszenzmarkierten Lipiden (POPC mit 0.1 % DOPE-Fluorescein) durch Spreiten von Lipidvesikeln (Durchmesser ca. 110 nm) auf einer hydrophoben DDP-Monoschicht auf einem Metalloxid-Dünnschichtwellenleit-er als Sensorplattform.

## Patentansprüche

1. Verfahren zur Abscheidung von Mono- oder Mehrfachschichten aus Organophosohorsäuren der allgemeinen Formel I(A)
Y-B-OPO₃H₂ (IA)
oder aus Organophosphonsäuren der allgemeinen Formel I(B)
Y-B-PO₃ H₂ (IB)
und deren Salzen, in denen B einen Alkyl-, Alkcnyl-, Alkinyl-, Aryl-, Aralkyl-, Hetaryl- oder Hetarylalkylrest und Y Wasserstoff oder eine funktionelle Gruppe aus der Reihe Hydroxy, Carboxy, Amino, gegebenfalls durch Niederalkyl substituiertes Mono-oder Dialkylamino, Thiol, oder eine negative Säuregruppe aus der Reihe Ester, Phosphat, Phosphonat, Sulfat, Sulfonat, Maleimid, Succinimydyl, Epoxy, Acrylat bedeutet, wobei an B oder Y ein biologisches, biochemisches oder synthetisches Erkennungselement durch Additions- oder Substitutionsreaktion angedockt sein kann, wobei auch Verbindungen angelagert sein können, die der Substratoberfläche eine Resistenz gegen Proteinadsorption und/oder Zelladhäsion verleihen und in B in der Kette gegebenenfalls anstelle einer oder mehrerer-CH₂- Gruppen eine oder mehrere Ethylenoxidgruppen enthalten sein können, auf Substratoberflächen von reinen oder gemischten Oxiden, Nitriden oder Carbiden von Metallen und Halbleitern, **dadurch gekennzeichnet, dass** zur Behandlung dieser Oberflächen, insbesondere als Oberflächen von Sensorplatten, Implantaten und medizinischen Hilfsgeräten, wasserlösliche Salze einer Verbindung der Formel (IA) bzw. (IB) in wässriger Lösung eingesetzt werden.

2. Verfahren nach Anspruch 1, **dadurch gekennzeichnet, dass** vor der Abscheidung besagter Mono- oder Mehrfachschichten das wasserlösliche Salz einer Verbindung der Formel (IA) und / oder (IB)in wässriger Lösung isoliert wird.

3. Verfahren nach einem der Ansprüche 1 - 2, **dadurch gekennzeichnet, dass** als Salze der Verbindungen der Formel (TA) und / oder (IB) insbesondere Natrium-, Kalium- und Ammoniumsalze verwendet werden.

4. Verfahren nach einem der Ansprüche 1-3, **dadurch gekennzeichnet, dass** als Substrate Oxide, Nitride oder Carbide von Tantal, Niob, Titan, Vanadium Zirkon, Hafnium, Molybdän, Wolfram, Silizium oder deren Mischungen in Form massiver Körper oder als Schichten auf beliebigen Unterlagen verwendet werden.

5. Verfahren nach einem der Ansprüche 1 -4, **dadurch gekennzeichnet, dass** man Verbindungen der Formel (IA) und / oder (IB), in denen die Gruppen B und Y gemeinsam eine Alkylgruppe oder eine gegebenenfalls substituierte Alkylgruppe von 2-24 C-Atomen bedeuten, zur Beschichtung verwendet.

6. Verfahren nach einem der Ansprüche 1- 5, **dadurch gekennzeichnet, dass** man Verbindungen der Formel (IA) und / oder (IB), in denen die Gruppen B und Y gemeinsam eine Alkylgruppe oder eine gegebenfalls substituierte Alkylgruppe von 2-12 C-Atomen bedeuten, zur Beschichtung verwendet.

7. Verfahren nach einem der Ansprüche 1 - 6, **dadurch gekennzeichnet, dass** man Verbindungen der Formel (IA) und / oder (IB), in denen die Gruppen B und Y gemeinsam eine Alkylgruppe von 10 - 12 C-Atomen bedeuten, zur Beschichtung verwendet.

8. Verfahren nach einem der Ansprüche 1 - 6, **dadurch gekennzeichnet, dass** man Verbindungen der Formel (IA) und / oder (IB), in denen die Gruppen B und Y gemeinsam eine Niederalkylgruppe von 2 - 5 C-Atomen bedeuten, zur Beschichtung verwendet.

9. Verfahren nach einem der Ansprüche 1 - 8, **dadurch gekennzeichnet, dass** als an B oder Y angedockte biochemische, biologische oder synthetische Erkennungselemente solche ausgewählt werden aus der Gruppe von Nukleinsäuren, wie z.B. DNA, RNA, Oligonukleotide, Nukleinsäureanaloge, wie z.B. PNA, mono- oder polyklonalen Antikörpern, Peptiden, Enzymen, Aptameren, synthetischen Peptidstrukturen, löslichen menbrangebundenen und aus einer Membran isolierten Proteinen, wie z.B. Rezeptoren, deren Liganden, Antigenen für Antikörper, Biotin, "Histidin-Tag-Komponenten" und deren Komplexbildungspartnern.

10. Verfahren nach einem der Ansprüche 1 - 8, **dadurch gekennzeichnet, dass** ein biologisch wirksames Erkennungselement Peptide, Proteine, Glycoproteine, Wachstumsfaktor, wie z.B. TGF-β oder BMP (Bone Morphogenic Protein) umfasst.

11. Verfahren nach einem der Ansprüche 1 - 10, **dadurch gekennzeichnet, dass** als Verbindungen, die der Substratoberfläche eine Resistenz gegen Proteinadsorption und/oder Zelladhäsion verleihen, diese vorzugsweise ausgewählt sind aus den Gruppen von Verbindungen, die von Oligo(ethylenoxid), Phosphorylcholin, Heparin, Sacchariden, Albuminen, insbesondere Rinderserumalbumin oder Humanserumalbumin, Casein, unspezifischen, polyklonalen oder monoklonalen, artfremden oder empirisch für den oder die nachzweisenden Analyten unspezifischen Antikörpern, insbesondere für Immunoassays, Detergentien - wie beispielsweise Tween 20 -, nicht mit zu analysierenden Polynukleotiden hybridisierender, fragmentierter natürlicher oder synthetischer DNA, wie beispielsweise ein Extrakt von Herings- oder Lachssperma, insbesondere für Polynuklcotid-Hybridisicrungsassays, oder auch ungeladenen, aber hydrophilen Polymeren, wie beispielsweise Polyethylenglycole oder Dextrane, gebildet werden.

12. Verfahren nach einem der Ansprüche 1 - 11, **dadurch gekennzeichnet, dass** Verbindungen der Formel (IA) und / oder (IB) verschiedene funktionelle Gruppen und / oder biologische oder biochemische oder synthetische Erkennungselemente Erkennungselemente und / oder Verbindungen zur Verleihung einer Resistenz der Oberfläche gegen Proteinadsorption und / oder Zelladhäsion in einem SAM-Molekül umfassen.

13. Verfahren nach einem der Ansprüche 1-12, **dadurch gekennzeichnet, dass** auf einer ersten Monoschicht aus Organophosphaten und / oder Organophosphonaten eine zweite oder sequentiell noch weitere Monoschichten in wässriger Lösung abgeschieden werden, so dass auf besagten Substratoberflächen eine Doppelschicht oder Mehrfachschicht erzeugt wird.

14. Verfahren nach Anspruch 1 und 13, **dadurch gekennzeichnet, dass** durch die erste Monoschicht aus Organophosphaten und / oder Organophosphonaten eine hydrophobe Oberfläche in wässriger Lösung abeschieden wird, auf der eine weitere Schicht aus synthetischen oder natürlichen Lipiden abgeschieden wird.

15. Verfahren nach Anspruch 1 und 14, **dadurch gekennzeichnet, dass** die besagte weitere Schicht aus synthetischen oder natürlichen Lipiden aus einer Lipidvesikelsuspension durch spontane Anlagerung der Vesikel an die hydrophobe Oberfläche einer ersten Monoschicht aus Organophosphaten und / oder Organophosphonaten, gefolgt vom Ausspreiten der Vesikelmembran auf dieser ersten Monoschicht, erzeugt wird.

16. Verfahren nach einem der Ansprüche 1, 14 - 15, **dadurch gekennzeichnet, dass** die synthetischen oder natürlichen Lipide ausgewählt sind aus der Gruppe, die von Phosphoglycerolipiden etc. in wässriger Lösung, gebildet wird, oder eine Mischung dieser Molckülc umfassen.

17. Verfahren nach einem der Ansprüche 1, 13 - 16, **dadurch gekennzeichnet, dass** mit der besagten weiteren Schicht molekulare Gruppen Y und / oder B und /oder biologische oder biochemische oder synthetische Erkennungselemente und / oder Verbindungen, die der Oberfläche eine Resistenz gegen Proteinadsorption und / oder Zelladhäsion in wässriger Lösung verleihen, nach einem der Ansprüche 1-11, assoziiert sind.

18. Verfahren nach einem der Ansprüche 1, 13 - 17, **dadurch gekennzeichnet, dass** auf einer Mehrfachschicht mit einer ersten Monoschicht aus Organophosphaten und / oder Organophosphonaten auf einer Substratoberfläche synthetische oder natürliche Vesikel oder Mikrosomen in wässriger Lösung immobilisiert werden, gegebenfalls mit damit assoziierten biologischen oder biochemischen oder synthetischen Erkennungselementen, welche ausgewählt sind aus der Gruppe, die von Nukleinsäuren, beispielsweise DNA, RNA, Oligonukleotiden, und Nukleinsäureanalogen, z. B. PNA, mono- oder polyklonalen Antikörpern, Peptiden, Enzymen, Aptameren, synthetischen Peptidstrukturen, löslichen, membrangebundenen und aus einer Membran isolierten Proteinen, wie beispielsweise Rezeptoren, deren Liganden, Antigenen für Antikörper, Biotin, "Histidin-Tag-Komponenten" und deren Komplexbildungspartnern gebildet wird.

19. Verfahren nach Ansprüchen 1 - 18, **dadurch gekennzeichnet, dass** es sich bei den abgeschiedenen Schichten um gemischte Mono- oder Mehrfachschichten aus Verbindungen der Formeln I(A) und/oder I(B) und deren Salzen handelt.

20. Verfahren nach einem der Ansprüchen 1 - 19, **dadurch gekennzeichnet, dass** vor der Abscheidung besagter gemischter Mono- oder Mehrfachschichten das wasserlösliche Salz einer Verbindung der Formel (IA) und / oder (IB) in wässriger Lösung isoliert wird.

21. Verfahren nach einem der Ansprüche 1, 19-21, **dadurch gekennzeichnet, dass** als Salze der Verbindungen der Formel (IA) und / oder (IB) insbesondere Natrium-, Kalium- und Ammoniumsalze in wässriger Lösung verwendet werden.

22. Verfahren nach einem der Ansprüche 1, 19 - 21, **dadurch gekennzeichnet, dass** als Substrate Oxide, Nitride oder Carbide von Tantal, Niob, Titan, Vanadium Zirkon, Hafnium, Molybdän, Wolfram, Silizium oder deren Mischungen in Form massiver Körper oder als Schichten auf beliebigen Unterlagen in wässriger Lösung verwendet werden.

23. Verfahren nach einem der Ansprüche 1, 19 - 22, **dadurch gekennzeichnet, dass** man Verbindungen der Formel (IA) und / oder (IB), in denen die Gruppen B und Y gemeinsam eine Alkylgruppe oder eine gegebenenfalls substituierte Alkylgruppe von 2-24 C-Atomen bedeuten, zur Beschichtung in wässriger Lösung verwendet.

24. Verfahren nach einem der Ansprüche 1, 19 - 23, **dadurch gekennzeichnet, dass** als an B oder Y angedockte biochemische, biologische oder synthetische Erkennungselemente solche ausgewählt werden aus der Gruppe von Nukleinsäuren, wie z.B. DNA, RNA, Oligonukleotide, Nukleinsäureanaloge, wie z.B. PNA, mono- oder polyklonalen Antikörpern, Peptiden, Enzymen, Aptameren, synthetischen Peptidstrukturen, löslichen menbrangebundenen und aus einer Membran isolierten Proteinen, wie z.B. Rezeptoren, deren Liganden, Antigenen für Antikörper, Biotin, "Histidin-Tag-Komponenten" und deren Komplexbildungspartnern.

25. Verfahren nach einem der Ansprüche 1, 19 - 24, **dadurch gekennzeichnet, dass** ein biologisch wirksames Erkennungselement Peptide, Proteine, Glycoproteinc, Wachstumsfaktor, wie z.B. TGF-β oder BMP (Bonc Morphogenic Protein) umfasst.

26. Verfahren nach einem der Ansprüche 1, 19 - 25, **dadurch gekennzeichnet, dass** als Verbindungen, die der Substratoberfläche eine Resistenz gegen Proteinadsorption und/oder Zelladhäsion verleihen, diese vorzugsweise ausgewählt sind aus den Gruppen von Verbindungen, die von Oligo(ethylenoxid), Phosphorylcholin, Heparin, Sacchariden, Albuminen, insbesondere Rinderserutnalbumin oder Hurnanserurnalbumin, Casein, unspezifischen, polyklonalen oder monoldonalen, artfremden oder empirisch für den oder die nachzweisenden Analyten unspezifischen Antikörpern, insbesondere für Immunoassays, Detergentien - wie beispielsweise Tween 20 -, nicht mit zu analysierenden Polynukleotiden hybridisierender, fragmentierter natürlicher oder synthetischer DNA, wie beispielsweise ein Extrakt von Herings- oder Lachssperma, insbesondere für Polynukleotid-Hybridisierungsassays, oder auch ungeladenen, aber hydrophilen Polymeren, wie beispielsweise Polyethylenglycole oder Dextrane, gebildet werden.

27. Verfahren nach einem der Ansprüche 1, 19 - 26, **dadurch gekennzeichnet, dass** Verbindungen der Formel (IA) und / oder (IB) verschiedene funktionelle Gruppen und / oder biologische oder biochemische oder synthetische Erkennungselemente in einem SAM-Molekül umfassen.

28. Verfahren nach einem der Ansprüche 1, 19 - 27, **dadurch gekennzeichnet, dass** die Substrate eine glatte Oberfläche mit niedriger Rauheit aufweisen oder rauhe Oberflächen besitzen, wobei diese Oberflächen gegebenenfalls eine zusätzliche Strukturierung aufweisen..

29. Verfahren nach einem der Ansprüche 1, 19-28, **dadurch gekennzeichnet, dass** durch Wahl des Mischungsverhältnisses die Hydrophilizität oder Hydrophobizität der Oberfläche kontrolliert wird.

30. Verfahren nach einem der Ansprüche 1, 19 -29 , **dadurch gekennzeichnet, dass** durch Wahl des Mischungsverhältnisses eine kontrollierte Dichte von positiven und / oder negativen Ladungen auf der Oberfläche eingestellt werden kann.

31. Verfahren nach einem der Ansprüche 19-30, **dadurch gekennzeichnet, dass** durch Wahl des Mischungsverhältnisses eine kontrollierte Dichte von reaktiven Gruppen und / oder biochemischen Erkennungselementen oder biologischen "Funktionen" eingestellt werden kann.

32. Verfahren nach Anspruch 1 zur Erzeugung von chemisch strukturierten Oberflächen durch lokale Abscheidung von Mono- oder Mehrfachschichten aus Organophosphaten und / oder Organophosphonaten auf Substratoberflächen von reinen oder gemischten Oxiden, Nitriden oder Carbiden von Metallen oder Halbleitern, **dadurch gekennzeichnet, dass** zur Behandlung der Oberflächen wasserlösliche salzartige Verbindungen der zugehörigen Organophosphorsäure oder Organophosphonsäure in wässriger Lösung eingesetzt werden und dass die Abscheidung der reinen oder gemischten Mono- oder Mehrfachschichten mittels eines Verfahrens nach einem der Ansprüche 1 - 31 erfolgt.

33. Verfahren nach Anspruch 32, **dadurch gekennzeichnet, dass** die Substratoberfläche ein definiertes Muster mit Siliziumoxid bzw. Übergungsmetalloxiden aufweist.

34. Verfahren nach Anspruch 33, **dadurch gekennzeichnet, dass** auf den Siliziumoxidbereichen eine weitere Abscheidung von Mono- oder Mehrfachschichten aus Organophosphaten und / oder Organophosphonaten aus wässrigen Lösungen durchgeführt wird.

35. Verfahren nach einem der Ansprüche 1 - 34, **dadurch gekennzeichnet, dass** die Abscheidung der Mono- oder Mehrfachschichten aus wässrigen Lösungen, gegebenenfalls lokal selektiv, unter Verwendung einer Abscheidungsmethode erfolgt, die ausgewählt ist aus der Gruppe, die von Tauchen, Aufstreichen, Aufpinseln, "Ink jet spotting", mechanischem Spotting mittels Stift, Feder oder Kapillare, "micro contact printing", fluidischer Kontaktierung der Substratoberfläche mit parallelen oder gekreuzten Mikrokanälen, unter Einwirkung von Druckunterschieden oder elektrischen oder elektromagnetischen Potentialen" etc. gebildet wird.

36. Verfahren zur Erzeugung von chemisch strukturierten Oberflächen nach einem der Ansprüche 1, 32 - 35, **dadurch gekennzeichnet, dass** durch lokale Abscheidung von Mono-oder Mehrfachschichten aus Organophosphaten und / oder Organophosphonaten auf Substratoberflächen von reinen oder gemischten Oxiden, Nitriden oder Carbiden von Metallen oder Halbleitern lokale hydrophile oder hydrophobe Bereiche erzeugt werden und deren umgebende Substratoberfläche anschliessend mit einer endständig hydrophoben beziehungsweise hydrophilen Monoschicht in wässriger Lösung abgedeckt wird.

37. Verfahren, **dadurch gekennzeichnet, dass** auf einer nach einem Verfahren gemäss einem der Ansprüche 1, 32 - 35 erzeugten chemisch strukturierten Oberfläche sequentiell eine oder mehrere Monoschichten nach einem der Ansprüche 13 - 18 lokal abgeschieden werden und damit lokal Mehrfaehschichten in wässriger Lösung erzeugt werden.

38. Verfahren nach Anspruch 1 zur Herstellung von Implantatoberflächen mit Implantaten aus oxidbedeckten Metallen wie z.B. Titan, Tantal, Niob, Legierungen wie Titan-Aluminium-Vanadium, Titan-Aluminium-Niob, Titan-Niob-Zirkon, Titan-Niob-Zirkon-Tantal, Cobalt-Chrom, Cobalt-Chrom-Molybdän, Eisten-Nickel-Chrom, **dadurch gekennzeichnet, dass** auf der Oberfläche die Abscheidung von reinen oder gemischten Mono- oder Mehrfachschichten aus Organophosphaten und / oder Organophosphonaten nach einem der Ansprüche 1 - 12 oder 19 - 37 in wässriger Lösung erfolgt.

39. Verfahren nach Anspruch 1 zur Herstellung von Sensorplattformoberflächen, **dadurch gekennzeichnet, dass** auf der Oberfläche die Abscheidung von reinen oder gemischten Mono- oder Mehrfachschichten aus Organophosphaten und / oder Organophosphonaten nach einem der Ansprüche 1 - 37 in wässriger Lösung erfolgt.

40. Implantat aus Metall oder Keramik mit einer Monoschicht aus Organophosphaten und / oder Organophosphonaten als Oberfläche, **dadurch gekennzeichnet, dass** die Oberfläche erhältlich ist durch ein Abscheidungsverfahren in wässriger Lösung nach einem der Ansprüche 1 - 12 oder 19 - 37.

41. Implantat nach Anspruch 40, **dadurch gekennzeichnet, dass** das Implantat ausgewählt ist aus der Gruppe von Wurzelimplantaten für den Dentalbereich, künstlichen Prothesen, wie z.B. Hüftgelenkschäften, -kugeln bzw. -pfannen, künstlichen Kniegelenken, Osteosynthesekomponenten, wie z.B. Knochenplatten, Schrauben, "fixateur externe", Komponenten zur Reparatur von Schäden im Schädelbereich, "maxillofacial devices", Komponenten im Bereich der Rückgratchirurgie, "spinal surgery implants", Stents, Herzschrittmacherkomponenten.

42. Medizinische Hilfsgcrätc aus Metallen oder Keramik mit einer Mono- oder Mehrfachschicht aus Organophosphaten und / oder Organophosphonaten als Oberfläche, **dadurch gekennzeichnet, dass** die Oberfläche erhältlich ist durch ein Abscheidungsverfahren in wässriger Lösung nach einem der Ansprüche 1 - 12 oder 19 - 37.

43. Medizinische Hilfsgeräte aus Metallen oder Keramik nach Anspruch 42, **dadurch gekennzeichnet, dass** diese ausgewählt sind aus der Gruppe umfassend Katheter, Ballonkatheter, Endoskope, Komponenten für körperexterne, blutführende Systeme wie z.B. Herz-Kreislaufmaschinen.

44. Sensorplattform mit einer Mono- oder Mehrfachschicht aus Organophosphaten und / oder Organophosphonaten als Oberfläche, **dadurch gekennzeichnet, dass** die Oberfläche erhältlich ist durch ein Abscheidungsverfahren in wässriger Lösung nach einem der Ansprüche 1 - 37.

45. Sensorplattform nach Anspruch 44, **dadurch gekennzeichnet, dass** sie mindestens ein Array von in diskreten Messbereichen (d) immobilisierten biologischen oder biochemischen oder synthetischen Erkennungselementen zur spezifischen Erkennung und / oder Bindung eines oder mehrerer Analyten und / oder spezifischen Wechselwirkung mit besagten Analyten umfasst.

46. Sensorplattform nach einem der Ansprüche 44-45, **dadurch gekennzeichnet, dass** der Nachweis des einen oder der mehreren Analyten mithilfe von Labeln erfolgt, welche ausgewählt sind aus der Gruppe, die von beispielsweise Lumincszenzlabeln, insbesondere lumineszenten Interkalatoren oder "molecular beacons", Absorptionslabeln, Massenlabeln, insbesondere Metallkolloiden oder Plastikbeads, Spin-Labeln, wie ESR- oder NMR-Labeln, radioaktiven Labeln gebildet wird.

47. Sensorplattfonn nach einem der Ansprüche 44 - 47, **dadurch gekennzeichnet, dass** der Analytnachweis auf der Bestimmung einer Änderung des effektiven Brechungsindex aufgrund molekularer Adsorption oder Desorption auf den Messbereichen (d) beruht.

48. Sensorplattform nach Anspruch 47, **dadurch gekennzeichnet, dass** der Analytnachweis auf der Bestimmung einer Änderung der Bedingungen zur Erzeugung eines Oberflächenplasmons in der Metallschicht eines Mehrschichtsystems beruht, wobei die Metallschicht vorzugsweise aus Gold oder Silber besteht.

49. Sensorplattform nach einem der Ansprüche 44 - 48, **dadurch gekennzeichnet, dass** der Analytnachweis auf der Bestimmung einer Änderung einer oder mehrerer Lumineszenzen beruht.

50. Sensorplattform nach Anspruch 49, **dadurch gekennzeichnet, dass** das Anregungslicht in einer Auflichtanregung eingestrahlt wird.

51. Sensorplattform nach einem der Ansprüche 44 - 50, **dadurch gekennzeichnet, dass** das mit den Messbereichen in Kontakt stehende Material der Sensorplattform innerhalb einer Tiefe von mindestens 200 nm von den Messbereichen bei mindestens einer Anregungswellenlänge transparent oder absorbierend ist.

52. Scnsorplattform nach Anspruch 50, **dadurch gekennzeichnet, dass** das Anregungslicht in einer Transmissionsanordnung eingestrahlt wird.

53. Sensorplattform nach Anspruch 52, **dadurch gekennzeichnet, dass** das Material der Sensorplattform bei mindestens einer Anregungswellenlänge transparent ist.

54. Sensorplattform nach einem der Ansprüche 44 - 53, **dadurch gekennzeichnet, dass** die Sensorplattform als ein optischer Wellenleiter ausgebildet ist, welcher vorzugsweise im wesentlichen planar ist.

55. Sensorplattform nach Anspruch 53, **dadurch gekennzeichnet, dass** die Sensorplatttörnt ein optisch transparentes Material aus der Gruppe umfasst, die von Silikaten, z. B. Glas oder Quarz, transparenten thermoplastischen oder spritzbaren Kunststoff, beispielsweise Palycarbonat, Polyimid, Acrylaten, insbesondere Polymethylmethaerylat, oder Polystyrolen gebildet wird.

56. Sensotplattform nach Anspruch 54, **dadurch gekennzeichnet, dass** die Sensorplarttorm einen optischen Dünnschichtwellenleiter mit einer bei mindestens einer Anregungswellenlänge transparenten Schicht (a) auf einer bei mindestens dieser Anregungswellenlänge ebenfalls transparenten Schicht (b) mit niedrigerem Brechungsindex als Schicht (a) umfasst.

57. Verfahren zum gleichzeitigen qualitativen und / oder quantitativen Nachweis einer Vielzahl von Analyten, **dadurch gekennzeichnet, dass** eine oder mehrere auf besagte Analyten zu untersuchende flüssige Proben mit den Messbereichen (d) auf einer Senserplattform nach einem der Ansprüche 44 - 56 in Kontakt gebracht und resultierende Änderungen von Signalen aus den Messbereichen gemessen werden.

58. Verfahren nach Anspruch 57, **dadurch gekennzeichnet, dass** der Analytnachweis auf der Bestimmung der Änderung einer oder mehrerer Lumineszenzen beruht.

59. Verfahren nach Anspruch 58, **dadurch gekennzeichnet, dass** das Anregungslicht von einer oder mehreren Anregungslichtquellen in einer Auflichtanregung eingestrahlt wird.

60. Verfahren nach Anspruch 58, **dadurch gekennzeichnet, dass** das Anregungslicht von einer oder mehreren Anregungslichtquellen in einer Transmissionsanordnung eingestrahlt wird.

61. Verfahren nach einem der Ansprüche 58 - 60, **dadurch gekennzeichnet, dass** die Sensorplattform als ein optischer Wellenleiter ausgebildet ist, welcher vorzugsweise im wesentlichen planar ist, und dass das Anregungslicht von einer oder mehrerer Lichtquellen eingekoppelt wird in den optischen Wellenleiter mittels eines Verfahrens, welches ausgewählt ist aus der Gruppe, die von Stirnflächenkopplung, Einkopplung über angebrachte optische Fasern als Lichtleiter, Prismenkopplung, Gitterkopplung oder evaneszenter Kopplung durch Überlappung des evaneszenten Feldes besagten optischen Wellenleiters mit dem evaneszenten Feld eines weiteren, damit in Nahfeldkontakt gebrachten Wellenleiters gebildet wird.

62. Verfahren nach einem der Ansprüche 57 - 61, **dadurch gekennzeichnet, dass** die eine oder mehreren Proben mit einer Mischung aus den verschiedenen Nachweisrcagenticn zur Bestimmung der in besagten Proben nachzuweisenden Analyten vorinkubiert werden und diese Mischungen dann in einem einzigen Zugabeschritt den dafür vorgesehenen Arrays auf der Sensorplattform zugeführt werden.

63. Verfahren nach einem der Ansprüche 58 - 62, **dadurch gekennzeichnet, dass** die Kalibration von infolge der Bindung eines oder mehrerer Analyten oder infolge der spezifischen Wechselwirkung mit einem oder mehreren Analyten im Nahfeld der Schicht (a) erzeugten Lumineszenzen die Zugabe von einer oder mehreren Kalibrationslösungen mit bekannten Konzentrationen besagter zu bestimmender Analyten auf die gleichen oder andere Messbereiche oder Segmente von Messbereichen oder Arrays von Messbereichen auf einer Sensorplattform umfasst, denen im gleichen oder einem separaten Zugabeschritt die eine oder die mehreren zu untersuchenden Proben zugeführt werden.

64. Verfahren nach einem der Ansprüche 57 - 63 zur gleichzeitigen oder sequentiellen, quantitativen oder qualitativen Bestimmung eines oder mehrerer Analyten aus der Gruppe von Antikörpern oder Antigenen, Rezeptoren oder Liganden, Chelatoren oder "Histidin-tag-Komponenten", Oligonuldeotiden, DNA- oder RNA-Strängen, DNA- oder RNA-Analoga, Enzymen, Enymcofaktoren oder Inbibitoren, Lektinen und Kohlehydraten.

65. Verfahren nach einem der Ansprüche 57 - 64, **dadurch gekennzeichnet, dass** die zu untersuchenden Proben natürlich vorkommende Körperflüssigkeiten wie Blut, Serum, Plasma, Lymphe oder Urin oder Eigelb oder optisch trübe Flüssigkeiten oder Gewebeflüssigkeiten oder Oberflächenwasser oder Boden- oder Pflanzenextrakte oder Bio- oder Syntheseprozessbrühen oder aus biologischen Gewebeteilen oder aus Zellkulturen oder -extrakten entnommen sind.

66. Verwendung einer Sensorplattform nach einem der Ansprüche 44-56 und / oder eines Verfahrens nach einem der Ansprüche 57 - 65 zu quantitativen oder qualitativen Analysen zur Bestimmung chemischer, biochemischer oder biologischer Analyten in Screeningverfahren in der Pharmaforschung, der Kombinatorischen Chemie, der Klinischen und Präklinischen Entwicklung, zu Echtzeitbindungsstudien und zur Bestimmung kinetischer Parameter im Affinitätsscreening und in der Forschung, zu qualitativen und quantitativen Analytbestimmungen, insbesondere für die DNA- und RNA-Analytik, für die Erstellung von Toxizitätsstudien sowie für die Bestimmung von Gen- oder Protein-Expressionsprofilen sowie zum Nachweis von Antikörpern, Antigenen, Pathogenen oder Bakterien in der pharmazeutischen Protduktentwicklung und -forschung, der Human- und Veterinärdiagnostik, der Agrochemischen Produktentwicklung und - forschung, der symptomatischen und präsymptomatischen Pflanzendiagnostik, zur Patientenstratifikation in der pharmazeutischen Produktentwicklung und für die therapeutische Medikamentenauswahl, zum Nachweis von Pathogenen, Schadstoffen und Erregern, insbesondere von Salmonellen, Prionon, Viren und Bakterien, in der Lebensmittel- und Umweltanalytik.

## Claims

1. Method for precipitating mono or multiple layers of organophosphoric acids of the general formula I (A)
Y-B-OPO₃H₂ (IA)
or of organophosphonic acids of the general formula I (B)
Y-B-PO₃ H₂ (IB)
and the salts thereof, on substrate surfaces of pure or mixed oxides, nitrides or carbides of metals and semiconductors, wherein B is an alkyl, alkenyl, alkinyl, aryl, aralkyl, hetaryl or hetarylalkyl residue and Y is hydrogen or a functional group from the hydroxy, carboxy, amino, optionally low-alkyl-substituted mono or dialkylamino series, thiol, or a negative acid group from the ester, phosphate, phosphonate, sulfate, sulfonate, maleimide, succinimydyl, epoxy, acrylate series, wherein a biological, biochemical or synthetic recognition element may be coupled to B or Y by addition or substitution reaction, wherein compounds may also be added conferring on the substrate surface a resistance to protein adsorption and/or to cell adhesion and in B in the chain may optionally be comprised one or more ethylene oxide groups, rather than one or more -CH₂- groups, **characterized in that** water-soluble salts of a compound of formula (IA) or (IB) in aqueous solution are used for the treatment of these surfaces, in particular as surfaces of sensor platforms, implants and medical accessory devices.

2. Method according to Claim 1, **characterized in that** the water-soluble salt of a compound of formula (IA) and/or (IB) in aqueous solution is isolated before the precipitation of said mono or multiple layers.

3. Method according to either of Claims 1 - 2, **characterized in that** sodium, potassium and ammonium salts in particular are used as salts of compounds of formula (IA) and/or (IB).

4. Method according to any of Claims 1-3 **characterized in that** oxides, nitrides or carbides of tantalum, niobium, titanium, vanadium, zirconium, hafnium, molybdenum, tungsten, silicon or mixtures thereof are used as substrates in the form of solid bodies or as layers on substrates of any kind.

5. Method according to any of Claims 1-4, **characterized in that** compounds of formula (IA) and/or (IB) are used for coating, wherein the B and Y groups are both an alkyl group or an optionally substituted alkyl group of 2 - 24 C atoms.

6. Method according to any of Claims 1-5, **characterized in that** compounds of formula (IA) and/or (IB) are used for coating, wherein the B and Y groups are both an alkyl group or an optionally substituted alkyl group of 2 - 12 C atoms.

7. Method according to any of Claims 1 - 6, **characterized in that** compounds of formula (IA) and/or (IB) are used for coating, wherein the B and Y groups are both an alkyl group of 10 - 12 C atoms.

8. Method according to any of Claims 1 - 6, **characterized in that** compounds of formula (IA) and/or (IB) are used for coating, wherein the B and Y groups are both a lower alkyl group of 2 - 5 C atoms.

9. Method according to any of Claims 1 - 8, **characterized in that** materials for biochemical, biological or synthetic recognition elements (coupled to B or Y) are selected from among the group of nucleic acids, such as DNA, RNA, oligonucleotides, nucleic acid analogs, such as PNA, monoclonal or polyclonal antibodies, peptides, enzymes, aptamers, synthetic peptide structures, soluble membrane-bound proteins and proteins isolated from a membrane, such as receptors, ligands thereof, antigens for antibodies, biotin, "histidine tag components" and complexing partners thereof.

10. Method according to any of Claims 1 - 8, **characterized in that** a biologically effective recognition element comprises peptides, proteins, glycoproteins, growth factor, such as TGF-β, or BMP (bone morphogenic protein).

11. Method according to any of claims 1-10, **characterized in that** compounds which confer on the substrate surface a resistance to protein adsorption and/or to cell adhesion are preferably selected from the groups of compounds which are formed from oligo(ethylene oxide), phosphoryl choline, heparin, saccharides, albumins, especially bovine serum albumin or human serum albumin, casein, nonspecific, polyclonal or monoclonal, heterologous or for the analyte or analytes to be determined empirically nonspecific antibodies (especially for immunoassays), detergents (such as Tween 20), fragmented natural DNA or synthetic DNA not hybridizing with polynucleotides for analysis, such as a herring or salmon sperm extract (especially for polynucleotide hybridization assays), or also uncharged, but hydrophilic polymers, such as polyethylene glycols or dextrans.

12. Method according to any of Claims 1-11, **characterized in that** formula (IA) and/or (IB) include various functional groups and/or biological or biochemical or synthetic recognition elements and/or compounds for conferring on the surface a resistance to protein adsorption and/or to cell adhesion in an SAM molecule.

13. Method according to any of Claims 1 - 12, **characterized in that** a second or further sequential monolayers are precipitated in aqueous solution on a first monolayer of organophosphates and/or organophosphonates, so that a double or multiple layer is produced on said substrate surfaces.

14. Method according to Claim 1 and 13, **characterized in that** the first monolayer of organophosphates and/or organophosphonates produces a hydrophobic surface in aqueoous solution, on which a further layer of synthetic or natural lipids is precipitated.

15. Method according to Claim 1 and 14, **characterized in that** said additional layer of synthetic or natural lipids is produced from a lipid vesicle suspension through spontaneous attachment of the vesicles to the hydrophobic surface of a first monolayer of organophosphates and/or organophosphonates, followed by distribution of the vesicle membrane over this first monolayer.

16. Method according to any of Claims 1, 14 - 15, **characterized in that** the synthetic or natural lipids are selected from the group which is formed from phosphoglycerol lipids etc. in aqueous solution, or comprise a mixture of these molecules.

17. Method according to any of Claims 1, 13 - 16, **characterized in that** molecular groups Y and/or B and/or biological or biochemical or synthetic recognition elements and/or compounds which confer on the surface a resistance to protein adsorption and/or to cell adhesion in aqueous solution are associated with said additional layer according to any of Claims 1 - 11.

18. Method according to any of Claims 1, 13-17, **characterized in that** synthetic or natural vesicles or microsomes in aqueous solution are immobilized on a multiple layer with a first monolayer of organophosphates and/or organophosphonates on a substrate surface, optionally with associated biological or biochemical or synthetic recognition elements selected from the group which is formed from nucleic acids (for example DNA, RNA, oligonucleotides) and nucleic acid analogs (e.g. PNA), monoclonal or polyclonal antibidies, peptides, enzymes, aptamers, synthetic peptide structures, soluble, membrane-bound proteins and proteins isolated from a membrane, such as receptors, ligands thereof, antigens for antibodies, biotin, "histidine tag components" and complex-forming partners thereof.

19. Method according to Claims 1 - 18, **characterized in that** the precipitated layers are mixed mono or multiple layers of compounds of the formula I (A) and/or I (B) and the salts thereof.

20. Method according to any of Claims 1 - 19, **characterized in that** the water-soluble salt of a compound of formula (IA) and/or (IB) is isolated in aqueous solution before the precipitation of said mixed mono or multiple layers.

21. Method according to any of Claims 1, 19-21, **characterized in that** sodium, potassium and ammonium salts in aqueous solution in particular are used as salts of compounds of formula (IA) and/or (IB).

22. Method according to any of Claims 1, 19 - 21, **characterized in that** oxides, nitrides or carbides of tantalum, niobium, titanium, vanadium, zirconium, hafnium, molybdenum, tungsten, silicon or mixtures thereof are used as substrates in the form of solid bodies or as layers on substrates of any kind in aqueous solution.

23. Method according to any of Claims 1, 19 - 22, **characterized in that** compounds of formula (IA) and/or (IB) are used for coating in aqueous solution, wherein the B and Y groups are both an alkyl group or an optionally substituted alkyl group of 2 - 24 C atoms.

24. Method according to any of Claims 1, 19 - 23, **characterized in that** materials for biochemical, biological or synthetic recognition elements (coupled to B or Y) are selected from among the group of nucleic acids, such as DNA, RNA, oligonucleotides, nucleic acid analogs, such as PNA, monoclonal or polyclonal antibodies, peptides, enzymes, aptamers, synthetic peptide structures, soluble membrane-bound proteins and proteins isolated from a membrane, such as receptors, ligands thereof, antigens for antibodies, biotin, "histidine tag components" and complexing partners thereof.

25. Method according to any of claims 1, 19 - 24, **characterized in that** a biologically effective recognition element comprises peptides, proteins, glycoproteins, growth factor, such as TGF-β, or BMP (bone morphogenic protein).

26. Method according to any of Claims 1, 19 - 25, **characterized in that** compounds which confer on the substrate surface a resistance to protein adsorption and/or to cell adhesion are preferably selected from the groups of compounds which are formed from oligo(ethylene oxide), phosphoryl choline, heparin, saccharides, albumins, especially bovine serum albumin or human serum albumin, casein, nonspecific, polyclonal or monoclonal, heterologous or for the analyte or analytes to be determined empirically nonspecific antibodies (especially for immunoassays), detergents (such as Tween 20), fragmented natural DNA or synthetic DNA not hybridizing with polynucleotides for analysis, such as a herring or salmon sperm extract (especially for polynucleotide hybridization assays), or also uncharged, but hydrophilic polymers, such as polyethylene glycols or dextrans.

27. Method according to any of Claims 1, 19 - 26, **characterized in that** compounds of formula (IA) and/or (IB) include various functional groups and/or biological or biochemical or synthetic recognition elements in an SAM molecule.

28. Method according to any of Claims 1, 19 - 27, **characterized in that** the substrates show a smooth surface with low roughness or possess rough surfaces, wherein these surfaces may optionally show additional structuring.

29. Method according to any of Claims 1, 19 - 28, **characterized in that** the hydrophilicity or hydrophobicity of the surface is controlled by selecting the ratio of the mixture.

30. Method according to any of Claims 1, 19 - 29, **characterized in that** a controlled density of positive and/or negative charges on the surface can be adjusted by selecting the ratio of the mixture.

31. Method according to any of Claims 19 - 30, **characterized in that** a controlled density of reactive groups and/or biochemical recognition elements or biological "functions" can be adjusted by selecting the ratio of the mixture.

32. Method according to Claim 1 for producing chemically structured surfaces by local precipitation of monolayers or multiple layers of organophosphates and/or organophosphonates on substrate surfaces of pure or mixed oxides, nitrides or carbides of metals or semiconductors, **characterized in that** aqueous saliniform compounds of the corresponding organophosphoric acid or organophosphonic acid in aqueous solution are used for the treatment of the surfaces and **in that** the pure or mixed monolayers or multiple layers are precipitated using a method according to any of Claims 1 - 31.

33. Method according to Claim 32, **characterized in that** the substrate surface shows a defined pattern with silicon oxide or transition metal oxides.

34. Method according to Claim 33, **characterized in that** mono or multiple layers of organophosphates and/or organophosphonates from aqueous solutions are further precipitated on the silicon oxide areas.

35. Method according to any of Claims 1 - 34, **characterized in that** the mono or multiple layers are precipitated from aqueous solutions, optionally in a local selective manner, using a method selected from the group comprising immersion, spreading, brushing, "inkjet spotting", mechanical spotting by means of a stylus, pen or capillary, "micro-contact printing", fluidic contact of the substrate surface with parallel or crossed microchannels, under the influence of pressure differences or electrical or electromagnetic potentials etc.

36. Method for producing chemically structured surfaces according to any of Claims 1, 32 - 35, **characterized in that** local hydrophilic or hydrophobic areas are produced by local precipitation of mono or multiple layers of organophosphates and/or organophosphonates on substrate surfaces of pure or mixed oxides, nitrides or carbides of metals or semiconductors and their surrounding substrate surface is then coated with a terminally hydrophobic or hydrophilic monolayer in aqueous solution.

37. Method, **characterized in that** one or more monolayers according to any of Claims 13-18 are sequentially locally precipitated on a chemically structured surface produced according to a method defined in any of Claims 1, 32 - 35, and thus multiple layers in aqueous solution are produced locally.

38. Method for according to Claim 1 the manufacture of implant surfaces with implants from oxide-coated metals, such as titanium, tantalum, niobium, alloys such as titanium-aluminium-vanadium, titanium-aluminium-niobium, titanium-niobium-zirconium, titanium-niobium-zirconium-tantalum, cobalt-chromium, cobalt-chromium-molybdenum, iron-nickel-chromium, **characterized in that** pure or mixed mono or multiple layers of organophosphates and/or organophosphonates in aqueous solution are precipitated on the surface according to any of Claims 1 - 12 or 19-37.

39. Method according to Claim 1 for manufacturing sensor platform surfaces, **characterized in that** pure or mixed mono or multiple layers of organophosphates and/or organophosphonates in aqueous solution are precipitated on the surface according to any of Claims 1 - 37.

40. Implant of metal or ceramic with a monolayer of organophosphates and/or organophosphonates as surface, **characterized in that** the surface can be produced using a precipitation method in aqueous solution according to any of Claims 1 - 12 or 19 - 37.

41. Implant according to Claim 40, **characterized in that** the implant is selected from the group of root implants for dental applications, artificial prostheses, such as hip joint stems, balls and sockets, artificial knee joints, osteosynthesis components, such as bone plates, screws, "fixateur externe", components for the repair of damage in the cranial region, "maxillofacial devices", components in the field of spinal surgery, "spinal surgery implants", stents, and cardiac pacemaker components.

42. Medical accessory devices of metal or ceramic with a mono or multiple layer of organophosphates and/or organophosphonates as surface, **characterized in that** the surface can be produced using a precipitation method in aqueous solution according to any of Claims 1 - 12 or 19 - 37.

43. Medical accessory devices of metal or ceramic according to Claim 42, **characterized in that** these accessory devices are selected from the group comprising catheters, balloon catheters, endoscopes, components for exogenous, blood-carrying systems, such as cardiovascular machines.

44. Sensor platform with a mono or multiple layer of organophosphates and/or organophosphonates as surface, **characterized in that** the surface can be produced using a precipitation method in aqueous solution according to any of Claims 1 - 37.

45. Sensor platform according to Claim 44, **characterized in that** it comprises at least one array of biological or biochemical or synthetic recognition elements, immobilized in discrete measurement areas (d), for the specific recognition and/or binding of one or more analytes and/or specific interaction with said analytes.

46. Sensor platform according to any of Claims 44-45, **characterized in that** the one or more analytes are detected by means of labels selected from the group formed from e.g. luminescence labels, especially luminescent intercalators or "molecular beacons", absorption labels, mass labels, especially metal colloids or plastic beads, spin labels, such as ESR or NMR labels, or radioactive labels.

47. Sensor platform according to any of Claims 44 - 47, **characterized in that** the analyte detection is based on the determination of a change in the effective refractive index as a result of molecular adsorption or desorption on the measurement areas (d).

48. Sensor platform according to Claim 47, **characterized in that** the analyte detection is based on the determination of a change in the conditions for generating a surface plasmon in the metal layer of a multiple layer system, wherein the metal layer preferably comprises gold or silver.

49. Sensor platform according to any of Claims 44 - 48, **characterized in that** the analyte detection is based on the determination of a change in one or more luminescences.

50. Sensor platform according to Claim 49, **characterized in that** the excitation light is delivered in a reflected light configuration.

51. Sensor platform according to any of Claims 44 - 50, **characterized in that** the material of the sensor platform that is in contact with the measurement areas is transparent or absorbent within a depth of at least 200 nm from the measurement areas in at least one excitation wavelength.

52. Sensor platform according to Claim 50, **characterized in that** the excitation light is delivered in a transmission configuration.

53. Sensor platform according to Claim 52, **characterized in that** the material of the sensor platform is transparent in at least one excitation wavelength.

54. Sensor platform according to any of Claims 44 - 53, **characterized in that** the sensor platform is formed as an optical waveguide which is preferably essentially planar.

55. Sensor platform according to Claim 53, **characterized in that** the sensor platform comprises an optically transparent material from the group of silicates, e.g. glass or quartz, transparent thermoplastic or moldable plastic, for example polycarbonate, polyimide, acrylates, especially polymethylmethacrylate, or polystyrenes.

56. Sensor platform according to Claim 54, **characterized in that** the sensor platform comprises an optical thin-layer waveguide with a layer (a) which is transparent in at least one excitation wavelength on a layer (b) which is likewise transparent in at least this excitation wavelength with a lower refractive index than layer (a).

57. Method for the simultaneous qualitative and/or quantitative detection of numerous analytes, **characterized in that** one or more liquid samples to be tested for said analytes are brought into contact with the measurement areas (d) on a sensor platform according to any of Claims 44 - 56 and the resulting changes in signals from the measurement areas are measured.

58. Method according to Claim 57, **characterized in that** the analyte detection is based on determining the change in one or more luminescences.

59. Method according to Claim 58, **characterized in that** the excitation light from one or more excitation light sources is delivered in a reflected light configuration.

60. Method according to Claim 58, **characterized in that** the excitation light from one or more excitation light sources is delivered in a transmission configuration.

61. Method according to any of Claims 58 - 60, **characterized in that** the sensor platform is formed as an optical waveguide which is preferably essentially planar, and **in that** the excitation light is coupled into the optical waveguide from one or more light sources using a method selected from the group comprising butt joint coupling, coupling via suitable optic fibres as optical waveguides, prism coupling, grating coupling or evanescent coupling by overlapping of the evanescent field of said optical waveguide with the evanescent field of a further waveguide brought into near-field contact therewith.

62. Method according to any of Claims 57 - 61, **characterized in that** one or more samples are incubated beforehand with a mixture of the various detection reagents for determining the analytes to be detected in said samples and these mixtures then are added in a single step to the arrays set up for this purpose on the sensor platform.

63. Method according to any of Claims 58 - 62, **characterized in that** the calibration of luminescences generated by the binding of one or more analytes or by the specific interaction with one or more analytes in the near field of layer (a) comprises the addition of one or more calibration solutions with known concentrations of said analytes to be determined to the same or different measurement areas or segments of measurement areas or arrays of measurement areas on a sensor platform to which the one or more samples to be tested are added in the same or in a separate step.

64. Method according to any of Claims 57 - 63 for simultaneous or sequential, quantitative or qualitative determination of one or more analytes from the group of antibodies or antigens, receptors or ligands, chelators or "histidine tag components", oligonucleotides, DNA or RNA strands, DNA or RNA analogs, enzymes, enzyme cofactors or inhibitors, lectins and carbohydrates.

65. Method according to any of Claims 57 - 64, **characterized in that** the samples to be tested are naturally occurring body fluids such as blood, serum, plasma, lymph or urine or egg yolk or optically turbid fluids or tissue fluids or surface water or soil or plant extracts or biological or synthetic process broths or are taken from biological tissue parts or from cell cultures or extracts.

66. Use of a sensor platform according to any of Claims 44 - 56 and/or of a method according to any of Claims 57 - 65 for quantitative or qualitative analysis for the determination of chemical, biochemical or biological analytes during screening procedures in pharmaceutical research, combinatorial chemistry, clinical and preclinical development, for real-time binding studies and for the determination of kinetic parameters in affinity screening and in research, for qualitative and quantitative determination of analytes, especially for DNA and RNA analysis, for the performance of toxicity studies, and for the determination of gene or protein expression profiles, as well as for the detection of antibodies, antigens, pathogens or bacteria in pharmaceutical product development and research, human and veterinary diagnosis, agrochemical product development and research, symptomatic and presymptomatic crop diagnosis, for patient stratification in pharmaceutical product development and for the therapeutic selection of medicines, for the detection of pathogens, noxae and germs, especially salmonellae, prions, viruses and bacteria, in food analysis and environmental analysis.

## Revendications

1. Procédé de précipitation de couches simples ou multiples d'acides organophosphoriques de la formule générale 1 (A) :
Y-B-OPO₃H₂ (IA)
ou d'acides organophosphoniques de la formule générale 1(B) :
Y-B-PO₃H₂ (IB)
et leurs sels, dans lesquels B représente un reste alkyle, alcényle, alcynyle, aryle, aralkyle, hétaryle ou hétarylalkyle et Y représente hydrogène ou un groupe fonctionnel de la série hydroxy, carboxy, amino, mono- ou dialkylamino le cas échéant substitué par un alkyle inférieur, thiol, ou un groupe acide négatif de la série ester, phosphate, phosphonate, sulfate, sulfonate, maléimide, succinimidyle, époxy, acrylate, où sur B ou Y, un élément de reconnaissance biologique, biochimique ou synthétique peut être fixé par une réaction d'addition ou de substitution, où également des composés peuvent être additionnés, lesquels confèrent à la surface de substrat, une résistance contre l'adsorption protéique et/ou l'adhésion cellulaire et dans B, un ou plusieurs groupes oxyde d'éthylène peuvent être présents dans la chaîne, le cas échéant à la place d'un ou de plusieurs groupes CH₂, sur des surfaces de substrat d'oxydes, nitrures ou carbures purs ou mixtes, de métaux ou semi-conducteurs, **caractérisé en ce que** pour le traitement de ces surfaces, en particulier comme surfaces de plaques de capteur, implants et appareils médicaux, des sels solubles dans l'eau d'un composé de formule (IA) ou (IB) sont mis en oeuvre en solution aqueuse.

2. Procédé selon la revendication 1, **caractérisé en ce qu'**avant la précipitation des dites couches simples ou multiples, le sel soluble dans l'eau d'un composé de la formule (IA) et/ou (IB) est isolé en solution aqueuse.

3. Procédé selon l'une des revendications 1 à 2, **caractérisé en ce que** comme sel des composés de la formule (IA) et/ou (IB), on utilise en particulier les sels de sodium, de potassium et d'ammonium.

4. Procédé selon l'une des revendications 1 à 3, **caractérisé en ce que** comme substrat, on utilise un oxyde, un nitrure ou un carbure de tantale, de niobium, de titane, de vanadium, de zirconium, de hafnium, de molybdène, de tungstène, de silicium ou leurs mélanges sous forme de corps massifs ou comme couches sur des substrats quelconques.

5. Procédé selon l'une des revendications 1 à 4, **caractérisé en ce que** l'on utilise pour le revêtement, des composés de la formule (IA) et/ou (IB), dans lesquels les groupes B et Y signifient en commun, un groupe alkyle ou un groupe alkyle le cas échéant substitué de 2-24 atomes C.

6. Procédé selon l'une des revendications 1 à 5, **caractérisé en ce que** l'on utilise pour le revêtement, des composés de la formule (IA) et/ou (IB), dans lesquels les groupes B et Y signifient en commun, un groupe alkyle ou un groupe alkyle le cas échéant substitué de 2-12 atomes C.

7. Procédé selon l'une des revendications 1 à 6, **caractérisé en ce que** l'on utilise pour le revêtement, des composés de la formule (IA) et/ou (IB), dans lesquels les groupes B et Y signifient en commun, un groupe alkyle de 10-12 atomes C.

8. Procédé selon l'une des revendications 1 à 6, **caractérisé en ce que** l'on utilise pour le revêtement, des composés de la formule (IA) et/ou (IB), dans lesquels les groupes B et Y signifient en commun, un groupe alkyle inférieur de 2-5 atomes C.

9. Procédé selon l'une des revendications 1 à 8, **caractérisé en ce que** comme élément de reconnaissance biologique, biochimique ou synthétique, lié à B ou Y, on choisit ceux du groupes des acides nucléiques, comme par exemple l'ADN, l'ARN, un oligonucléotide, un analogue d'acide nucléique, comme par exemple un PNA, des anticorps mono- ou polyclonaux, des peptides, des enzymes, des aptamères, des structures peptidiques synthétiques, des protéines solubles associées à des membranes ou isolées d'une membrane, par exemple des récepteurs, leurs ligands, des antigènes pour des anticorps, la biotine, des « composants de queue histidine » et leur partenaire de complexation.

10. Procédé selon l'une des revendications 1 à 8, **caractérisé en ce qu'**un élément de reconnaissance biologique comprend un peptide, une protéine, une glycoprotéine, un facteur de croissance, comme par exemple TGF-β ou BMP (Bone Morphogenic protein).

11. Procédé selon l'une des revendications 1 à 10, **caractérisé en ce que** comme composés, qui confèrent à la surface du substrat, une résistance contre l'adsorption de protéines et/ou l'adhésion cellulaire, ceux-ci sont choisis de préférence, parmi le groupe consistant en des composés, qui sont formés d'oligo(oxyde d'éthylène), la phosphorylcholine, l'héparine, des saccharides, l'albumine, en particulier la sérumalbumine bovine ou la sérumalbumine humaine, la caséine, des anticorps non spécifiques, polyclonaux ou monoclonaux, étrangers ou empiriques pour le ou les analytes à détecter, en particulier pour des immunodosages, des détergents, comme par exemple le Tween 20, un ADN fragmenté naturel ou synthétique, ne s'hybridant pas avec les polynucléotides à analyser, comme par exemple un extrait de sperme de hareng ou de saumon, en particulier pour des dosages d'hybridation de polynucléotide, ou également des polymères non chargés, mais hydrophiles, comme par exemple des polyéthylèneglycols ou des dextranes.

12. Procédé selon l'une des revendications 1 à 11, **caractérisé en ce que** les composés de la formule (IA) et/ou (IB) comprennent différents groupes fonctionnels et/ou éléments de reconnaissance biologiques ou biochimiques ou synthétiques et/ou composés pour conférer une résistance de la surface contre l'adsorption protéique et/ou l'adhésion cellulaire dans une molécule SAM.

13. Procédé selon l'une des revendications 1 à 12, **caractérisé en ce que** sur une première monocouche d'organophosphates et/ou organophosphonates, on précipite une deuxième ou séquentiellement encore d'autres monocouches, en solution aqueuse, de sorte que sur ladite surface de substrat, on produit une couche double ou multiple.

14. Procédé selon l'une des revendications 1 à 13, **caractérisé en ce que** par la première monocouche d'organophosphates et/ou organophosphonates, on précipite une surface hydrophobe en solution aqueuse, sur laquelle une autre couche de lipides synthétiques ou naturels est précipitée.

15. Procédé selon les revendications 1 et 14, **caractérisé en ce que** ladite autre couche est produite à partir de lipides synthétiques ou naturels depuis une suspension de vésicules lipidiques par fixation spontanée de la vésicule sur la surface hydrophobe d'une première monocouche d'organophosphates et/ou organophosphonates, suivi de l'étalement des membranes de vésicule sur cette première couche.

16. Procédé selon l'une des revendications 1, 14 à 15, **caractérisé en ce que** les lipides synthétiques ou naturels sont choisis parmi le groupe, qui est formé de phosphoglycérolipides etc. en solution aqueuse, ou comprennent un mélange de ces molécules.

17. Procédé selon l'une des revendications 1, 13 à 16, **caractérisé en ce que** des groupes moléculaires Y et/ou B et/ou éléments de reconnaissance biologiques ou biochimiques ou synthétiques et/ou composés qui confèrent à la surface, une résistance contre l'adsorption protéique et/ou l'adhésion cellulaire en solution aqueuse, selon l'une des revendications 1 à 11, sont associés à ladite autre couche.

18. Procédé selon l'une des revendications 1, 13 à 17, **caractérisé en ce que** sur une couche multiple avec une première monocouche d'organophosphates et/ou d'organophosphonates, des vésicules ou microsomes synthétiques ou naturels sont immobilisés en solution aqueuse, sur une surface de substrat, le cas échéant avec des éléments de reconnaissance biologiques ou biochimiques ou synthétiques ainsi associés, qui sont choisis parmi le groupe qui est formé des acides nucléiques, comme par exemple l'ADN, l'ARN, des oligonucléotides, des analogues d'acide nucléique, comme par exemple un PNA, des anticorps mono- ou polyclonaux, des peptides, des enzymes, des aptamères, des structures peptidiques synthétiques, des protéines solubles liées à des membranes ou isolées d'une membrane, par exemple des récepteurs, leurs ligands, des antigènes pour des anticorps, la biotine, des « composants de queue histidine » et leur partenaire de complexation.

19. Procédé selon l'une des revendications 1 à 18, **caractérisé en ce que** les couches précipitées consistent en des mélanges de couches simples ou multiples en des composés des formules I(A) et/ou I(B) et de leurs sels.

20. Procédé selon l'une des revendications 1 à 19, **caractérisé en ce qu'**avant la précipitation desdites couches simples ou multiples mixtes, le sel soluble dans l'eau d'un composé de la formule I(A) et/ou I(B) est isolé en solution aqueuse.

21. Procédé selon l'une des revendications 1, 19 à 21, **caractérisé en ce que** comme sel des composés de la formule (IA) et/ou (IB), on utilise en particulier les sels de sodium, de potassium et d'ammonium en solution aqueuse.

22. Procédé selon l'une des revendications 1, 19 à 21, **caractérisé en ce que** comme substrat, on utilise un oxyde, un nitrure ou un carbure de tantale, de niobium, de titane, de vanadium, de zirconium, de hafnium, de molybdène, de tungstène, de silicium ou leurs mélanges sous forme de corps massifs ou comme couches sur des substrats quelconques en solution aqueuse.

23. Procédé selon l'une des revendications 1, 19 à 22, **caractérisé en ce que** l'on utilise pour le revêtement, des composés de la formule (IA) et/ou (IB), dans lesquels les groupes B et Y signifient en commun, un groupe alkyle ou un groupe alkyle le cas échéant substitué de 2-24 atomes C.

24. Procédé selon l'une des revendications 1, 19 à 23, **caractérisé en ce que** comme élément de reconnaissance biologique, biochimique ou synthétique, lié à B ou Y, on choisit ceux du groupes des acides nucléiques, comme par exemple l'ADN, l'ARN, un oligonucléotide, un analogue d'acide nucléique, comme par exemple un PNA, des anticorps mono- ou polyclonaux, des peptides, des enzymes, des aptamères, des structures peptidiques synthétiques, des protéines solubles associées à des membranes ou isolées d'une membrane, par exemple des récepteurs, leurs ligands, des antigènes pour des anticorps, la biotine, des « composants de queue histidine » et leur partenaire de complexation.

25. Procédé selon l'une des revendications 1, 19 à 24, **caractérisé en ce qu'**un élément de reconnaissance biologique comprend un peptide, une protéine, une glycoprotéine, un facteur de croissance, comme par exemple TGF-β ou BMP (Bone Morphogenic protein).

26. Procédé selon l'une des revendications 1, 19 à 25, **caractérisé en ce que** comme composés, qui confèrent à la surface du substrat, une résistance contre l'adsorption de protéines et/ou l'adhésion cellulaire, ceux-ci sont choisis de préférence, parmi le groupe consistant en des composés, qui sont formés d'oligo(oxyde d'éthylène), la phosphorylcholine, l'héparine, des saccharides, l'albumine, en particulier la sérumalbumine bovine ou la sérumalbumine humaine, la caséine, des anticorps non spécifiques, polyclonaux ou monoclonaux, étrangers ou empiriques pour le ou les analytes à détecter, en particulier pour des immunodosages, des détergents, comme par exemple le Tween 20, un ADN fragmenté naturel ou synthétique, ne s'hybridant pas avec les polynucléotides à analyser, comme par exemple un extrait de sperme de hareng ou de saumon, en particulier pour des dosages d'hybridation de polynucléotide, ou également des polymères non chargés, mais hydrophiles, comme par exemple des polyéthylèneglycols ou des dextranes.

27. Procédé selon l'une des revendications 1, 19 à 26, **caractérisé en ce que** les composés de la formule (IA) et/ou (IB) comprennent différents groupes fonctionnels et/ou éléments de reconnaissance biologiques ou biochimiques ou synthétiques dans une molécule SAM.

28. Procédé selon l'une des revendications 1, 19 à 27, **caractérisé en ce que** le substrat présente une surface lisse avec une faible rugosité ou possède une surface rugueuse, où ces surfaces présentent le cas échéant, une structure supplémentaire.

29. Procédé selon l'une des revendications 1, 19 à 28, **caractérisé en ce que** par le choix des rapports de mélange, on contrôle l'hydrophile ou l'hydrophobie de la surface.

30. Procédé selon l'une des revendications 1, 19 à 29, **caractérisé en ce que** par le choix du rapport de mélange, on peut ajuster une densité contrôlée de charges positives et/ou négatives sur la surface.

31. Procédé selon l'une des revendications 19 à 30, **caractérisé en ce que** par le choix du rapport de mélange, on peut ajuster une densité contrôlée des groupes réactifs et/ou éléments de reconnaissance biochimiques ou « fonctions » biologiques.

32. Procédé selon la revendication 1, pour produire des surfaces chimiquement structurées par précipitation locale de couches simples ou multiples d'organophosphates et/ou organophosphonates sur la surface du substrat en oxydes, nitrures ou carbures de métal ou semi-conducteur, purs ou mixtes, **caractérisé en ce que** pour le traitement de la surface, des composés salins solubles dans l'eau des acides organophosphoriques ou acides organophosphoniques adéquats sont mis en oeuvre en solution aqueuse et que la précipitation des couches simples ou multiples pures ou mixtes est réalisée par un procédé selon l'une des revendications 1 à 31.

33. Procédé selon la revendication 32, **caractérisé en ce que** la surface du substrat présente un motif défini d'oxyde de silicium et respectivement, d'oxydes de métal de transition.

34. Procédé selon la revendication 33, **caractérisé en ce que** sur les zones d'oxyde de silicium, une autre précipitation de couches simples ou multiples d'organophosphates et/ou organophosphonates est réalisée à partir de solutions aqueuses.

35. Procédé selon l'une des revendications 1 à 34, **caractérisé en ce que** la précipitation des couches simples ou multiples est réalisée à partir de solutions aqueuses, le cas échéant localement sélectivement, en utilisant un procédé de précipitation, qui est choisi parmi le groupe qui est formé de l'immersion, l'étalement, la pose au pinceau, la « Ink jet spotting », le spotting mécanique à l'aide d'un crayon, une plume ou un capillaire, le « micro contact printing », le contact fluide de la surface du substrat avec des microcanaux parallèles ou croisés, par action de pressions différentes ou de potentiels électriques ou électromagnétiques, etc.

36. Procédé pour produire des surfaces chimiquement structurées selon une des revendications 1, 32 à 35, **caractérisé en ce que** par précipitation locale de couches simples ou multiples d'organophosphates et/ou organophosphonates sur la surface du substrat en oxydes, nitrures ou carbures de métal ou semi-conducteur, purs ou mixtes, on produit des zones locales hydrophiles ou hydrophobes et dont la surface environnante du substrat est ensuite couverte d'une couche simple à terminaisons hydrophiles et respectivement, hydrophobes en solution aqueuse.

37. Procédé **caractérisé en ce que** sur une surface chimiquement structurée, produite selon un procédé suivant une des revendications 1, 32 à 35, on précipite localement, séquentiellement une ou plusieurs couches simples selon une des revendications 13 à 18 et on produit ainsi, localement, des couches multiples en solution aqueuse.

38. Procédé selon la revendication 1, pour la préparation de surfaces d'implant avec implants en métaux revêtus d'oxyde, comme par exemple le titane, le tantale, le niobium, des alliages tels que titane-aluminium-vanadium, titane-aluminium-niobium, titane-niobium-zirconium, titane-niobium-zirconium-tantale, cobalt-chrome, cobalt-chrome-molybdène, fer-nickel-chrome, **caractérisé en ce que** sur la surface, on réalise la précipitation de couches simples ou multiples, pures ou mixtes, d'organophosphates et/ou d'organophosphonates selon une des revendications 1 à 12 ou 19 à 37 en solution aqueuse.

39. Procédé selon la revendication 1, pour la préparation de surfaces de plate-forme de capteur, **caractérisé en ce que** sur la surface, on réalise la précipitation de couches simples ou multiples, pures ou mixtes, d'organophosphates et/ou d'organophosphonates selon une des revendications 1 à 37 en solution aqueuse.

40. Implant en métal ou céramique avec une monocouche d'organophosphates et/ou d'organophosphonates comme surface, **caractérisé en ce que** la surface est obtenue par un procédé de précipitation en solution aqueuse selon une des revendications 1 à 12 ou 19 à 37.

41. Implant selon la revendication 40, **caractérisé en ce que** l'implant est choisi parmi le groupe des implants racinaires pour la dentisterie, des prothèses artificielles, comme par exemple de tiges, de cols ou de plaques de hanche, des rotules artificielles, des composants d'ostéosynthèse, comme par exemple des plaques osseuses, des vis, des composants « fixateur externe » pour la réparation de dégâts dans une zone de dégâts, des composants de « dispositif maxillo-facial », dans le domaine de la chirurgie de la colonne vertébrale, « spinal surgery implants », des stents, des composants de stimulateur cardiaque.

42. Appareil médical en métal ou céramique avec couches simples ou multiples d'organophosphates et/ou d'organophosphonates comme surface, **caractérisé en ce que** la surface est obtenue par un procédé de précipitation en solution aqueuse selon une des revendications 1 à 12 ou 19 à 37.

43. Appareil médical en métal ou céramique selon la revendication 42, **caractérisé en ce que** celui-ci est choisi parmi le groupe comprenant un cathéter, un ballonnet, un endoscope, des composants de systèmes conduisant le sang, externes, comme par exemple des machines de circulation externe.

44. Plate-forme de capteur avec une couche simple ou multiple d'organophosphates et/ou d'organophosphonates comme surface, **caractérisée en ce que** la surface est obtenue par un procédé de précipitation en solution aqueuse selon une des revendications 1 à 37.

45. Plate-forme de capteur selon la revendication 44, **caractérisée en ce qu'**elle comprend au moins un assemblage de zones discrètes de mesure (d) d'éléments de reconnaissance biologiques ou biochimiques ou synthétiques immobilisés pour la reconnaissance spécifique et/ou la liaison d'un ou de plusieurs analytes et/ou interactions spécifiques avec lesdits analytes.

46. Plate-forme de capteur selon l'une des revendications 44 à 45, **caractérisée en ce que** la détection du ou des plusieurs analytes est réalisée à l'aide de marqueurs, qui sont choisis parmi le groupe, qui est formé par exemple, de marqueurs luminescents, en particulier d'intercalants luminescents ou « molecular beacons », des marqueurs par absorption, des marqueurs massiques, en particulier des colloïdes métalliques ou des billes plastiques, des marqueurs de spin, comme des marqueurs pour RSE ou RMN, des marqueurs radioactifs.

47. Plate-forme de capteur selon l'une des revendications 44 à 46, **caractérisée en ce que** la détection des analytes repose sur la détermination d'une modification de l'indice de réfraction efficace sur base de l'absorption ou de la désorption moléculaire dans la zone de mesure (d).

48. Plate-forme de capteur selon la revendication 47, **caractérisée en ce que** la détection des analytes repose sur la détermination d'une modification des conditions de production d'un plasmon de surface dans la couche métallique d'un système à couche multiple, où la couche métallique consiste de préférence, en or ou argent.

49. Plate-forme de capteur selon l'une des revendications 44 à 48, **caractérisée en ce que** la détection des analytes repose sur la détermination d'une modification d'une ou de plusieurs luminescences.

50. Plate-forme de capteur selon la revendication 49, **caractérisée en ce que** la lumière d'excitation est irradiée dans une excitation de lumière incidente.

51. Plate-forme de capteur selon l'une des revendications 44 à 50, **caractérisée en ce que** le matériau se trouvant en contact avec la zone de mesure de la plate-forme de capteur est transparent ou absorbant à une profondeur d'au moins 200 nm de la zone de mesure, pour au moins une longueur d'onde d'excitation.

52. Plate-forme de capteur selon la revendication 50, **caractérisée en ce que** la lumière d'excitation est irradiée dans un agencement de transmission.

53. Plate-forme de capteur selon la revendication 52, **caractérisée en ce que** le matériau de la plate-forme de capteur est transparent pour au moins une longueur d'onde d'excitation.

54. Plate-forme de capteur selon l'une des revendications 44 à 53, **caractérisée en ce que** la plate-forme de capteur est formée comme un guide d'ondes optiques, qui est de préférence, essentiellement plane.

55. Plate-forme de capteur selon la revendication 53, **caractérisée en ce que** la plate-forme de capteur comprend un matériau optiquement transparent du groupe qui est formé de silicates, par exemple verre ou quartz, des matières thermoplastiques ou injectables transparentes, par exemple un polycarbonate, un polyimide, un acrylate, en particulier le poly(méthacrylate de méthyle), ou des polystyrènes.

56. Plate-forme de capteur selon la revendication 54, **caractérisée en ce que** la plate-forme de capteur comprend un guide d'ondes optiques à couche fine, avec une couche (a) transparente pour au moins une longueur d'onde d'excitation, sur une couche (b) également transparente pour au moins cette longueur d'onde d'excitation avec un indice de réfraction plus faible que la couche (a).

57. Procédé pour la détection qualitative et/ou quantitative simultanée d'un certain nombre d'analytes, **caractérisé en ce que** l'on met en contact un ou plusieurs échantillons liquides à examiner pour les analytes, avec les zones de mesure (d) sur une plate-forme de capteur selon une des revendications 44 à 56, et l'on mesure les modifications résultantes des signaux dans les zones de mesure.

58. Procédé selon la revendication 57, **caractérisé en ce que** la détection des analytes repose sur la détermination de la modification d'une ou de plusieurs luminescences.

59. Procédé selon la revendication 58, **caractérisé en ce que** la lumière d'excitation est irradiée depuis une ou plusieurs sources de lumière d'excitation dans une excitation de lumière incidente.

60. Procédé selon la revendication 58, **caractérisé en ce que** la lumière d'excitation est irradiée depuis une ou plusieurs sources de lumière d'excitation dans un agencement de transmission.

61. Procédé selon l'une des revendications 58 à 60, **caractérisé en ce que** la plate-forme de capteur est formée comme un guide d'ondes optiques, qui est de préférence, essentiellement plan, et **en ce que** la lumière d'excitation est couplée à une ou plusieurs sources de lumière dans le guide d'ondes optiques par un procédé qui est choisi dans le groupe, qui est formé du couplage frontal, du couplage par fibre optique comme guide d'ondes, couplage par prisme, couplage de réseau ou couplage évanescent par chevauchement du champ évanescent du dit guide d'ondes optiques avec le champ évanescent d'un autre guide d'ondes, en contact par champ proche.

62. Procédé selon l'une des revendications 57 à 61, **caractérisé en ce que** le ou les échantillons sont préincubés avec un mélange de différents réactifs de détection pour la détermination des analytes à détecter dans l'échantillon, et ces mélanges sont alors alimentés dans une seule étape d'addition, sur les assemblages prévus pour cela sur la plate-forme de capteur.

63. Procédé selon l'une des revendications 58 à 62, **caractérisé en ce que** l'étalonnage de la luminescence produite suite à la liaison d'un ou de plusieurs analytes ou suite à l'interaction spécifique avec un ou plusieurs analytes dans le champ proche de la couche (a), comprend l'addition d'une ou de plusieurs solutions d'étalonnage avec concentrations connues en lesdits analytes à déterminer sur les mêmes ou d'autres zones de mesure ou segments de zones de mesure ou assemblages de zones de mesure sur une plate-forme de capteur, où dans une étape identique ou séparée d'addition, on alimente le ou les plusieurs échantillons à examiner.

64. Procédé selon l'une des revendications 57 à 63, pour la détermination quantitative ou qualitative, simultanée ou séquentielle, d'un ou de plusieurs analytes du groupe des anticorps, récepteurs ou ligands, chélates ou « composants queue d'histidine », oligonucléotides, brins d'ADN ou d'ARN, analogues d'ADN ou d'ARN, enzymes, facteurs ou inhibiteurs enzymatiques, lectines ou hydrates de carbone.

65. Procédé selon l'une des revendications 57 à 64, **caractérisé en ce que** les échantillons à examiner proviennent de fluides corporels naturels, comme le sang, le sérum, le plasma, la lymphe ou l'urine ou le jaune d'oeuf ou des fluides optiques ou des fluides tissulaires ou de l'eau de surface ou des extraits de sols ou de végétaux ou des bouillons biologiques ou de synthèse ou des tissus biologiques ou des cultures ou extraits de cellules.

66. Utilisation d'une plate-forme de capteur selon une des revendications 44 à 56 et/ou procédé selon une des revendication 57 à 65 pour l'analyse quantitative ou qualitative pour la détermination d'analytes chimiques, biochimiques ou biologiques dans un procédé de criblage en recherche pharmaceutique, en chimie combinatoire, en développement clinique ou préclinique, pour des études de liaison en temps réel et pour la détermination de paramètres cinétiques dans un criblage d'affinité et en recherche, pour les déterminations qualitatives et quantitatives d'analytes, en particulier pour l'analyse d'ADN et d'ARN, pour la réalisation d'études de toxicité ainsi que pour la détermination de profils d'expression de gènes ou de protéines, ainsi que pour la détection d'anticorps, antigènes, pathogènes ou bactéries dans le développement et l'étude d'un produit pharmaceutique, les diagnostics humain et vétérinaire, le développement et l'étude de produits agrochimiques, le diagnostic végétal symptomatique et présymptomatique, pour la stratification des patients dans le développement d'un produit pharmaceutique et pour le choix thérapeutique d'un médicament, pour la détection de pathogènes, de substances nuisibles, en particulier des Salmonelles, des prions, des virus et des bactéries, en analyse alimentaire et environnementale.
